# EUROPEAN PATENT SPECIFICATION

(11) **EP 1 360 308 B1**
(45) Date of publication and mention of the grant of the patent: **10.10.2007**
(21) Application number: 02709978.7
(22) Date of filing: 25.01.2002
(51) Int. Cl.: C12N 15/66, C12N 15/10

(54) **CONCATEMERS OF DIFFERENTIALLY EXPRESSED MULTIPLE GENES**
CONCATEMERE UNTERSCHIEDLICH EXPRIMIERTER MULTIPLER GENE
CONCATEMERES DE MULTIPLES GENES EXPRIMES DE FA ON DIFFERENTIELLE

(30) Priority: 25.01.2001 DK 200100127; 27.06.2001 US 301022 P
(43) Date of publication of application: 12.11.2003
(73) Proprietor: Evolva Ltd., 4123 Allschwil (CH)
(72) Inventor: GOLDSMITH, Neil, Oxford OX4 1NX (GB); SORENSEN, Alexandra, M., P., SantAna, DK-2840 Holte (DK); NIELSEN, Soren, V., S., DK-3450 Allerod (DK); NAESBY, Michael, DK-2500 Valby (DK)
(74) Representative: HOEIBERG A/S
(86) International application number: PCT/DK2002/000055
(87) International publication number: WO 2002/059296

(56) References cited:
- WO-A1-00/52180
- US-A- 5 316 922

## Description

This application is a non provisional of U.S. provisional application Serial No. 60/301,022 filed 27. June 2001, which is hereby incorporated by reference in its entirety. The application claims priority from Danish patent application number PA 2001 00127 filed 25. January 2001, which is hereby incorporated by reference in its entirety. All patent and nonpatent references cited in the application, or in the present application, are also hereby incorporated by reference in their entirety.

In the present invention are disclosed concatemers of concatenated expression cassettes and vectors that enable the synthesis of such concatemers. The main purpose of these concatemers is the controllable and co-ordinated expression of large numbers of heterologous genes in a single host cell. Furthermore, the invention relates to a concatemer of cassettes of nucleotide sequences and a method for preparing the concatemers: In a further aspect, the invention relates to transgenic host cells comprising at least one concatemer according to the invention, as well as to a method for preparing the transgenic host cells. Finally, the invention relates to a vector comprising a cassette of nucleotides, a method for preparing said vector, a nucleotide library comprising at least two primary vectors each comprising a cassette of nucleotides, a method for preparing the library.

### Prior art

The design of expression constructs and expression libraries is well known in the art.

WO 96/34112 discloses a combinatorial gene expression library with a pool of expression constructs each construct containing a cDNA or genomic DNA fragment from a plurality of donor organisms. The DNA fragments are operably associated with regulatory regions that drive expression in a host cell. The publication also discloses a combinatorial gene expression library in which each cell comprises a concatemer of cDNA fragments being operably associated with regulatory regions to drive expression of the genes encoded by the concatenated cDNA in a host organism. The host organism may be a yeast cell. The vector used for constructing the library may be a plasmid vector, a phage, a viral vector, a cosmid vector or an artificial chromosome (BAC or YAC). Suitable promoters include natural and synthetic promoters as well as constitutive and inducible promoters.

The genes used for the the concatemers are prepared in a highly ordered multi-step procedure consisting of a number of discrete reaction steps. First, cDNA inserts are prepared using PCR and methylated dCTP to protect internal Not I and Bam HI restriction sites from later digestion. Promoter and terminator fragments are ligated to the 5' and 3' ends respectively using modified Bam HI adapters. The gene cassettes have the basic structure: promoter-coding sequence-terminator with different restriction sites in each end. The restriction site in the 3' end is protected. Similar gene, cassettes can be prepared from genomic DNA which is randomly fragmented using a restriction enzyme.

The concatemers disclosed in WO 96/34112 are prepared in a highly ordered multi-step procedure, where the first gene cassette is ligated to an adapter nucleotide sequence linked to a bead having a blunt end corresponding to the blunt 5' end of the gene cassette. After ligation, the restriction site is no longer functional, since it was assembled using two compatible but not identical restriction sites. After linking the first gene cassette to the bead, the protected restriction site in the 3' end is "opened" and the second gene cassette is linked to the first. After 5 to 10 rounds of ligation of gene cassettes, the vector is ligated to the 3' end of the concatemer, the concatemer-vector construct is liberated from the bead and the 5' end is ligated to the other end of the vector. It is emphasised that the 3' and 5' ends of the concatemer should be non-compatible to avoid self assembly during cloning into the vector.

Due to the plurality of discrete steps in the preparation procedure, the method is not suitable for preparing concatemers of significantly larger size. Once the gene cassettes have been cloned into the vector, it is not possible to excise the cassettes or the complete concatemer from the vector using a restriction enzyme.

US 6,057,103 (Diversa) discloses a method for identification of clones having a specified enzyme activity through isolation of DNA from a microorganism and hybridisation with a probe DNA comprising at least part of a sequence encoding an enzyme having a specified activity. The identified sequences are linked to a promoter sequence (e.g. eukaryotic promoters: CMV immediate/early, HSV thymidine kinase, early and late SV40, LTRs from retrovirus, and mouse metallothionein-I) and inserted into a vector, which may be a YAC or a P1 based artificial chromosome. Host cells used for transformation with the identified nucleotide sequences include bacterial cells, yeast, insect cells, mammalian cells. The disclosed vectors are not adapted for cloning of high numbers of expressible nucleotide sequences, and the reference does not disclose the concatemers for cloning of multiple genes into a host cell. More specifically the reference does not render possible the combination and screening of combinations of genes under conditions that allow genes to be combined in new ways.

US 5,958,672 (Diversa) discloses a method for identifying protein activity of interest by culturing a gene expression library obtained from uncultivated microorganisms (or lower eukaryotic species). The gene expression library may be obtained from genomic DNA or from cDNA. The DNA clones are controllably associated to regulatory sequences, which drive the expression of the sequences in the host cell. The libraries may be screened against a DNA probe as described in US 6,054,267. As is the case in the other references cited above, the combination of genes isolated from the uncultivated micro-organisms is a static combination. Once, the isolated genes are inserted into the host cells, no further gene combinations and no further optimisation of gene combinations are intended. WO 0 052 180 describes combinatorial libraries prepared using gene cassettes which each contains a promoter and a terminator. However the gene cassettes according to this document contain a BglII restriction site and a Bam H-I restriction site at their 5'-end and 3'-end, respectively. Since those restriction sites produce overlapping overhangs, it is possible to ligate gene cassettes to produce concatemers. However ligation of those sites does not produce a novel functional reduction site and hence the individual cassettes cannot be excised from the concatemer.

It is one objective of the present invention to provide methods and vectors for cloning of large numbers of expressible nucleotide sequences, which are especially adapted for later random ligation into concatemers, which can in turn be inserted into an expression host for expression of the genes in the concatemer or a sub-set of the genes or of different sub-sets of the genes in the concatemer. Thereby it becomes possible to optimise combinations of expressible nucleotide sequences for any screenable trait.

it is a further objective of the present invention to provide methods and vectors, which allow for the production of concatemers, which are flexible. By flexible in this context is meant, that the gene cassettes of the concatemers can be disassembled and reassembled easily using standard molecular techniques, such as excision by the use of restriction enzymes.

### Summary of the invention

According to a first aspect the invention relates to a nucleotide concatemer comprising in the 5'→3' direction a cassette of nucleotide sequence of the general formula

[rs₂-SP-PR-X-TR-SP-rs₁]ₙ

wherein
rs₁ and rs₂ together denote a restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases, and
n ≥ 2, and
wherein at least a first cassette is different from a second cassette.

The concatemers according to the invention may comprise a selection of expressible nucleotide sequences from just one expression state and can thus be assembled from one library representing this expression state or it may comprise cassettes from a number of different expression states mixed in any suitable ratio. The concatemers according to the invention are especially suitable for ligating into an artificial chromosome, which may be inserted into a host cell for coordinated expression. For this purpose, the variation among and between cassettes may be such as to minimise the chance of cross over as the host cell undergoes cell division such as through minimising the level of repeat sequences occurring in any one concatemer, since it is not an object of this embodiment of the invention to obtain recombination of concatemers with a segment in the host genome or an epitope of the host cells or any intraconcatemer recombination.

The concatemers can be used to make novel and non-native combinations of genes for co-ordinated expression in a host cell. Thereby new metabolic pathways can be generated, which may lead to the production of new metabolites, and/or to the metabolisation of compounds, which are otherwise not metabolisable by the host cells. The new gene combinations may also lead to metabolic pathways which produce metabolites in new quantities or in new compartments of the cell or outside the cell. Depending on the purpose, the selection of genes can be made completely random based on sourcing of expressible nucleotide sequences across the different kingdoms. However, it may also be advantageous to source genes from sources known to have certain metabolic pathways in order to make targeted new gene combinations. It may also be advantageous to source genes from organisms/tissues known to have relevant properties, e.g. pharmaceutical activity.

One of several advantages of the concatemers of the present invention is that the expression cassettes can be cut out from the concatemers at any point to make new combinations of expression cassettes. During re-assembly, further genes comprised in similar expression cassettes may be added if desired to modify the expression pattern. In this way, the concatemers according to the present invention present a powerful tool in generating novel gene combinations.

One advantage of the structure of the concatemer is that cassettes can be recovered from the host cell through nucleotide isolation and subsequent digestion with a restriction enzyme specific for the rs₁-rs₂ restriction site. The building blocks of the concatemers may thus be disassembled and reassembled at any point

The cassettes of the concatemer may be joined head to tail or head to head or tail to tail, which does not affect expression of the expressible nucleotide sequences because each expressible nucleotide sequence is under the control of its own promoter. This is due to the fact that most restriction enzymes leave two identical overhangs, which may combine in either orientation at the same frequency.

However the restriction sites can also be selected so that head to tail arrangement is favoured, for example by using restriction enzymes that generate non-palindromic overhangs. Examples of such enzymes are listed in example 6c and most of the enzymes in 6d. Non-palindromic overhangs will prevent head to head and tail to tail ligation. By the use of two or more different entry vectors, the sequence of the cutting region can be designed to yield different overhangs after digestion with a single of these enzymes. Examples of such enzymes are most of the enzymes in example 6c and one in 6d, and have variable nucleotides (N or W) in the overhang. In this way a cassette can be excised with one enzyme that has non-identical overhangs.

This will prevent intramolecular religation and prevent that identical cassettes ligate to each other, decreasing the risk of intramolecular recombination.

The invention in a further aspect relates to a method for concatenation comprising the steps of concatenating at least two cassettes of nucleotide sequences each cassette - comprising a first sticky end, a spacer sequence, a promoter, an expressible nucleotide sequence, a terminator, and a second sticky end.

Preferably, the method comprises starting from primary vectors comprising a cassette having the following nucleotide sequence

[RS1-RS2-SP-PR-X-TR-SP-RS2'-RS1'],

wherein X denotes an expressible nucleotide sequence,
RS1 and RS1' denote restriction sites,
RS2 and RS2' denote restriction sites different from RS1 and RS1',
SP individually denotes a spacer sequence of at least two nucleotides,
PR denotes a promoter,
TR denotes a terminator,
i) cutting the primary vector with the aid of at least one restriction enzyme specific for RS2 and RS2' obtaining cassettes having the general formula [rs₂-SP-PR-X-TR-SP-rs₁] wherein rs₁ and rs₂ together denote a functional restriction site RS2 or RS2',
ii) assembling the cut out cassettes through interaction between rs₁ and rs₂.

According to this embodiment, excision and concatenation is carried out in a "one step" reaction, i.e. without an intervening purification step, starting from vectors containing the expression cassettes. The expression cassettes can be cut out using two restriction enzymes specific for RS1 and RS2. Preferably for this one step reaction RS1 leaves blunt ends and RS2 leaves sticky ends. Upon addition of a ligase, the concatemer can be assembled in the mixture without any need for purification, since the vector backbone and the small RS1-RS2 fragments do not interfere with the concatenation reaction.

In the case, where the concatemer is to be inserted into an artificial chromosome vector for later transformation into an expression host cell, the AC vector arms can be added directly to the concatenation reaction mixture, so that the complete artificial chromosome vector containing the concatemer can be assembled in one step. By controlling the ratio of vector arms to cassettes, the size of the concatemer can be controlled. It is of course also possible to control the size of the concatemers by adding stopper fragments, the stopper fragments each having a RS2 or RS2' in one end and a non-complementaty overhang or a blunt end in the other end. Vector arms may also be added later on in a separate step.

Advantageously, the method comprises addition of vector arms each having a RS2 or RS2' in one end and a non-complementary overhang or a blunt end in the other end. These can be added to the concatenation mixture, and even in this complex mixture, concatemers with one vector arm in each end will be produced under appropriate conditions. Host cells transformed with the desired construction, including the appropriate vector arms, can be selected by utilizing marker genes present on the arms.

In one aspect the invention relates to a host cell, which comprises at least one concatemer of individual oligonucleotide cassettes, each concatemer comprising oligonucleotide of the following formula in 5'→3' direction: [rs₂-SP-PR-X-TR-SP-rs₁]ₙ, wherein rs₁ and rs₂ together denote a restriction site, SP individually denotes a spacer of at least two nucleotide bases, PR denotes a promoter, capable of functioning in the cell, X denotes an expressible nucleotide sequence, TR denotes a terminator, and SP individually denotes a spacer of at least two nucleotide bases, wherein n ≥ 2, and wherein at least two expressible- nucleotide sequences are from different expression states.

In another aspect the invention relates to a cell comprising at least one concatemer of individual oligonucleotide cassettes, each concatemer comprising oligonucleotide of the following formula in 5'→3' direction:

[rs₂SP-PR-X-TR-SP-rs₁]ₙ

wherein
rs₁ and rs₂ together denote a restriction site,
SP individually denotes a spacer of at least two nucleotide bases,
PR denotes a promoter, capable of functioning in the cell,
X denotes an expressible nucleotide sequence,
TR denotes a terminator, and
SP individually denotes a spacer of at least two nucleotide bases,
wherein n ≥ 2, and
wherein rs₁-rs₂ in at least two cassettes is recognised by the same restriction enzyme.

Thereby non-naturally occurring combinations of expressible genes can be combined in one cell in such a way that co-ordinated expression of a subset of genes is made possible or all the inserted genes may be expressed at the same time. Through external regulation of the promoters controlling the expressible nucleotides sequences novel and non-naturally occurring combinations of expressed genes can be obtained. Since these novel and non-natural combinations of gene products are found in one and the same cell, the heterologous gene products may affect the metabolism of the host cell in novel ways and thus cause it to produce novel and/or non-native primary or secondary metabolites and/or known metabolites in novel amounts and/or known metabolites in novel compartments of the cell or outside the cells. The novel metabolic pathways and/or novel or modified metabolites may be obtained without substantially recombining the introduced genes with any segment in the host genome or any episome of the host cells.

The cells containing the concatemers, preferably in the form of artificial chromosomes, may be used for directed evolution by subjecting populations of cells to selective conditions. One advantage of the structure of the concatemers is that expression cassettes from different cells or from different populations of cells can be combined easily in a few steps thereby increasing the potential of the evolution. When the concatemers are inserted in the form of artificial chromosomes, evolution may also be carried out using traditional breeding and selection.

It is likely that through the combination of a high number of non-native genes in a host cell combinations of genes or single genes are inserted that are lethal or sub-lethal to the host cell. Through the co-ordinated expression of the genes in the host cell it is possible not only to induce the expression of any subset of genes but also to repress such expression, e.g. of lethal or sub-lethal genes.

By producing cells with combinations of concatemers comprising cassettes with expressible nucleotide sequences from a number of different expression states, which may be from any number of expression states, from the same or from selected species, from unrelated or distantly related species, or from species from different kingdoms, novel and random combinations of gene products are produced in one single cell. By furthermore having expressible nucleotide sequences under the control of a number of independently inducible or repressible promoters, a large number of different expression states can be created inside one single cell by selectively turning on and off groups of the inserted expressible nucleotide sequences. The number of independently inducible and/or repressible promoters in one cell may vary from 1 to 100, 1 to 50, 1 to 10, or such as up to 15, 20, 25 or above 50 promoters.

By inserting novel genes into the host cell, and especially by inserting a high number of novel genes from a wide variety of species into a host cell, it is highly likely that the gene products from this array of novel genes will interact with the pool of metabolites of the host cell and modify known metabolites and/or intermediates in novel ways to create novel compounds. Since the interaction is performed at the enzyme level it is furthermore likely that the result will be novel compounds with chiral centres, which are especially difficult to synthesise via chemical synthesis.

Novel metabolic pathways may also be made that are not capable of functioning, due to the absence of a substrate in the host cell. Such metabolic pathways may be made active by addition of non-host-cell specific substrates, which are metabolisable by the novel pathways.

One special advantage of the cells according to the present invention, is that incompatibility barriers between species do not limit the combinations of genes in one single cell.

According to a further aspect the invention relates to a method for producing a transgenic cell comprising inserting into a host cell a concatemer comprising a heterologous nucleotide sequence comprising at least two genes each controlled by a promoter, wherein the two genes and/or the two promoters are different.

According to a further aspect, the invention concerns a primary vector comprising a nucleotide sequence cassette of the general formula in 5'→3' direction:

[RS1-RS2-SP-PR-CS-TR-SP-RS2'-RS1']

wherein
RS1 and RS1' denote restriction sites,
RS2 and RS2' denote restriction sites different from RS1 and RS1',
SP individually denotes a spacer sequence of at least two nucleotides,
PR denotes a promoter,
CS denotes a cloning site,
TR denotes a terminator.

The cassette within the primary vector is useful for cloning and storing in a host cell random expressible nucleotide sequences, which are under the control of the promoter comprised in the cassette. The cassette may be inserted and removed by using restriction enzymes specific for either of the four restriction sites, of which RS1 and RS1' are preferably identical and RS2 and RS2' are also preferably identical. One special advantage of the cassette is that a collection of cassettes may be assembled into a concatemer of cassettes according to the invention by excising the cassettes from the primary vector, e.g. through use of the restriction enzyme(s) specific for RS2 and RS2', and concatenation of a population of random cassettes in a solution. The easiest concatenation is obtained when-RS1 and RS1' leave blunt ends and RS2 and RS2' leave sticky ends. In this way it can be avoided that the empty vector takes part in the concatenation. Furthermore, it has been observed that the small fragments containing RS1-RS2 and RS1'-RS2' do not have to be removed, since they do not interfere with the concatenation. If desired, the small fragments can easily be removed using e.g. precipitation or filtration.

The primary vector according to the invention is especially adapted for expression with cDNA into it, because it is equipped with a promoter. Integration of genomic DNA: is also possible however this may cause interaction between the native promoter sequences of the inserted genomic DNA and the external control obtainable through the promoter of the vector.

Preferably, PR is not functional in the host of the primary vector but only functional in the expression host, into which the concatemers are going to be inserted for expression. This is to avoid selection against genes which are lethal to the library host, in which the primary vector is stored and/or amplified.

The spacer sequence is inserted in the cassette in order to increase stability of the concatemers after concatenation. The cassettes are built so that they can be joined head to tail, head to head or tail to tail after concatenation. A concatemer of two cassettes can thus have the following structure:
3' rs₂-SP-PR-X-TR-SP-rs₁-rs₁-SP-PR-X-TR-SP-rs₁ 5'
3' rs₂SP-TR-X-PR-SP-rs₁-rs₂SP-PR-X-TR-SP-rs₁ 5'
3' rs₂-SP-PR-X-TR-SP-rs₁-rs₂-SP-TR-X-PR-SP-rs₁ 5'
(rs₁-rs₂ together denote a restriction site)

The presence of the spacer reduces the risk of hairpin formation between two adjacent terminator or promoter sequences, which may be identical. As a consequence, the presence of the spacer is also intended to increase the stability of the concatemers in the expression host into which they are inserted.

Using enzymes that leave non-palindromic overhangs it is possible to generate concatemers with essentially head to tail orientation.

In another aspect the invention relates to a method of preparing a primary vector comprising
inserting an expressible nucleotide sequence into a cloning site in a primary vector comprising a cassette, the cassette comprising a nucleotide sequence of the general formula in 5'→3' direction:

   [RS1-RS2-SP-PR-CS-TR-SP-RS2'-RS1']

   wherein
   RS1 and RS1' denote restriction sites,
   RS2 and RS2' denote restriction sites different from RS1 and RS1',
   SP individually denotes a spacer sequence of at least two nucleotides,
   PR denotes a promoter,
   CS denotes a cloning site, and
   TR denotes a terminator.

In a further aspect the invention relates to a nucleotide library comprising at least two primary vectors each vector comprising a nucleotide sequence cassette of the general formula in 5'→3' direction:

[RS1-RS2-SP-PR-X-TR-SP-RS2'-RS1']

wherein
RS1 and RS1' denote restriction sites,
RS2 and RS2' denote restriction sites different from RS1 and RS1',
SP individually denotes a spacer sequence of at least two nucleotides,
PR denotes a promoter,
X denotes an expressible nucleotide sequence,
TR denotes a terminator.
- wherein the expressible nucleotide sequences are isolated from one expression state, and
- wherein at least two cassettes are different.

The nucleotide library may also be referred to as an entry (=initial) library, the intention being to use the nucleotide library as a suitable means for storing and amplifying a high number of vectors comprising the nucleotide cassettes according to the invention. It is also intended to excise the cassettes after amplification in order to use the excised cassettes for concatenation: Conveniently one nucleotide library may cover expressible nucleotide sequences from the same source pool, such as from the same expression state. Therefore, the library is conveniently used for introducing cDNA synthesised from mRNA isolated from one expression state.

Preferably, the PR sequences are not capable of functioning in the library host cells. This is to ensure that none of the expressible nucleotide sequences are lethal to the library host cell and therefore may be lost.

The nucleotide library furthermore provides a suitable means for later assembly of concatemers of cassettes stored in the library. According to an especially preferred embodiment of the invention, substantially all cassettes in the library are different. This difference is partly introduced to be able to coordinately express different subset of genes and partly to minimise the level of repeat sequences occurring in the concatemers.

In a still further aspect the invention relates to a method for preparing a nucleotide library comprising obtaining expressible nucleotide sequences, cloning the expressible nucleotide sequences into cloning sites of a mixture of primary vectors, the primary vectors comprising a cassette, the cassettes comprising a nucleotide sequence of the general formula in 5'→3' direction:

[RS1-RS2-SP-PR-CS-TR-SP-RS2'-RS1']

wherein
RS1 and RS1' denote restriction sites,
RS2 and RS2' denote restriction sites different from RS1 and RS1',
SP individually denotes a spacer sequence of at least two nucleotides,
PR denotes a promoter,
CS denotes a cloning site, and
TR denotes a terminator,
and transferring the primary vectors into a host cell obtaining a library.

Conveniently the method may comprise building a cDNA library from mRNA isolated from one expression state or starting from a cDNA library and cloning the cDNA sequences into a mixture of primary vectors according to the invention. In order to have the library and sub-libraries organised in a proper manner, each library comprises expressible nucleotide sequences representative of a given expression state.

For all the concatemers and libraries discussed herein the spacers (SP, promotors (PR), and terminators (TR) may be identical in all cassettes, but in preferred embodiments the spacers (SP, promotors (PR), and terminators (TR) are different for at least a part of the cassettes in a concatemer and a library.

### Brief description of the drawings

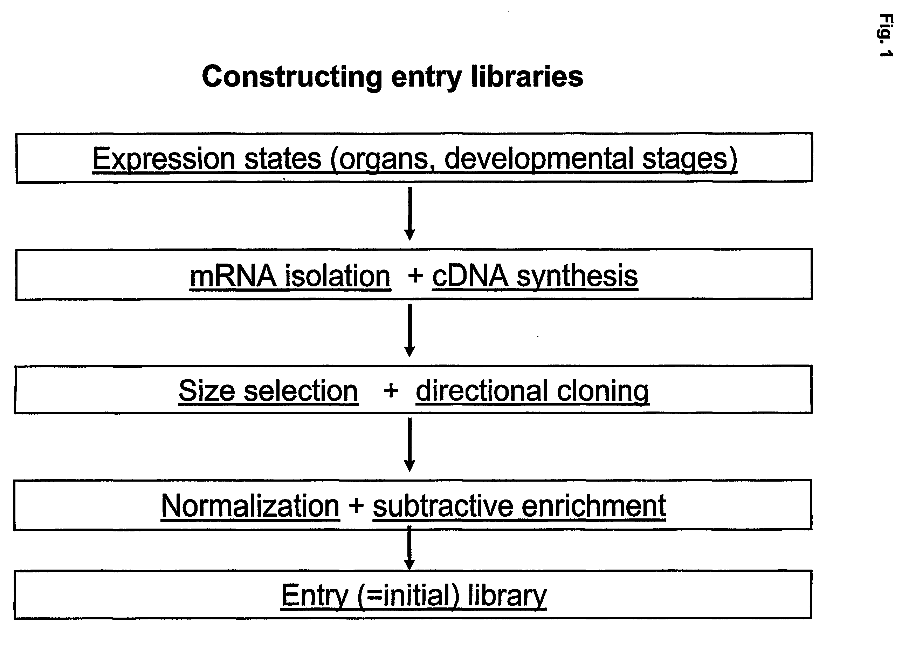

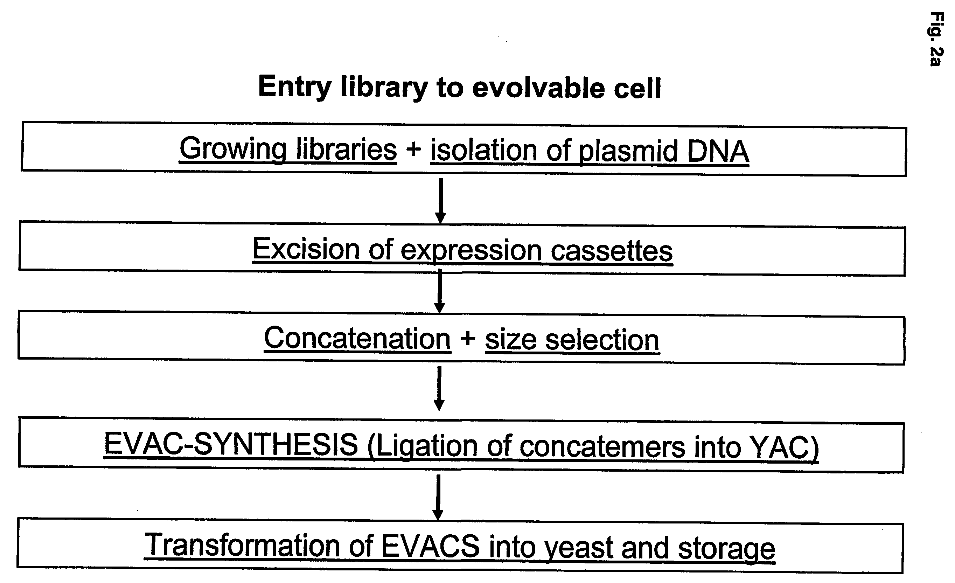

Fig. 1 shows a flow chart of the steps leading from an expression state to incorporation of the expressible nucleotide sequences in an entry library (a nucleotide library according to the invention).

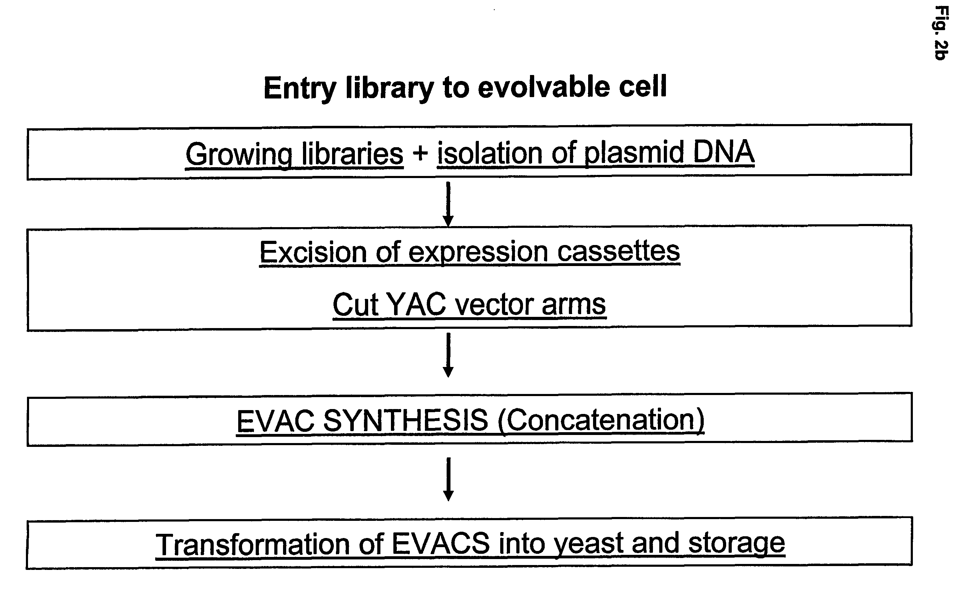

Fig. 2 shows a flow chart of the steps leading from an entry library comprising expressible nucleotide sequences to evolvable artificial chromosomes (EVAC) transformed into an appropriate host cell. Fig. 2a shows one way of producing the EVACs which includes concatenation, size selection and insertion into an artificial chromosome vector. Fig. 2b shows a one step procedure for concatenation and ligation of vector arms to obtain EVACs.

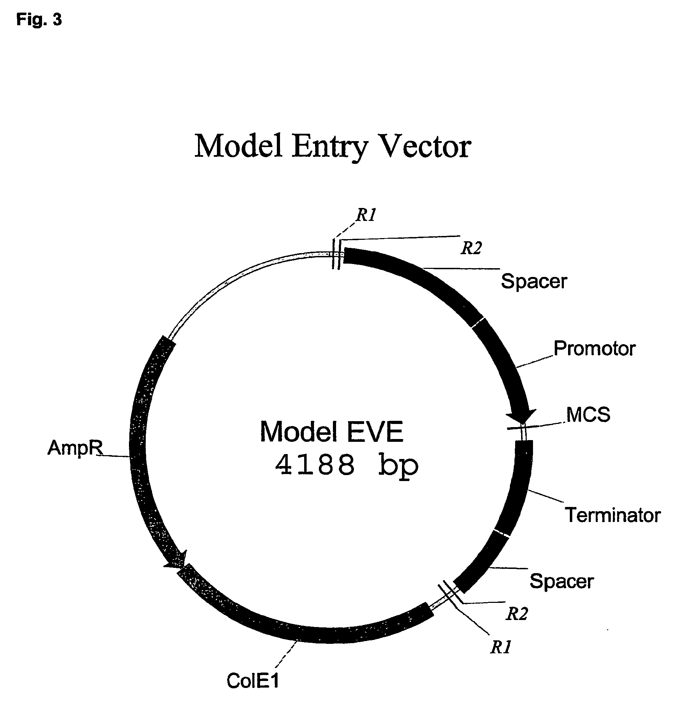

Fig. 3 shows a model entry vector. MCS is a multi cloning site for inserting expressible nucleotide sequences. Amp R is the gene for ampicillin resistance. Col E is the origin of replication in E. coli. R1 and R2 are restriction enzyme recognition sites.

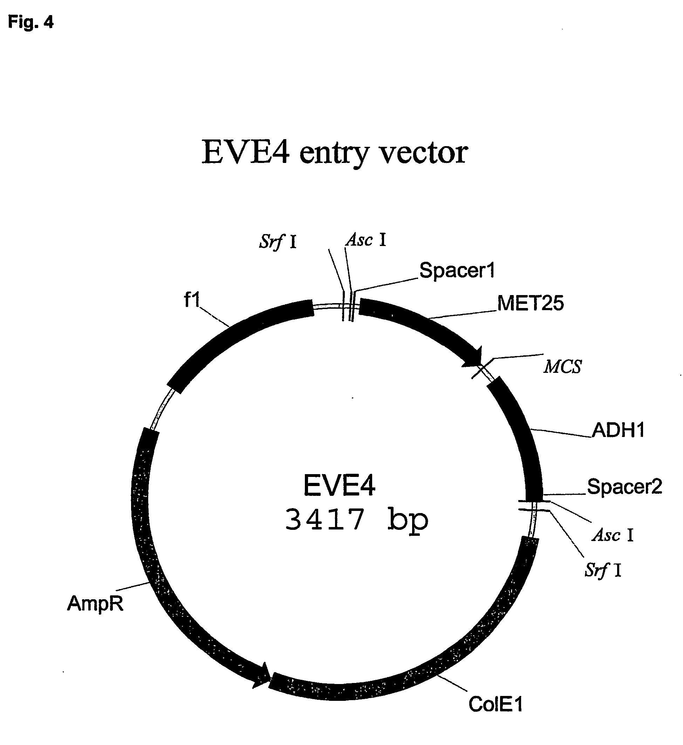

Fig. 4 shows an example of an entry vector according to the invention, EVE4. MET25 is a promoter, ADH1 is a terminator, f1 is an origin of replication for filamentous phages, e.g. M13. Spacer 1 and spacer 2 are constituted by a few nucleotides deriving from the multiple cloning site, MCS, SrfI and AscI are restriction enzyme recognition sites. Other abbreviations, see Fig. 3. The sequence of the vector is set forth in SEQ ID NO 1.

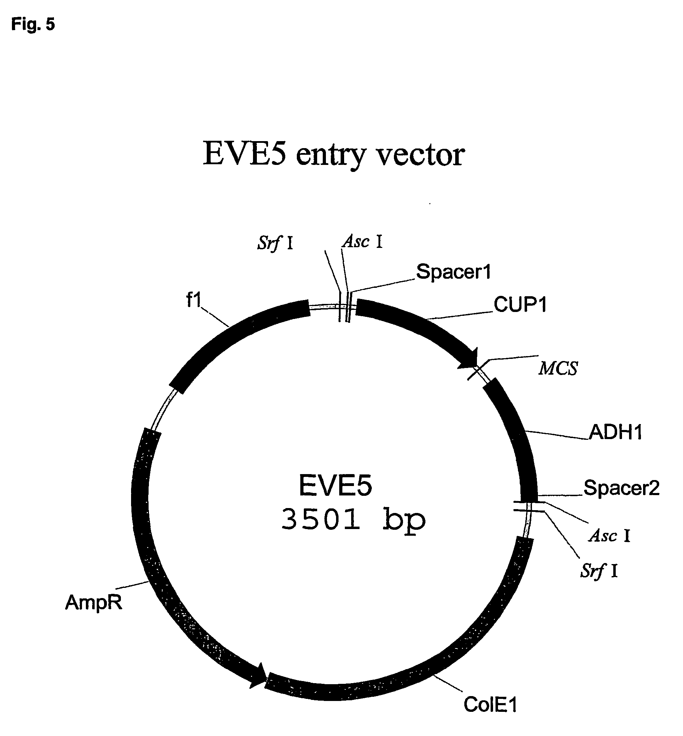

Fig 5 shows an example of an entry vector according to the invention, EVE5. CUP1 is a promoter, ADH1 is a terminator, f1 is an origin of replication for filamentous phages, e.g. M13. Spacer 1 and spacer 2 are constituted by a few nucleotides deriving from the multiple cloning site, MCS, SrfI and AscI are restriction enzyme recognition sites. Other abbreviations, see Fig. 3. The sequence of the vector is set forth in SEQ ID NO 2.

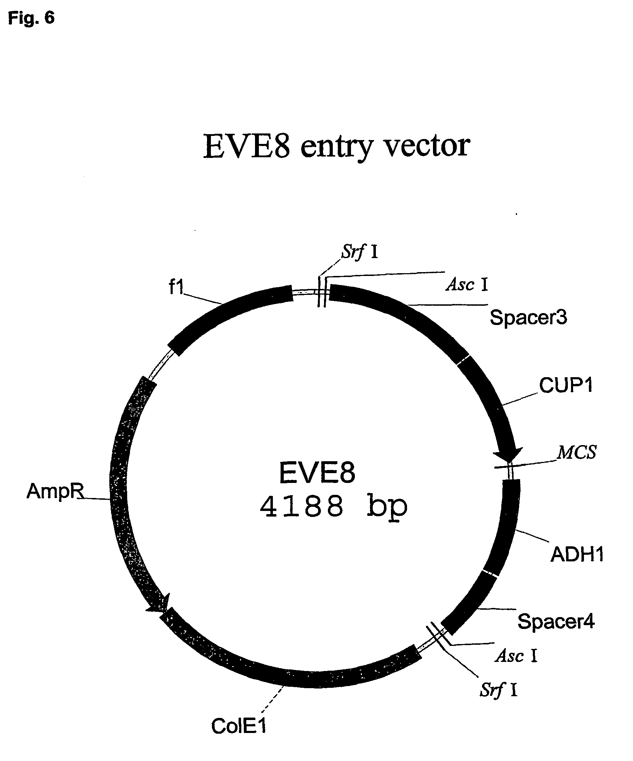

Fig 6 shows an example of an entry vector according to the invention, EVE8. CUP1 is a promoter, ADH1 is a terminator, f1 is an origin of replication for filamentous phages, e.g. M13. Spacer3 is a 550 bp fragment of lambda phage DNA fragment. Spacer4 is a ARS1 sequence from yeast. SrfI and AscI are restriction enzyme recognition sites. Other abbreviations, see Fig. 3. The sequence of the vector is set forth in SEQ ID NO 3.

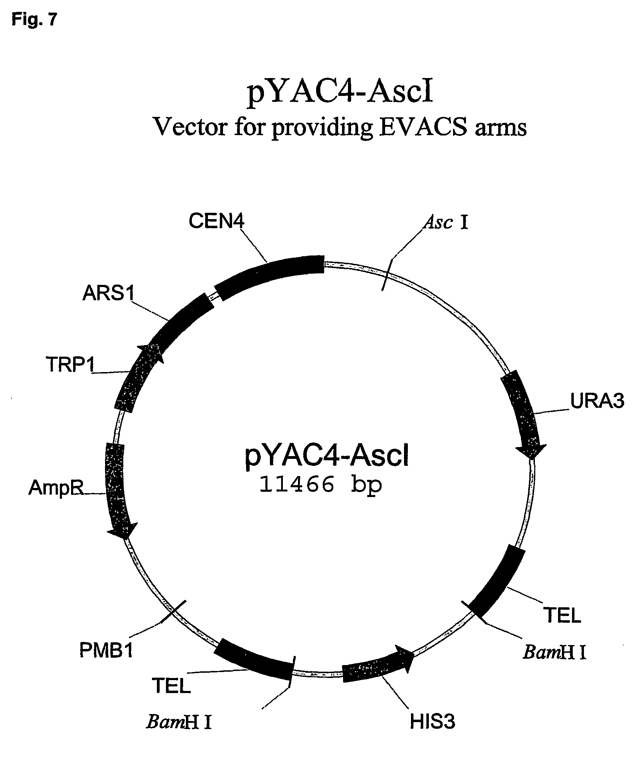

Fig. 7 shows a vector (pYAC4-AscI) for providing arms for an evolvable artificial chromosome (EVAC) into which a concatemer according to the invention can be cloned. TRP1, URA3, and HIS3 are yeast auxotrophic marker genes, and AmpR is an E. coli antibiotic marker gene. CEN4 is a centromere and TEL are telomeres. ARS1 and PMB1 allow replication in yeast and E. coli respectively. BamH I and AscI are restriction enzyme recognition sites. The nucleotide sequence of the vector is set forth in SEQ ID NO 4.

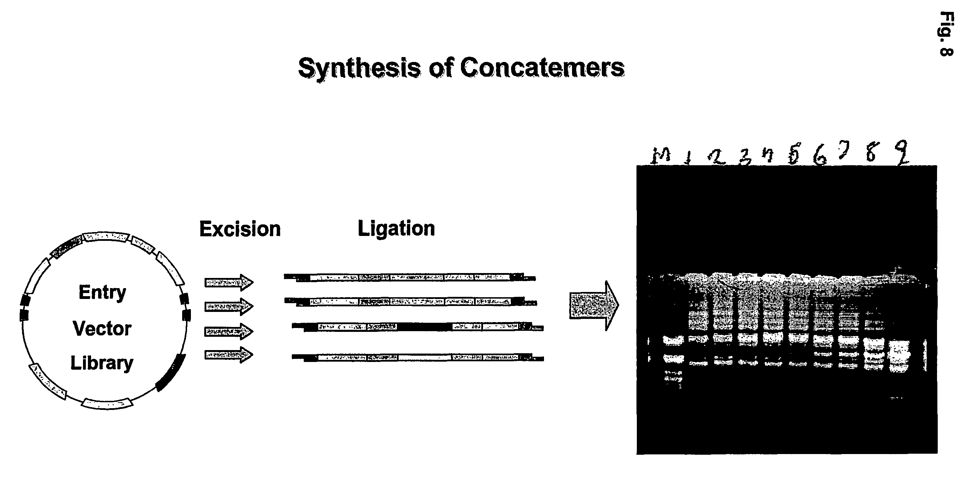

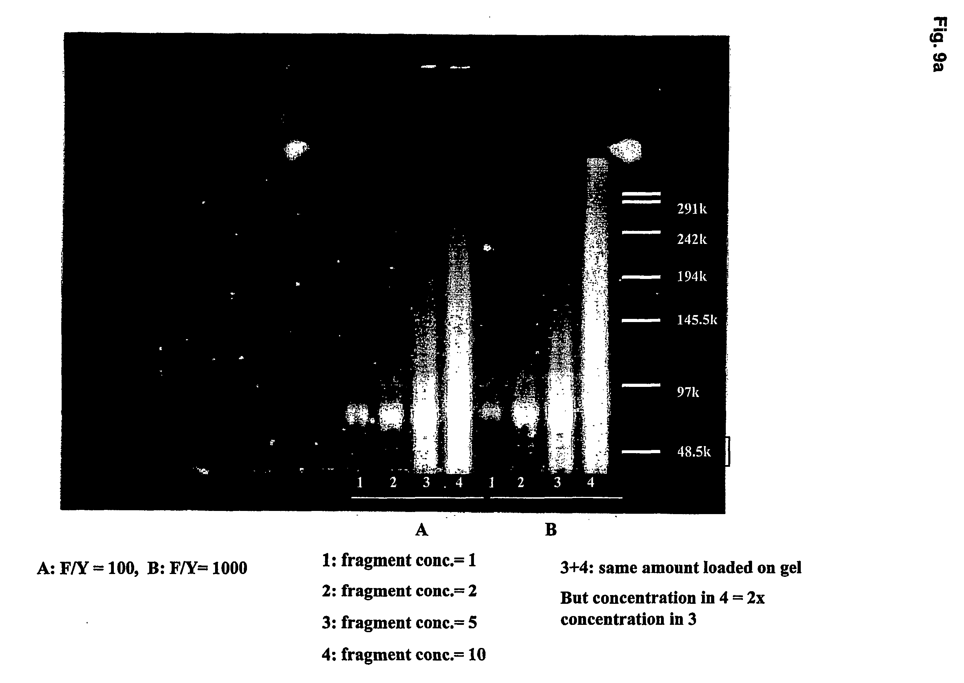

Fig 8. shows the general concatenation strategy. On the left is shown a circular entry vector with restriction sites, spacers, promoter, expressible nucleotide sequence and terminator. These are excised and ligated randomly.

| Lane | F/Y |
|---|---|
| 1 | 100/1 |
| 2 | 50/1 |
| 3 | 20/1 |
| 4 | 10/1 |
| 5 | 5/1 |
| 6 | 2/1 |
| 7 | 1/1 |
| 8 | 1/2 |
| 9 | 1/5 |

| | |
|---|---|
| Legend: Lane M: molecular weight marker, λ-phage DNA digested w. Pst1. Lanes 1-9, concatenation reactions. Ratio of fragments to yac-arms(F/Y) as in table. | |

Fig 9a and 9b. illustrates the integration of concatenation with synthesis of evolvable artificial chromosomes and how concatemer size can be controlled by controlling the ratio of vector arms to expression cassettes, as described in example 7.

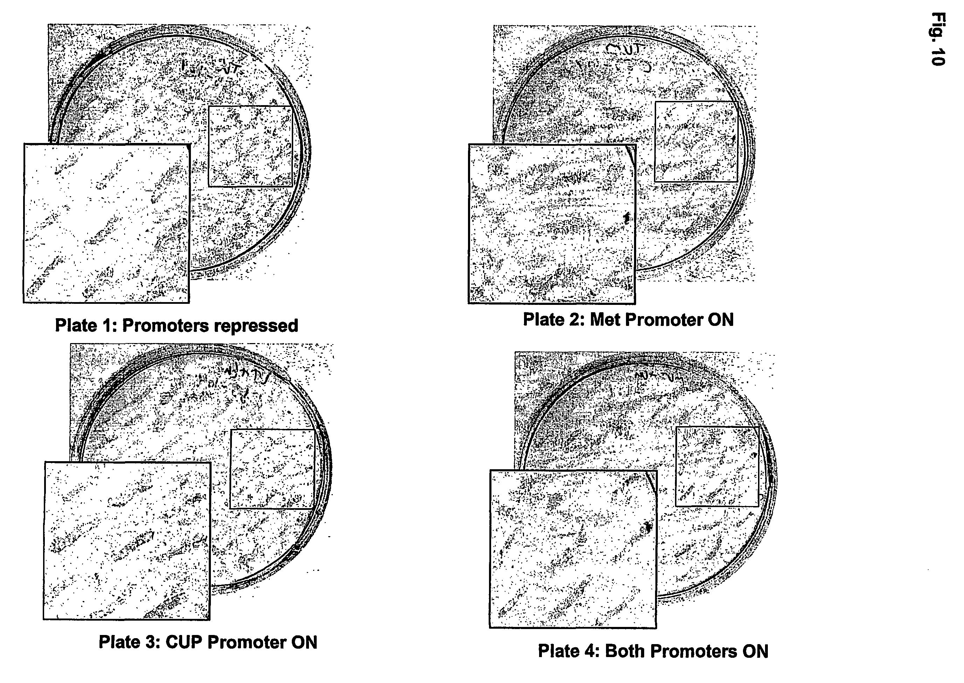

Fig 10. Library of EVAC transformed population shown under 4 different growth conditions. Coloured phenotypes can be readily detected upon induction of the Met25 and/or the Capl promoters.

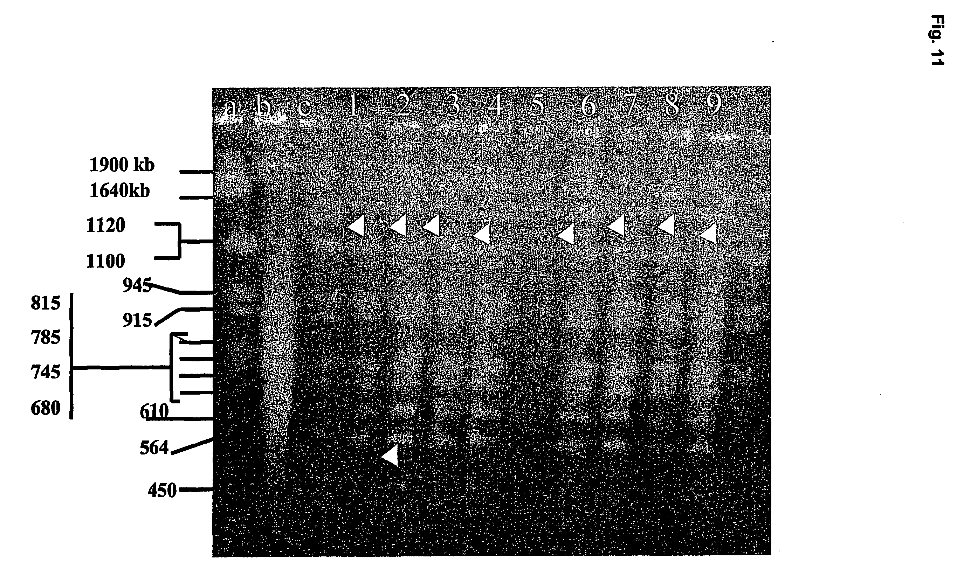

Fig 11. EVAC gel Legend: PFGE of EVAC containing clones:
Lanes. a: Yeast DNA PFGE markers(strain YNN295), b: lambda ladder, c: non-transformed host yeast,1 - 9 : EVAC containing clones. EVACs in size range 1400-1600 kb. Lane 2 shows a clone containing 2 EVACs sized ~1500 kb and ~550 kb respectively. The 550kb EVAC is comigrating with the 564kb yeast chromosome and is resulting in an increased intensity of the band at 564 kb relative to the other bands in the lane. Arrows point up to EVAC bands.

### Definitions

### Oligonucleotides

Any fragment of nucleic acids having approximately from 2 to 10000 nucleic acids.

### Restriction site

For the purposes of the present invention the abbreviation RSn (n=1,2,3, etc) is used to designate a nucleotide sequence comprising a restriction site. A restriction site is defined by a recognition sequence and a cleavage site. The cleavage site may be located within or outside the recognition sequence. The abbreviation "rs₁" or "rs₂" is used to designate the two ends of a restriction site after cleavage. The sequence "rs₁-rs₂" together designate a complete restriction site.

The cleavage site of a restriction site may leave a double stranded polynucleotide sequence with either blunt or sticky ends. Thus, "rs₁" or "rs₂" may designate either a blunt or a sticky end.

In the notation used throughout the present invention, formulae like:

RS1-RS2-SP-PR-X-TR-SP-RS2-RS1

should be interpreted to mean that the individual sequences follow in the order specified. This does not exclude that part of the recognition sequence of e.g. RS2 overlap with the spacer sequence, but it is a strict requirement that all the items except RS1 and RS1' are functional and remain functional after cleavage and reassemblage. Furthermore the formulae do not exclude the possibility of having additional sequences inserted between the listed items. For example introns can be inserted as described in the invention below and further spacer sequences can be inserted between RS1 and RS2 and between TR and RS2. Important is that the sequences remain functional.

Furthermore, when reference is made to the size of the restriction site and/or to specific bases within it, only the bases in the recognition sequence are referred to.

### Expression state

An expression state is a state in any specific tissue of any individual organism at any one time. Any change in conditions leading to changes in gene expression leads to another expression state. Different expression states are found in different individuals, in different species but they may also be found in different organs in the same species or individual, and in different tissue types in the same species or individual. Different expression states may also be obtained in the same organ or tissue in any one species or individual by exposing the tissues or organs to different environmental conditions comprising but not limited to changes in age, disease, infection, drought, humidity, salinity, exposure to xenobiotics, physiological effectors, temperature, pressure, pH, light, gaseous environment, chemicals such as toxins.

### Artificial chromosome

As used herein, an artificial chromosome (AC) is a piece of DNA that can stably replicate and segregate alongside endogenous chromosomes. For eukaryotes the artificial chromosome may also be described as a nucleotide sequence of substantial length comprising a functional centromer, functional telomeres, and at least one autonomous replicating sequence. It has the capacity to accommodate and express heterologous genes inserted therein. It is referred to as a mammalian artificial chromosome (MAC) when it contains an active mammalian centromere. Plant artificial chromosome and insect artificial chromosome (BUGAC) refer to chromosomes that include plant and insect centromers, respectively. A human artificial chromosome (HAC) refers to a chromosome that includes human centromeres, AVACs refer to avian artificial chromosomes. A yeast artificial chromosome (YAC) refers - to chromosomes are functional in yeast, such as chromosomes that include a yeast centromere.

As used herein, stable maintenance of chromosomes occurs when at least about 85%, preferably 90%, more preferably 95% of the cells retain the chromosome. Stability is measured in the presence of a selective agent. Preferably these chromosomes are also maintained in the absence of a selective agent. Stable chromosomes also retain their structure during cell culturing, suffering neither intrachromosomal nor interchromosomal rearrangements.

### Detailed description of the invention

In the following the invention is described in the order in which the steps of obtaining a transformed host cell containing an evolvable artificial chromosome may be performed, starting with the entry vector.

### Origin of expressible nucleotide sequences

The expressible nucleotide sequences that can be inserted into the vectors, concatemers, and cells according to this invention encompass any type of nucleotide such as RNA, DNA. Such a nucleotide sequence could be obtained e.g. from cDNA, which by its nature is expressible. But it is also possible to use sequences of genomic DNA, coding for specific genes. Preferably, the expressible nucleotide sequences correspond to full length genes such as substantially full length cDNA, but nucleotide sequences coding for shorter peptides than the original full length mRNAs may also be used. Shorter peptides may still retain the catalytic activity similar to that of the native proteins.

Another way to obtain expressible nucleotide sequences is through chemical synthesis of nucleotide sequences coding for known peptide or protein sequences. Thus the expressible DNA sequences does not have to be a naturally occurring sequence, although it may be preferable for practical purposes to primarily use naturally occurring nucleotide sequences. Whether the DNA is single or double stranded will depend on the vector system used.

In most cases the orientation with respect to the promoter of an expressible nucleotide sequence will be such that the coding strand is transcribed into a proper mRNA. It is however conceivable that the sequence may be reversed generating an antisense transcript in order to block expression of a specific gene.

### Cassettes

An important aspect of the invention concerns a cassette of nucleotides in a highly ordered sequence, the cassette having the general formula in 5'→3' direction:

[RS1-RS2-SP-PR-CS-TR-SP-RS2'-RS1']

wherein RS1 and RS1' denote restriction sites, RS2 and RS2' denote restriction sites different from RS1 and RS1', SP individually denotes a spacer sequence of at least two nucleotides, PR denotes a promoter, CS denotes a cloning site, and TR denotes a terminator.

It is an advantage to have two different restriction sites flanking both sides of the expression construct. By treating the primary vectors with restriction enzymes cleaving both restriction sites, the expression construct and the primary vector will be left with two non-compatible ends. This facilitates a concatenation process, since the empty vectors do not participate in the concatenation of expression constructs.

### Restriction sites

in principle, any restriction site, for which a restriction enzyme is known can be used. These include the restriction enzymes generally known and used in the field of molecular biology such as those described in Sambrook, Fritsch, Maniatis, "A laboratory Manual", 2nd edition. Cold Spring Harbor Laboratory Press, 1989.

The restriction site recognition sequences preferably are of a substantial length, so that the likelihood of occurrence of an identical restriction site within the cloned oligonucleotide is minimised. Thus the first restriction site may comprise at least 6 bases, but more preferably the recognition sequence comprises at least 7 or 8 bases. Restriction sites having 7 or more non N bases in the recognition sequence are generally known as "rare restriction sites" (see example 6). However, the recognition sequence may also be at least 10 bases, such as at least 15 bases, for example at least 16 bases, such as at least 17 bases, for example at least 18 bases, such as at least 18 bases, for example at least 19- bases, for example at least 20 bases, such as at least 21 bases, for example at least 22 bases, such as at least 23 bases, for example at least 25 bases, such as at least 30 bases, for example at least 35 bases, such as at least 40 bases, for example at least 45 bases, such as at least 50 bases.

Preferably the first restriction site RS1 and RS1' is recognised by a restriction enzyme generating blunt ends of the double stranded nucleotide sequences. By generating blunt ends at this site, the risk that the vector participates in a subsequent concatenation is greatly reduced. The first restriction site may also give rise to sticky ends, but these are then preferably, non-compatible with the sticky ends resulting from the second restriction site, RS2 and RS2' and with the sticky ends in the AC.

According to a preferred embodiment of the invention, the second restriction site, RS2 and RS2' comprises a rare restriction site. Thus, the longer the recognition sequence of the rare restriction site the more rare it is and the less likely is it that the restriction enzyme recognising it will cleave the nucleotide sequence at other - undesired - positions.

The rare restriction site may furthermore serve as a PCR priming site. Thereby it is possible to copy the cassettes via PCR techniques and thus indirectly "excise" the cassettes from a vector.

### Spacer sequence

The spacer sequence located between the RS2 and the PR sequence is preferably a non-transcribed spacer sequence. The purpose of the spacer sequence(s) is to minimise recombination between different concatemers present in the same cell or between cassettes present in the same concatemer, but it may also serve the purpose of making the nucleotide sequences in the cassettes more "host" like- A further purpose of the spacer sequence is to reduce the occurrence of hairpin formation between adjacent palindromic sequences, which may occur when cassettes are assembled head to head or tail to tail. Spacer sequences may also be convenient for introducing short conserved nucleotide sequences that may serve e.g. as PCR primer sites or as target for hybridization to e.g. nucleic acid or PNA or LNA probes allowing affinity purification of cassettes.
The cassette may also optionally comprise another spacer sequence of at least two nucleotides between TR and RS2. When cassettes are cut out from a vector and concatenated into concatemers of cassettes, the spacer sequences together ensure that there is a certain distance between two successive identical promoter and/or terminator sequences. This distance may comprise at least 50 bases, such as at least 60 bases, for example at least 75 bases, such as at least 100 bases, for example at least 150 bases, such as at least 200 bases, for example at least 250 bases, such as at least 300 bases, for example at least 400 bases, for example at least 500 bases, such as at least 750 bases, for example at least 1000 bases, such as at least 1100 bases, for example at least 1200 bases, such as at least 1300 bases, for example at least 1400 bases, such as at least 1500 bases, for example at least 1600 bases, such as at least 1700 bases, for example at least 1800 bases, such as at least 1900 bases, for example at least 2000 bases, such as at least 2100 bases, for example at least 2200 bases, such as at least 2300 bases, for example at least 2400 bases, such as at least 2500 bases, for example at least 2600 bases, such as at least 2700 bases, for example at least 2800 bases, such as at least 2900 bases, for example at least 3000 bases, such as at least 3200 bases, for example at least 3500 bases, such as at least 3800 bases, for example at least 4000 bases, such as at least 4500 bases, for example at least 5000 bases, such as at least 6000 bases.

The number of the nucleotides between the spacer located 5' to the PR sequence and the one located 3' to the TR sequence may be any. However, it may be advantageous to ensure that at least one of the spacer sequences comprises between 100 and 2500 bases, preferably between 200 and 2300 bases, more preferably between 300 and 2100 bases, such as between 400 and 1900 bases, more preferably between 500 and 1700 bases, such as between 600 and 1500 bases, more preferably between 700 and 1400 bases.

If the intended host cell is yeast, the spacers present in a concatemer should perferably comprise a combination of a few ARSes with varying lambda phage DNA fragments.

Preferred examples of spacer sequences include but are not limited to: Lamda phage DNA, prokaryotic genomic DNA such as E. coli genomic DNA, ARSes.

### Promoter

A promoter is a DNA sequence to which RNA polymerase binds and initiates transcription. The promoter determines the polarity of the transcript by specifying which strand will be transcribed.
- Bacterial promoters normally consist of -35 and -10 (relative to the transcriptional start) consensus sequences which are bound by a specific sigma factor and RNA polymerase.
- Eukaryotic promoters are more complex. Most promoters utilized in expression vectors are transcribed by RNA polymerase II. General transcription factors (GTFs) first bind specific sequences near the transcriptional start and then recruit the binding of RNA polymerase 11. In addition to these, minimal promoter elements, small sequence elements are recognized specifically by modular DNA-binding / trans-activating proteins (e.g. AP-1, SP-1) which regulate the activity of a given promoter.
- Viral promoters may serve the same function as bacterial and eukaryotic promoters. Upon viral infection of their host, viral promoters direct transcription either by using host transcriptional machinery or by supplying virally encoded enzymes to substitute part of the host machinery. Viral promoters are recognised by the transcriptional machinery of a large number of host organisms and are therefore often used in cloning and expression vectors.

Promoters may furthermore comprise regulatory elements, which are DNA, sequence elements which act in conjunction with promoters and bind either repressors (e.g., lacO/ LAC Iq repressor system in E. coli) or inducers (e.g., gal1 /GAL4 inducer system in yeast). In either case, transcription is virtually "shut off" until the promoter is derepressed or induced, at which point transcription is "turned-on". The choice of promoter in the cassette is primarily dependent on the host organism into which the cassette is intended to be inserted. An important requirement to this end is that the promoter should preferably be capable of functioning in the host cell, in which the expressible nucleotide sequence is to be expressed.

Preferably the promoter is an externally controllable promoter, such as an inducible promoter and/or a repressible promoter. The promoter may be either controllable (repressible/inducible) by chemicals such as the absence/presence of chemical inducers, e.g. metabolites, substrates, metals, hormones, sugars. The promoter may likewise be controllable by certain physical parameters such as temperature, pH, redox status, growth stage, developmental stage, or the promoter may be inducible/repressible by a synthetic inducer/repressor such as the gal inducer.

In order to avoid unintentional interference with the gene regulation systems of the host cell, and in order to-improve controllability of the co-ordinated gene expression the promoter is preferably a synthetic promoter. Suitable promoters are described in US 5,798,227, US 5,667,986. Principles for designing suitable synthetic eukaryotic promoters are disclosed in US 5,559,027, US 5,877,018 or US 6,072,050.

Synthetic inducible eukaryotic promoters for the regulation of transcription of a gene may achieve improved levels of protein expression and lower basal levels of gene expression. Such promoters preferably contain at least two different classes of regulatory elements, usually by modification of a native promoter containing one of the inducible elements by inserting the other of the inducible elements. For example, additional metal responsive elements IR:Es) and/or glucocorticoid responsive elements (GREs) may be provided to native promoters. Additionally, one or more constitutive elements may be functionally disabled to provide the lower basal levels of gene expression.

Preferred examples of promoters include but is not limited to those promoters being induced and/or repressed by any factor selected from the group comprising carbohydrates, e.g. galactose; low inorganic phosphase levels; temperature, e.g. low or high temperature shift; metals or metal ions, e.g. copper ions; hormones, e.g. dihydrotestosterone; deoxycorticosterone; heat shock (e.g. 39°C); methanol; redox-status; growth stage, e.g. developmental stage; synthetic inducers, e.g. gal inducer. Examples of such promoters include ADH 1, PGK 1, GAP 491, TPI, PYK, ENO, PMA 1, PHO5, GAL 1, GAL 2, GAL 10, MET25, ADH2, MEL 1, CUP 1, HSE, AOX, MOX, SV40, CaMV, Opaque-2, GRE; ARE, PGK/ARE hybrid, CYC/GRE hybrid, TPI/α2 operator, AOX 1, MOX A.

More preferably, however the promoter is selected from hybrid promoters such as PGK/ARE hybrid, CYC/GRE hybrid or from synthetic promoters. Such promoters can be controlled without interfering too much with the regulation of native genes in the expression host.

### Yeast promoters

In the following, examples of known yeast promoters that may be used in conjunction with the present invention are shown. The examples are by no way limiting and only serve to indicate to the skilled practitioner how to select or design promoters that are useful according to the present invention.

Although numerous transcriptional promoters which are functional in yeasts have been described in the literature, only some of them have proved effective for the production of polypeptides by the recombinant route. There may be mentioned in particular the promoters of the PGK genes (3-phosphoglycerate kinase, TDH genes encoding GAPDH (Glyceraldehyde phosphate dehydrogenase), TEF1 genes (Elongation factor 1), MFα1 (α sex pheromone precursor) which are considered as strong constitutive promoters or alternatively the regulatable promoter CYCI which is repressed in the presence of glucose or PHO5 which can be regulated by thiamine. However, for reasons which are often unexplained, they do not always allow the effective expression of the genes which they control. In this context, it is always advantageous to be able to have new promoters in order to generate new effective host/vector systems. Furthermore, having a choice of effective promoters in a given cell also makes it possible to envisage the production of multiple proteins in this same cell (for example several enzymes of the same metabolic chain) while avoiding the problems of recombination between homologous sequences.

In general, a promoter region is situated in the 5' region of the genes and comprises all the elements allowing the transcription of a DNA fragment placed under their control, in particular.
(1) a so-called minimal promoter region comprising the TATA box and the site of initiation of transcription, which determines the position of the site of initiation as well as the basal level of transcription. In Saccharomyces cerevisiae, the length of the minimal promoter region is relatively variable. Indeed, the exact location of the TATA box varies from one gene to another and may be situated from -40 to -120 nucleotides upstream of the site of the initiation (Chen and Struhl, 1985, EMBO J., 4, 3273-3280)
(2) sequences situated upstream of the TATA box (immediately upstream up to several hundreds of nucleotides) which make it possible to ensure an effective level of transcription either constitutively (relatively constant level of transcription all along the cell cycle, regardless of the conditions of culture) or in a regulatable manner (activation of transcription in the presence of an activator and/or repression in the presence of a repressor). These sequences, may be of several types: activator, inhibitor, enhancer, inducer, repressor and may respond to cellular factors or varied culture conditions.

Examples of such promoters are the ZZA1 and ZZA2 promoters disclosed in US 5,641,661, the EF1-α protein promoter and the ribosomal protein S7 gene promoter disclosed in WO 97/44470,, the COX 4 promoter and two unknown promoters (SEQ ID No: 1 and 2 in the document) disclosed in US 5,952,195. Other useful promoters include the HSP150 promoter disclosed in WO 98/54339 and the SV40 and RSV promoters disclosed in US 4,870,013 as well as the PyK and GAPDH promoters disclosed in EP 0 329 203 A1.

### Synthetic yeast promoters

More preferably the invention employs the use of synthetic promoters. Synthetic promoters are often constructed by combining the minimal promoter region of one gene with the upstream regulating sequences of another gene. Enhanced promoter control may be obtained by modifying specific sequences in the upstream regulating sequences, e.g. through substitution or deletion or through inserting multiple copies of specific regulating sequences. One advantage of using synthetic promoters is that they may be controlled without interfering too much with the native promoters of the host cell.

One such synthetic yeast promoter comprises promoters or promoter elements of two different yeast-derived genes, yeast killer toxin leader peptide, and amino terminus of IL-1β (W.0 98/54339).

Another example of a yeast synthetic promoter is disclosed in US 5,436,136 (Hinnen et al), which concerns a yeast hybrid promoter including a 5' upstream promoter element comprising upstream activation site(s) of the yeast PH05 gene and a 3' downstream promoter element of the yeast GAPDH gene starting at nucleotide -300 to -180 and ending at nucleotide -1 of the GAPDH gene.

Another example of a yeast synthetic promoter is disclosed in US 5,089,398 (Rosenberg et al). This disclosure describes a promoter with the general formula

-(P.R.(2)-P.R.(1))-

wherein:
P.R.(1) is the promoter region proximal to the coding sequence and having the transcription initiation site, the RNA polymerase binding site, and including the TATA box, the CAAT sequence, as well as translational regulatory signals, e.g., capping sequence, as appropriate;
P.R.(2) is the promoter region joined to the 5'-end of P.R.(1) associated with enhancing the efficiency of transcription of the RNA polymerase binding region;

In US 4,945,046 (Horii et al) discloses a further example of how to design a synthetic yeast promoter. This specific promoter comprises promoter elements derived both from yeast and from a mammal. The hybrid promoter consists essentially of Saccharomyces cérevisiae PH05 or GAP-DH promoter from which the upstream activation site (UAS) has been deleted and replaced by the early enhancer region derived from SV40 virus.

### Cloning site

The cloning site in the cassette in the primary vector should be designed so that any nucleotide sequence can be cloned into it.

The cloning site in the cassette preferably allows directional cloning. Hereby is ensured that transcription in a host cell is performed from the coding strand in the intended direction and that the translated peptide is identical to the peptide for which the original nucleotide sequence codes.

However according to some embodiments it may be advantageous to insert the sequence in opposite direction. According to these embodiments, so-called antisense constructs may be inserted which prevent functional expression of specific genes involved in specific pathways. Thereby it may become possible to divert metabolic intermediates from a prevalent pathway to another less dominant pathway.

The cloning site in the cassette may comprise multiple cloning sites, generally known as MCS or polylinker sites, which is a synthetic DNA sequence encoding a series of restriction endonuclease recognition sites. These sites are engineered for convenient cloning of DNA into a vector at a specific position and for directional cloning of the insert.

Cloning of cDNA does not have to involve the use of restriction enzymes. Other alternative systems include but are not limited to:
- Creator^{™} Cre-loxP system from Clontech, which uses recombination and loxP sites
- use of Lambda attachment sites (att-λ), such as the Gateway^{™} system from Life Technologies.
Both of these systems are directional.

### Terminator

The role of the terminator sequence is to limit transcription to the length of the coding sequence. An optimal terminator sequence is thus one, which is capable of performing this act in the host cell.

In prokaryotes, sequences known as transcriptional terminators signal the RNA polymerase to release the DNA template and stop transcription of the nascent RNA.

In eukaryotes, RNA molecules are transcribed well beyond the end of the mature mRNA molecule. New transcripts are enzymatically cleaved and modified by the addition of a long sequence of adenylic acid residues known as the poly-A tail. A polyadenylation consensus sequence is located about 10 to 30 bases upstream from the actual cleavage site.

Preferred examples of yeast derived terminator sequences include, but are not limited to: ADN1, CYC1, GPD, ADH1 alcohol dehydrogenase.

### Intron

Optionally, the cassette in the vector comprises an intron sequence, which may be located 5' or 3' to the expressible nucleotide sequence. The design and layout of introns is well known in the art. The choice of intron design largely depends on the intended host cell, in which the expressible nucleotide sequence is eventually to be expressed: The effects of having intron sequence in the expression cassettes are those generally associated with intron sequences.

Examples of yeast introns can be found in the literature and in specific databases such as Ares Lab Yeast-Intron Database (Version 2.1) as updated on 15 April 2000. Earlier versions of the database as well as extracts of the database have been published in: "Genome-wide bioinformatic and molecular analysis of introns in Saccharomyces cerevisiae." by Spingola M, Grate L, Haussler D, Ares M Jr. (RNA 1999 Feb;5(2):221-34) and "Test of intron predictions reveals novel splice sites, alternatively spliced mRNAs and new introns in meiotically regulated genes of yeast." by Davis CA, Grate L, Spingola M, Ares M Jr, (Nucleic Acids Res 2000 Apr 15;28(8):1700-6).

### Primary vectors (entry vectors)

By the term entry vector is meant a vector for storing and amplifying cDNA or other expressible nucleotide sequences using the cassettes according to the present invention. The primary vectors are preferably able to propagate in E. coli or any other suitable standard host cell. It should preferably be amplifiable and amenable to standard normalisation and enrichment procedures.

The primary vector may be of any type of DNA that has the basic requirements of a) being able to replicate itself in at least one suitable host organism and b) allows insertion of foreign DNA which is then replicated together with the vector and c) preferably allows selection of vector molecules that contain insertions of said foreign DNA. In a preferred embodiment the vector is able to replicate in standard hosts like yeasts, and bacteria and it should preferably have a high copy number per host cell. It is also preferred that the vector in addition to a host specific origin of replication, contains an origin of replication for a single stranded virus, such as e.g. the f1 origin for filamentous phages. This will allow the production of single stranded nucleic acid which may be useful for normalisation and enrichment procedures of cloned sequences. A vast number of cloning vectors have been described which are commonly used and references may be given to e.g. Sambrook,J; Fritsch, E.F; and Maniatis T. (1989) Molecular Cloning: A laboratory manual. Cold Spring Harbour Laboratory Press, USA, Netherlands Culture Collection of Bacteria (www.cbs.knaw.nl/NCCB/collection.htm) or Department of Microbial Genetics, National Institute of Genetics, Yata 1111 Mishima Shizuoka 411-8540, Japan (www.shigen.nig.ac.ip/cvector/cvector.html). A few type-examples that are the parents of many popular derivatives are M13mp10, pUC18, Lambda gt 10, and pYAC4. Examples of primary vectors include but are not limited to M13K07, pBR322, pUC18; pUC19, pUC118, pUC119, pSP64, pSP65, pGEM-3, pGEM-3Z, pGEM-3Zf(-), pGEM-4, pGEM-4Z, πAN13, pBluescript II, CHARON 4A, λ⁺, CHARON 21A, CHARON 32, CHARON 33, CHARON 34, CHARON 35, CHARON 40, EMBL3A, λ2001, λDASH, λFIX, λgt10, λgt11, λgt18, λgt20, λgt22, λORF8, λZAP/R, pJB8, c2RB, pcos1EMBL

Methods for cloning of cDNA or genomic DNA into a vector are well known in the art. Reference may be given to J. Sambrook, E.F. Fritsch, T. Maniatis: Molecular Cloning, A Laboratory Manual (2nd edition, Cold Spring Harbor Laboratory Press, 1989).

One example of a circular model entry vector is described in Figure 3. The vector, EVE contains the expression cassette, R1-R2-Spacer-Promoter-Multi Cloning Site-Terminator-Spacer-R2-R1. The vector furthermore contains a gene for ampicillin resistance, AmpR, and an origin of replication for E.coli, ColE1.

The entry vectors EVE4, EVE5, and EVE8 shown in Figures 4, 5, and 6. These all contain SrfI as R1 and AscI as R2. Both of these sites are palindromic and are regarded as rare restriction sites having 8 bases in the recognition sequence. The vectors furthermore contain the AmpR ampicillin resistance gene, and the ColE1 origin or replication for E.coli as well as f1, which is an origin of replication for filamentous phages, such as M13. EVE4 (Fig. 4) contains the MET25 promoter and the ADH1 terminator. Spacer 1 and spacer 2 are short sequences deriving from the multiple cloning site, MCS. EVE5 (Fig. 5) contains the CUP1 promoter and the ADH1 terminator. EVE8 (Fig. 6) contains the CUP1 promoter and the ADH1 terminator. The spacers of EVE8 are a 550 bp lambda phage DNA (spacer 3) and an ARS sequence from yeast (spacer 4).

### Nucleotide library (entry library)

Methods as well as suitable vectors and host cells for constructing and maintaining a library of nucleotide sequences in a cell are well known in the art. The primary requirement for the library is that is should be possible to store and amplify in it a number of primary vectors (constructs) according to this invention, the vectors (constructs) comprising expressible nucleotide sequences from at least one expression state and wherein at least two vectors (constructs) are different.

One specific example of such a library is the well known and widely employed cDNA libraries. The advantage of the cDNA library is mainly that it contains only DNA sequences corresponding to transcribed messenger RNA in a cell. Suitable methods are also present to purify the isolated mRNA or the synthesised cDNA so that only substantially full-length cDNA is cloned into the library.

Methods for optimisation of the process to yield substantially full length cDNA may comprise size selection, e.g. electrophoresis, chromatography, precipitation or may comprise ways of increasing the likelihood of getting full length cDNAs, e.g. the SMART^{™} method (Clonetech) or the CapTrap^{™} method (Stratagene).

Preferably the method for making the nucleotide library comprises obtaining a substantially full length cDNA population comprising a normalised representation of cDNA species. More preferably a substantially full length cDNA population comprises a normalised representation of cDNA species characteristic of a given expression state.

Normalisation reduces the redundancy of clones representing abundant mRNA species and increases the relative representation of clones from rare mRNA species.

Methods for normalisation of cDNA libraries are well known in the art. Reference may be given to suitable protocols for normalisation such as those described in US 5,763,239 (DIVERSA) and WO 95/08647 and WO 95/11986. and Bonaldo, Lennon, Soares, Genome Research 1996, 6:791-806; Ali, Holloway, Taylor, Plant Mol Biol Reporter, 2000,18:123-132.

Enrichment methods are used to isolate clones representing mRNA which are characteristic of a particular expression state. A number of variations of the method broadly termed as subtractive hybrisation are known in the art. Reference may be given to Sive, John, Nucleic Acid Res, 1988, 16:10937; Diatchenko, Lau, Campbell et al, PNAS, 1996, 93:6025-6030; Caminci, Shibata, Hayatsu, Genome Res, 2000, 10:1617-30, Bonaldo, Lennon, Soares, Genome Research 1996, 6:791-806; Ali, Holloway, Taylor, Plant Mol Biol Reporter, 2000, 18:123-132. For example, enrichment may be achieved by doing additional rounds of hybridization similar to normalization procedures, using e.g. cDNA from a library of abundant clones or simply a library representing the uninduced state as a driver against a tester library from the induced state. Alternatively mRNA or PCR amplified cDNA derived from the expression state of choice can be used to subtract common sequences from a tester library. The choice of driver and tester population will depend on the nature of target expressible nucleotide sequences in each particular experiment

In the library an expressible nucleotide sequence coding for one peptide is preferably found in different but similar vectors under the control of different promoters. Preferably the library comprises at least three primary vectors with an expressible nucleotide sequence coding for the same peptide under the control of three different promoters. More preferably the library comprises at least four primary vectors with an expressible nucleotide sequence coding for the same peptide under the control of four different promoters. More preferably the library comprises at least five primary vectors with an expressible nucleotide sequence coding for the same peptide under the control of five different promoters, such as comprises at lest six primary vectors with an expressible nucleotide sequence coding for the same peptide under the control of six different promoters, for example comprises at least seven primary vectors with an expressible nucleotide sequence coding for the same peptide under the control of seven different promoters, for example comprises at least eight primary vectors with an expressible nucleotide sequence coding for the same peptide under the control of eight different promoters, such as comprises at least nine primary vectors with an expressible nucleotide sequence coding for the same peptide under the control of nine different promoters, for example comprises at least ten primary vectors with an expressible nucleotide sequence coding for the same peptide under the control of ten different promoters.

The expressible nucleotide sequence coding for the same peptide preferably comprises essentially the same nucleotide sequence, more preferably the same nucleotide sequence.

By having a library with what may be termed one gene under the control of a number of different promoters in different vectors, it is possible to construct from the nucleotide library an array of combinations of genes and promoters. Preferably, one library comprises a complete or substantially complete combination such as a two dimensional array of genes and promoters, wherein substantially all genes are found under the control of substantially all of a selected number of promoters.

According to another embodiment of the invention the nucleotide library comprises combinations of expressible nucleotide sequences combined in different vectors with different spacer sequences and/or different intron sequences. Thus any one expressible nucleotide sequence may be combined in a two, three, four or five dimensional array with different promoters and/or different spacers and/or different introns and/or different terminators. The two, three, four or five dimensional array may be complete or incomplete, since not all combinations will have to be present.

The library may suitably be maintained in a host cell comprising prokaryotic cells or eukaryotic cells. Preferred prokaryotic host organisms may include but are not limited to Escherichia coli, Bacillus subtilis, Streptomyces lividans, Streptomyces coelicolor Pseudomonas aeruginosa, Myxococcus xanthus.

Yeast species such as Saccharomyces cerevisiae (budding yeast), Schizosaccharomyces pombe (fission yeast), Pichia pastoris, and Hansenula polymorpha (methylotropic yeasts) may also be used. Filamentous ascomycetes, such as Neurospora crassa and Aspergillus nidulans may also be used. Plant cells such as those derived from Nicotiana and Arabidopsis are preferred. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990).

### Concatemers

A concatemer is a series of linked units. In the present context a concatemer is used to denote a number of serially linked nucleotide cassettes, wherein at least two of the serially linked nucleotide units comprises a cassette having the basic structure

[rs₂-SP-PR-X-TR-SP-rs₁]

wherein
rs₁ and rs₂ together denote a restriction site,
SP individually denotes a spacer of at least two nucleotide bases,
PR denotes a promoter, capable of functioning in a cell,
X denotes an expressible nucleotide sequence,
TR denotes a terminator, and
SP individually denotes a spacer of at least two nucleotide bases.

Optionally the cassettes comprise an intron sequence between the promoter and the expressible nucleotide sequence and/or between the terminator and the expressible sequence.

The expressible nucleotide sequence in the cassettes of the concatemer may comprise a DNA sequence selected from the group comprising cDNA and genomic DNA.

According to one aspect of the invention, a concatemer comprises cassettes with expressible nucleotide from different expression states, so that non-naturally occurring combinations or non-native combinations of expressible nucleotide sequences are obtained. These different expression states may represent at least two different tissues, such as at least two organs, such as at least two species, such as at least two genera. The different species may be from at least two different phylae, such as from at least two different classes, such as from at least two different divisions, more preferably from at least two different sub-kingdoms, such as from at least two different kingdoms.

For example, the expressible nucleotide sequences may originate from eukaryots such as mammals such as humans, mice or whale, from reptiles such as snakes crocodiles or turtles, from tunicates such as sea squirts, from lepidoptera such as butterflies and moths, from coelenterates such as jellyfish, anenomes, or corals, from fish such as bony and cartilaginous fish, from plants such as dicots, e.g. coffee, oak or monocots such as grasses, lilies, and orchids; from lower plants such as algae and gingko, from higher fungi such as terrestrial fruiting fungi, from marine actinomycetes. The expressible nucleotide sequences may also originate from protozoans such as malaria or trypanosomes, or from prokaryotes such as E. coli or archaebacteria. Furthermore, the expressible nucleotide sequences may-originate from one or more preferably from more expression states from the species and genera listed in the table below.

| | |
|---|---|
| Bacteria | Streptomyces , Micromonospora, Norcadia, Actinomadura, Actinoplanes, Streptosporangium, Microbispora, Kitasatosporiam, Azobacterium, Rhizobium, Achromobacterium, Enterobacterium, Brucella, Micrococcus, Lactobacillus, Bacillus (B.t toxins), Clostridium (toxins), Brevibacterium, Pseudomonas, Aerobacter, Vibrio, Halobacterium, Mycoplasma, Cytophaga, Myxococcus |
| Fungi | Amanita muscaria (fly agarie, ibotenic acid, muscimol), Psilocybe (psilocybin) Physarium, Fuligo, Mucor, Phytophtora, Rhizopus, Aspergillus, Penicillium (penicillin), Coprinus, Phanerochaete, Acremonium (Cephalosporin), Trochoderma, Helminthosporium, Fusarium, Alternaria, Myrothecium, Saccharomyces |
| Algae | Digenea simplex (kainic acid, antihelminthic), Laminaria anqustata (laminine, hypotensive) |
| Lichens | Usnea fasciata (vulpinicacid, antimicrobial; usnic acid, antitumor) |
| Higher Plants | Artemisia (artemisinin), Coleus (forskolin), Desmodium (K channel agonist), Catharanthus (Vinca alkaloids), Digitalis (cardiac glycosides), Podophyllum (podophyllotoxin), Taxus (taxol), Cephalotaxus (homoharringtonine), Camptotheca (Camptothecin), Camellia sinensis (Tea), Cannabis indica, Cannabis sativa (Hemp), Erythroxylum coca (Coca), Lophophora williamsii (PeyoteMyristica fragrans |
| | (Nutmeg), Nicotiana, Papaver somniferum (Opium Poppy), Phalaris arundinacea (Reed canary grass) |
| Protozoa | Ptychodiscus brevis; Dinoflagellates (brevitoxin, cardiovascular) |
| Sponges | Microciona prolifera (ectyonin, antimicrobial) Cryptotethya cryta (D-arabino furanosides) |
| Coelenterata | Portuguese Man o War & other jellyfish and medusoid toxins. |
| Corals | Pseudoterogonia species (Pseudoteracins, anti-inflammatory), Erythropodium (erythrolides, anti-inflammatory) |
| Aschelminths | Nematode secretory compounds |
| Molluscs | Conus toxins, sea slug toxins, cephalapod neurotransmitters, squid inks |
| Annelida | Lumbriconereis heteropa (nereistoxin, insecticidal) |
| Arachnids | Dolomedes ("fishing spider" venoms) |
| Crustacea | Xenobalanus (skin adhesives) |
| Insects | Epilachna (mexican bean beetle alkaloids) |
| Spinunculida | Bonellia viridis (bonellin,neuroactive) |
| Bryozoans | Bugula neritina (bryostatins,anti cancer) |
| Echinoderms | Crinoid chemistry |
| Tunicates | Trididemnum solidum (didemnin, anti-tumor and anti-viral; Ecteinascidia turbinata ecteinascidins, anti-tumor) |
| Vertebrates | Eptatretus stoutii (eptatretin, cardioactive), Trachinus draco (proteinaceous toxins, reduce blood pressure, respiration and reduce heart rate). Dendrobatid frogs (batrachotoxins, pumiliotoxins, histrionicotoxins, and other polyamines); Snake venom toxins; Orinthorhynohus anatinus (duck-billed platypus venom), modified carotenoids, retinoids and steroids; Avians: histrionicotoxins, modified carotenoids, retinoids and steroids |

According to a preferred embodiment of the invention the concatemer comprises at least a first cassette and a second cassette, said first cassette being different from said second cassette. More preferably, the concatemer comprises cassettes, wherein substantially all cassettes are different. The difference between the cassettes may arise from differences between promoters, and/or expressible nucleotide sequences, and/or spacers, and/or terminators, and/or introns.

The number of cassettes in a single concatemer is largely determined by the host species into which the concatemer is eventually to be inserted and the vector through which the insertion is carried out The concatemer thus may comprise at least 10 cassettes, such as at least 15, for example at least 20, such as at least 25, for example at least 30, such as from 30 to 60 or more than 60, such as at least 75, for example at least 100, such as at least 200, for example at least 500, such as at least 750, for example at least 1000, such as at least 1500, for example at least 2000 cassettes.

Each of the cassettes may be laid out as described above.

Once the concatemer has been assembled or concatenated it may be ligated into a suitable vector. Such a vector may advantageously comprise an artificial chromosome. The basic requirements for a functional artificial chromosome have been described in US 4,464,472, the contents of which is hereby incorporated by reference. An artificial chromosome or a functional minichromosome, as it may also be termed must comprise a DNA sequence capable of replication and stable mitotic maintenance in a host cell comprising a DNA segment coding for centromere-like activity during mitosis of said host and a DNA sequence coding for a replication site recognized by said host.

Suitable artificial chromosomes include a Yeast Artificial Chromosome (YAC) (see e.g. Murray et al, Nature 305:189-193; or US 4,464,472), a mega Yeast Artificial Chromosome (mega YAC), a Bacterial Artificial Chromosome (BAC), a mouse artificial chromosome, a Mammalian Artificial Chromosome (MAC) (see e.g. US 6,133,503 or US 6,077,697), an Insect Artificial Chromosome (BUGAC), an Avian Artificial Chromosome (AVAC), a Bacteriophage. Artificial Chromosome, a Baculovirus Artificial Chromosome, a plant artificial chromosome (US 5,270,201), a BIBAC vector (US 5,977,439) or a Human Artificial Chromosome (HAC).

The artificial chromosome is preferably so large that the host cell perceives it as a "real" chromosome and maintains it and transmits it as a chromosome. For yeast and other suitable host species, this will often correspond approximately to the size of the smallest native chromosome in the species. For Saccharomyces, the smallest chromosome has a size of 225 Kb.

MACs may be used to construct artificial chromosomes from other species, such as insect and fish species. The artificial chromosomes preferably are fully functional stable chromosomes. Two types of artificial chromosomes may be used. One type, referred to as SATACs [satellite artificial chromosomes] are stable heterochromatic chromosomes, and the other type are minichromosomes based on amplification of euchromatin.

Mammalian artificial chromosomes provide extra-genomic specific integration sites for introduction of genes encoding proteins of interest,and permit megabase size DNA integration, such as integration of concatemers according to the invention.

According to another embodiment of the invention, the concatemer may be integrated into the host chromosomes or cloned into other types of vectors, such as a plasmid vector, a phage vector, a viral vector or a cosmid vector.

A preferable artificial- chromosome vector is one that is capable of being conditionally amplified in the host cell, e.g. in yeast. The amplification preferably is at least a 10 fold amplification. Furthermore, it is advantageous that the cloning site of the artificial chromosome vector can be modified to comprise the same restriction site as the one bordering the cassettes described above, i.e. RS2 and/or RS2'.

### Concatenation

Cassettes to be concatenated are normally excised from a vector either by digestion with restriction enzymes or by PCR. After excision the cassettes may be separated from the vector through size fractionation such as gel filtration or through tagging of known sequences in the cassettes. The isolated cassettes may then be joined together either through interaction between sticky ends or through ligation of blunt ends.

Single-stranded compatible ends may be created by digestion with restriction' enzymes. For concatenation a preferred enzyme for excising the cassettes would be a rare cutter, i.e. an enzyme that recognises a sequence of 7 or more nucleotides. Examples of enzymes that cut very rarely are the meganucleases, many of which are intron encoded, like e.g. I-Ceu I, I-Sce I, I-Ppo I, and PI-Psp I (see example 6d for more). Other preferred enzymes recognize a sequence of 8 nucleotides like e.g. Asc I, AsiS I, CciN I, CspB I, Fse I, MchA I, Not I, Pac I, Sbf I, Sda I, Sgf I, SgrA I, Sse232 I, and Sse8387 I, all of which create single stranded, palindromic compatible ends.

Other preferred rare cutters, which may also be used to control orientation of individual cassettes in the concatemer are enzymes that recognize non-palindromic sequences like e.g. Aar I, Sap I, Sfi I, Sdi I, and Vpa (see example 6c for more)..

Alternatively, cassettes can be prepared by the addition of restriction sites to the ends, e.g. by PCR or ligation to linkers (short synthetic dsDNA molecules). Restriction enzymes are continuously being isolated and characterised and it is anticipated that many of such novel enzymes can be used to generate single-stranded compatible ends according to the present invention.

It is conceivable that single stranded compatible ends can be made by cleaving the vector with synthetic cutters. Thus, a reactive chemical group that will normally be able to cleave DNA unspecifically can cut at specific positions when coupled to another molecule that recognises and binds to specific sequences. Examples of molecules that recognise specific dsDNA sequences are DNA, PNA, LNA, phosphothioates, peptides, and amides. See e.g. Armitage, B.(1998) Chem. Rev. 98: 1171-1200, who describes photocleavage using e.g. anthraquinone and UV light; Dervan P.B. & Bürli R.W. (1999) Curr. Opin. Chem. Biol. 3: 688-93 describes the specific binding of polyamides to DNA; Nielsen, P.E. (2001) Curr. Opin. Biotechnol. 12: 16-20 describes the specific binding of PNA to DNA, and Chemical Reviews special thematic issue: RNA/DNA Cleavage (1998) vol. 98 (3) Bashkin J.K. (ed.) ACS publications, describes several examples of chemical DNA cleavers.

Single-stranded compatible ends may also be created by using e.g. PCR primers including dUTP and then treating the PCR product with Uracil-DNA glycosylase (Ref: US 5,035,996) to degrade part of the primer. Alternatively, compatible ends can be created by tailing both the vector and insert with complimentary nucleotides using Terminal Transferase (Chang, LMS, Bollum TJ (1971) J Biol Chem 246:909).

It is also conceivable that recombination can be used to generate concatemers, e.g. through the modification of techniques like the Creator^{™} system (Clontech) which uses the Cre-loxP mechanism (Sauer B 1993 Methods Enzymol 225:890-900) to directionally join DNA molecules by recombination or like the Gateway^{™} system (Life Technologies, US 5,888,732) using lambda *att* attachment sites for directional recombination (Landy A 1989, Ann Rev Biochem 58:913). It is envisaged that also lambda cos site dependent systems can be developed to allow concatenation.

More preferably the cassettes may be concatenated without an intervening purification step through excision from a vector with two restriction enzymes, one leaving sticky ends on the cassettes and the other one leaving blunt ends in the vectors. This is the preferred method for concatenation of cassettes from vectors having the basic structure of [RS1-RS2-SP-PR-X-TR-SP-RS2'-RS1].

An alternative way of producing concatemers free of vector sequences would be to PCR amplify the cassettes from a single-stranded primary vector. The PCR product must include the restriction sites RS2 and RS2' which are subsequently cleaved by its cognate enzyme(s). Concatenation can then be performed using the digested PCR product, essentially without interference from the single stranded primary vector template or the small double stranded fragments, which have been cut from the ends.

The concatemer may be assembled or concatenated by concatenation of at least two cassettes of nucleotide sequences each cassette comprising a first sticky end, a spacer sequence, a promoter, an expressible nucleotide sequence, a terminator, a spacer sequence, and a second sticky end. A flow chart of the procedure is shown in figure 2a.

Preferably concatenation further comprises
starting from a primary vector

   [RS1-RS2-SP-PR-X-TR-SP-RS2'-RS1'],

   wherein X denotes an expressible nucleotide sequence,
   RS1 and RS1' denote restriction sites,
   RS2 and RS2' denote restriction sites different from RS1 and RS1',
   SP individually denotes a spacer sequence of at least two nucleotides,
   PR denotes a promoter,
   TR denotes a terminator,
   i) cutting the primary vector with the aid of at least one restriction enzyme specific for RS2 and RS2' obtaining cassettes having the general formula [rs₂-SP-PR-X-TR-SP-rs₁] wherein rs₁ and rs₂ together denote a functional restriction site RS2 or RS2',
   ii) assembling the cut out cassettes through interaction between rs₁ and

In this way at least 10 cassettes can be concatenated, such as at least 15, for example at least 20, such as at least 25, for example at least 30, such as from 30 to 60 or more than 60, such as at least 75, for example at least 100, such as at least 200, for example at least 500, such as at least 750, for example at least 1000; such as at least 1500, for example at least 2000.

According to an especially preferred embodiment, vector arms each having a RS2 or RS2' in one end and a non-complementary overhang or a blunt end in the other end are added to the concatenation mixture together with the cassettes described above to further simplify the procedure (see Fig. 2b). One example of a suitable vector for providing vector arms is disclosed in Fig. 7 TRP1, URA3, and HIS3 are auxotrophic marker genes, and AmpR is an E. coli antibiotic marker gene. CEN4 is a centromer and TEL are telomeres. ARS1 and PMB1 allow replication in yeast and E. coli respectively. BamH I and Asc I are restriction enzyme recognition sites. The nucleotide sequence of the vector is set forth in SEQ ID NO 4. The vector is digested with BamHI and AscI to liberate the vector arms, which are used for ligation to the concatemer.

The ratio of vector arms to cassettes determines the maximum number of cassettes in the concatemer as illustrated in figure 8. The vector arms preferably are artificial chromosome vector arms such as those described in Fig. 7.

It is of course also possible to add stopper fragments to the concatenation solution, the stopper fragments each having a RS2 or RS2' in one end and a non-complementary overhang or a blunt end in the other end. The ratio of stopper fragments to cassettes can likewise control the maximum size of the concatemer.

The complete sequence of steps to be taken when starting with the isolation of mRNA until inserting into an entry vector may include the following steps
i) isolating mRNA from an expression state,
ii) obtaining substantially full length cDNA corresponding to the mRNA sequences,
iii) inserting the substantially full length cDNA into a cloning site in a cassette in a primary vector, said cassette being of the general formula in 5'→3' direction:

   [RS1-RS2-SP-PR-CS-TR-SP-RS2'-RS1']

   wherein CS denotes a cloning site.

In preparation of the concatemer, genes may be isolated from different entry libraries to provide the desired selection of genes. Accordingly, concatenation may further comprise selection of vectors having expressible nucleotide sequences from at least two different expression states, such as from two different species. The two different species may be from two different classes, such as from two different divisions, more preferably from two different sub-kingdoms, such as from two different kingdoms.

As an alternative to including vector arms in the concatenation reaction it is possible to ligate the concatemer into an artificial chromosome selected from the group comprising yeast artificial chromosome, mega yeast artificial chromosome, bacterial artificial chromosome, mouse artificial chromosome, human artificial chromosome.

Preferably at least one inserted concatemer further comprises a selectable marker. The marker(s) are conveniently not included in the concatemer as such but rather in an artificial chromosome vector, into which the concatemer is inserted. Selectable markers generally provide a means to select, for growth, only those cells which contain a vector. Such markers are of two types: drug resistance and auxotrophy. A drug resistance marker enables cells to grow in the presence of an otherwise toxic compound. Auxotrophic markers allow cells to grow in media lacking an essential component by enabling cells to synthesise the essential component (usually an amino acid).

Illustrative and non-limiting examples of common compounds for which selectable markers are available with a brief description of their mode of action follow:

### Prokaryotic

- Ampicillin: interferes with a terminal reaction in bacterial cell wall synthesis. The resistance gene (bla) encodes beta-lactamase which cleaves the betalactam ring of the antibiotic thus detoxifying it.
- Tetracycline: prevents bacterial protein synthesis by binding to the 30S ribosomal subunit. The resistance gene (tet) specifies a protein that modifies the bacterial membrane and prevents accumulation of the antibiotic in the cell.
- Kanamycin: binds to the 70S ribosomes and causes misreading of messenger RNA. The resistant gene (nptH) modifies the antibiotic and prevents interaction with the ribosome.
- Streptomycin: binds to the 30S ribosomal subunit, causing misreading of messenger RNA. The resistance gene (Sm) modifies the antibiotic and prevents interaction with the ribosome.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.

### Eukaryotic

- Hygromycin: a aminocyclitol that inhibits protein synthesis by disrupting ribosome translocation and promoting mistranslation. The resistance gene (hph) detoxifies hygromycin -B- phosphorylation.
- Histidinol: cytotoxic to mammalian cells by inhibiting histidyl-tRNA synthesis in histidine free media. The resistance gene (hisD) product inactivates histidinol toxicity by converting it to the essential amino acid, histidine.
- Neomycin (G418): blocks protein synthesis by interfering with ribosomal functions. The resistance gene ADH encodes amino glycoside phosphotransferase which detoxifies G418.
- Uracil: Laboratory yeast strains carrying a mutated gene which encodes orotidine -5'- phosphate decarboxylase, an enzyme essential for uracil biosynthesis, are unable to grow in the absence of exogenous uracil. A copy of the wild-type gene (ura4+, S. pombe or URA3 S. cerevisiae) carried on the vector will complement this defect in transformed cells.
- Adenosine: Laboratory strains carrying a deficiency in adenosine synthesis may be complemented by a vector carrying the wild type gene, ADE 2.
- Amino acids: Vectors carrying the wild-type genes for LEU2, TRP 1, HIS 3 or LYS 2 may be used to complement strains of yeast deficient in these genes.
- Zeocin: this new bleomycin-family antibiotic intercalates into the DNA and cleaves it. The Zeocin resistance gene encodes a 13,665 dalton protein. This protein confers resistance to Zeocin by binding to the antibiotic and preventing it from binding DNA. Zeocin is effective on most aerobic cells and can be used for selection in mammalian cell lines, yeast, and bacteria.

### Transgenic-cells

In one aspect of the invention, the concatemers comprising the multitude of cassettes are introduced into a host cell, in which the concatemers can be maintained and the expressible nucleotide sequences can be expressed in a coordinated way. The cassettes comprised in the concatemers may be isolated from the host cell and re-assembled due to their uniform structure with -preferably-concatemer restriction sites between the cassettes.

The host cells selected for this purpose are preferably cultivable under standard laboratory conditions using standard culture conditions, such as standard media and protocols. Preferably the host cells comprise a substantially stable cell line, in which the concatemers can be maintained for generations of cell division. Standard techniques for transformation of the host cells and in particular methods for insertion of artificial chromosomes into the host cells are known.

It is also of advantage if the host cells are capable of undergoing meiosis to perform sexual recombination. It is also advantageous that meiosis is controllable through external manipulations of the cell culture. One especially advantageous host cell type is one where the cells can be manipulated through external manipulations into different mating types.

The genome of a number of species have already been sequenced more or less completely and the sequences can be found in databases. The list of species for which the whole genome has been sequenced increases constantly. Preferably the host cell is selected from the group of species, for which the whole genome or essentially the whole genome has been sequenced. The host cell should preferably be selected from a species that is well described in the literature with respect to genetics, metabolism, physiology such as model organism used for genomics research.

The host organism should preferably be conditionally deficient in the abilities to undergo homologous recombination. The host organism should preferably have a codon usage similar to that of the donor organisms. Furthermore, in the case of genomic DNA, if eukaryotic donor organisms are used, it is preferable that the host organism has the ability to process the donor messenger RNA properly, e.g., splice out introns.

The host cells can be bacterial, archaebacteria, or eukaryotic and can constitute a homogeneous cell line or mixed culture. Suitable cells include the bacterial and eukaryotic cell lines commonly used in genetic engineering and protein expression.

Preferred prokaryotic host organisms may include but are not limited to Escherichia coli, Bacillus subtilis, B licehniformis, B. cereus, Streptomyces lividans, Streptomyces coelicolor, Pseudomonas aeruginosa, Myxococcus xanthus. Rhodococcus, Streptomycetes, Actinomycetes, Corynebacteria, Bacillus, Pseudomonas, Salmonella, and Erwinia. The complete genome sequences of E. coli and Bacillus subtilis are described by Blattner et al., Science 277, 1454-1462 (1997); Kunst et al., Nature 390, 249-256 (1997)).

Preferred eukaryotic host organisms are mammals, fish, insects, plants, algae and fungi.

Examples of mammalian cells include those from, e.g., monkey, mouse, rat, hamster, primate, and human, both cell lines and primary cultures. Preferred mammalian host cells include but are not limited to those derived from humans, monkeys and rodents, such as chinese hamster ovary (CHO) cells, NIH/3T3, COS, 293, VERO, HeLa etc (see Kriegler M. in "Gene Transfer and Expression: A Laboratory Manual", New York, Freeman & Co. 1990), and stem cells, including non human embryonic stem cells and hemopoietic stem cells, zygotes, fibroblasts, lymphocytes, kidney, liver, muscle, and skin cells.

Examples of insect cells include baculo lepidoptera.

Examples of plant cells include maize, rice, wheat, cotton, soybean, and sugarcane. Plant cells such as those derived from Nicotiana and Arabidopsis are preferred

Examples of fungi include penicillium, aspergillus, such as Aspergillus nidulans, podospora, neurospora, such as Neurospora crassa, saccharomyces, such as Saccharomyces cerevisiae (budding yeast), Schizosaccharomyces, such as Schizosaccharomyces pombe (fission yeast), Pichia spp, such as Pichia pastoris, and Hansenula polymorpha (methylotropic yeasts).

In a preferred embodiment the host cell is a yeast cell, and an illustrative and not limiting list of suitable yeast host cells comprise: baker's yeast, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e.g. C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Pichia pastoris, Kluyveromyces, Hansenula, Kloeckera, Pichia, Pachysolen spp., or Torulopsis bombicola.

The choice of host will depend on a number of factors, depending on the intended use of the engineered host, including pathogenicity, substrate range, environmental hardiness, presence of key intermediates, ease of genetic manipulation, and likelihood of promiscuous transfer of genetic information to other organisms. Particularly advantageous hosts are E. coli, lactobacilli, Streptomycetes, Actinomycetes, Saccharomyces and filamentous fungi.

In any one host cell it is possible to make all sorts of combinations of expressible nucleotide sequences from all possible sources. Furthermore, it is possible to make combinations of promoters and/or spacers and/or introns and/or terminators in combination with one and the same expressible nucleotide sequence.

Thus in any one cell there may be expressible nucleotide sequences from two different expression states. Furthermore, these two different expression states may be from one species or advantageously from two different species. Any one host cell may also comprise expressible nucleotide sequences from at least three species, such as from at least four, five, six, seven, eight, nine or ten species, or from more than 15 species such as from more than 20 species, for example from more than 30, 40 or 50 species, such as from more than 100 different species, for example from more than 300 different species, such as form more than 500 different species, for example from more than 1000 different species, thereby obtaining combinations of large numbers of expressible nucleotide sequences from a large number of species. In this way potentially unlimited numbers of combinations of expressible nucleotide sequences can be combined across different expression states. These different expression states may represent at least two different tissues, such as at least two organs, such as at least two species, such as at least two genera. The different species may be from at least two different phylae, such as from at least two different classes, such as from at least two different divisions, more preferably from at least two different sub-kingdoms, such as from at least two different kingdoms.

Any two of these species may be from two different classes, such as from two different divisions, more preferably from two different sub-kingdoms, such as from two different kingdoms. Thus expressible nucleotide sequences may be combined from a eukaryot and a. prokaryot into one and the same cell.

According to another embodiment of the invention, the expressible nucleotide sequences may be from one and the same expression state. The products of these sequences may interact with the products of the genes in the host cell and form new enzyme combinations leading to novel biochemical pathways. Furthermore, by putting the expressible nucleotide sequences under the control of a number of promoters it becomes possible to switch on and off groups of genes in a coordinated manner. By doing this with expressible nucleotide sequences from only one expression states, novel combinations of genes are also expressed.

The number of concatemers in one single cell may be at least one concatemer per cell, preferably at least 2 concatemers per cell, more preferably 3 per cell, such as 4 per cell, more preferably 5 per cell, such as at least 5 per cell, for example at least 6 per cell, such as 7, 8, 9 or 10 per cell, for example more than 10 per cell. As described above, each concatemer may preferably comprise up to 1000 cassettes, and it is envisages that one concatemer may comprise up to 2000 cassettes. By inserting up to 10 concatemers into one single cell, this cell may thus be enriched with up to 20,000 heterologous expressible genes, which under suitable conditions may be turned on and off by regulation of the regulatable promoters.

Often it is more preferable to provide cells having anywhere between 10 and 1000 heterologous genes, such as 20-900 heterologous genes, for example 30 to 800 heterologous genes, such as 40 to 700 heterologous genes, for example 50 to 600 heterologous genes, such as from 60 to 300 heterologous genes or from 100 to 400 heterologous genes which are inserted as 2 to 4 artificial chromosomes each containing one concatemer of genes. The genes may advantageously be located on 1 to 10 such as from 2 to 5 different concatemers in the cells. Each concatemer may advantageously comprise from 10 to 1000 genes, such as from 10 to 750 genes, such as from 10 to 500 genes, such as from 10 to 200 genes, such as from 20 to 100 genes, for example from 30 to 60 genes, or from 50 to 100 genes.

The concatemers may be inserted into the host cells according to any known transformation technique, preferably according to such transformation techniques that ensure stable and not transient transformation of the host cell. The concatemers may thus be inserted as an artificial chromosome which is replicated by the cells as they divide or they may be inserted into the chromosomes of the host cell. The concatemer may also be inserted in the form of a plasmid such as a plasmid vector, a phage vector, a viral vector, a cosmid vector, that is replicated by the cells as they divide. Any combination of the three insertion methods is also possible. One or more concatemers may thus be integrated into the chromosome(s) of the host cell and one or more concatemers may be inserted as plasmids or artificial chromosomes. One or more concatemers may be inserted as artificial chromosomes and one or more may be inserted into the same cell via a plasmid.

### Examples

### Example 1

In the examples 1-3 an Asc1 site was introduced into the EcoR1 site in pYAC4 (Sigma, Burke DT et al. 1987, Science vol 236, p 806), so that sticky ends match the Asc1 site(= RS2 in general formula of this patent) of the cassettes in pEVE vectors

### Preparation of EVACs (EVolvable Artificial Chromosomes) including size fractioning

### preparation of pYAC4-Asc arms

1. inoculate 150 ml of LB (sigma) with a single colony of E. coli DH5α containing pYAC4-Asc
2. grow to OD600 ~ 1, harvest cells and make plasmid preparation
3. digest 100µg pYAC4-Asc w. BamH1 and Asc1
4. dephosphorylate fragments and heat inactivate phosphatase(20 min, 80 C)
5. purify fragments(e.g. Qiaquick Gel Extraction Kit)
6. run 1 % agarose gel to estimate amount of fragment

### Preparation of expression cassettes

1. take 100 µg of plasmid preparation from each of the following libraries
   a) pMA-CAR
   b) pCA-CAR
   c) Phaffia cDNA library
   d) Carrot cDNA library
2. digest w. Srf1 (10 units/prep, 37C overnight)
3. dephosphorylate (10 units/prep, 37C, 2h)
4. heat inactivate 80C, 20 min
5. concentrate and change buffer (precipitation or ultra filtration),
6. digest w. Asc1. (10 units/prep, 37 C, overnight)
7. adjust volume of preps to 100 µL

### preparation of EVACs

Different types of EVACs have been made by varying the ratio of the different libraries that goes into the ligation reaction.

| | **pMA-CAR** | **pCA-CAR** | **Phaffia cDNA** | **Carrot cDNA** |
|---|---|---|---|---|
| **EVAC** | | | | |
| A | 40% | 40% | 10% | 10% |
| B | 25% | 25% | 25% | 25% |

1. add ~100 ng arms of pYAC4-Asc /100 µg of cassette mixture
2. concentrate to < 33.5 µL
3. add 2.5 units of T4 DNA-ligase + 4 µL 10x ligase buffer. Adjust to 40 µL
4. ligate 3 h, 16 C
5. stop reaction by adding 2 µL of 500 mM EDTA
6. bring reaction volume to 125 µL, add 25 µL loading mix, heat at 60C for 5 min
7. distribute evenly in 10 wells of a 1 % LMP agarose gel
8. run pulsed field gel (CHEF III, 1% LMP agarose, % strength TBE (BioRad), angle 120, temperature 12 C, voltage 5.6V/cm, switch time ramping 5 - 25 s, run time 30 h)
9. stain part of the gel that contains molecular weight markers + 1 sample lane for quality check
10. cut remaining 9 sample lanes corresponding to mw. 97-194 kb(fraction 1); 194 - 291 kb(fraction 2); 291-365 kb(fraction 3) from the gel
11. agarase gel in high NaCl agarase buffer. 1 u agarase / 100µg gel. 40C 3 h
12. concentrate preparation to < 20 µL
13. transform suitable yeast strain w. preparation using alkali/cation transformation
14. plate on selective minimal media plates
15. incubate 30 C for 4-5 days
16. pick colonies
17. analyse colonies

### Example 2

### Preparation of EVACs (EVolvable Artificial Chromosomes) with direct transformation

### preparation of pYAC4-Asc arms

1. inoculate 150 ml of LB with a single colony of DH5α containing pYAC4-Asc
2. grow to OD600 ~ 1, harvest cells and make plasmid preparation
3. digest 100µg pYAC4-Asc w. BamH1 and Asc1
4. dephosphorylate fragments and heat inactivate phosphatase(20 min, 80 C)
5. purify fragments(e.g. Qiaquick Gel Extraction Kit)
1. run 1 % agarose gel to estimate amount of fragment

### Preparation of expression cassettes

1. take 100 µg of plasmid preparation from each of the following libraries
   e) pMA-CAR
   f) pCA-CAR
   g) Phaffia cDNA library
   h) Carrot cDNA library
2. digest w. Srf1 (10 units/prep, 37C overnight)
3. dephosphorylate (10 units/prep, 37C, 2h)
4. heat inactivate 80C, 20 min
5. concentrate and change buffer (precipitation or ultra filtration),
6. digest w. Asc1. (10 units/prep, 37 C, overnight)
7. adjust volume of preps to 100 µL

### preparation of EVACs

Different types of EVACs have been made by varying the ratio of the different libraries that goes into the ligation reaction.

| | **pMA CAR** | **pCA-CAR** | **Phaffia cDNA** | **Carrot cDNA** |
|---|---|---|---|---|
| **EVAC** | | | | |
| A | 40% | 40% | 10% | 10% |
| B | 25% | 25% | 25% | 25% |

1. concentrate to < 32 µL
2. add 1 unit of T4 DNA-ligase + 4 µL 10x ligase buffer. Adjust to 40 µL
3. ligate 2 h, 16 C
4. stop reaction by adding 2 µL of 500 mM EDTA, heat inactivate 60C, 20 min
5. bring reaction volume to 500 µL with dH₂O, concentrate to 30 µL
6. add 10 U Asc1, 4 µL 10X Asc1 buffer, bring to 40 µL
7. incubate at 37C for 1 h (alternatively 15 min 30 min)
8. heat inactivate 60C, 20 min
9. add 2 µg YAC4-Asc arms, 1 U T4 DNA ligase, 10 µL 10X ligase buffer, bring to 100 µL
10. incubate ON, 16C
11. add water to 500 uL
12. concentrate to 25 µL
13. transform suitable yeast strain w. preparation using alkali/cation transformation or other suitable transformation method
14. plate on selective minimal media plates
15. incubate 30 C for 4-5 days
16. pick colonies
17. analyse colonies

### Example 3

### Preparation of EVACs (EVolvable Artificial Chromosomes) (Small scale preparation)

### Preparation of expression cassettes

1. inoculate 5 ml of LB-medium (Sigma) with library inoculum corresponding to a 10+ fold representation of library. Grow overnight
2. make plasmid miniprep from 1.5 ml of culture (E.g. Qiaprep spin miniprep kit)
3. digest plasmid w. Srf 1
4. dephosphorylate fragments and heat inactivate phosphatase(20 min, 80 C)
5. digest w. Asc1
6. run 1/10, of reaction in 1 % agarose to estimate amount of fragment

### preparation of pYAC4-Asc arms

1. inoculate 150 ml of LB with a single colony of E. coli DH5α containing pYAC4-Asc
2. grow to OD600 ~ 1, harvest cells and make plasmid preparation
3. digest 100µg pYAC4-Asc w. BamH1 and Asc1
4. dephosphorylate fragments and heat inactivate phosphatase (20 min, 80 C)
5. purify fragments(E.g. Qiaquick Gel Extraction Kit)
6. run 1 % agarose gel to estimate amount of fragment

### preparation of EVACs

1. mix expression cassette fragments with YAC-arms so that cassette/arm ration is ~1000/1
2. if needed concentrate mixture(use e.g. Microcon YM30) so fragment concentration > 75 ng/µL reaction
3. add 1 U T4 DNA ligase, incubate 16C, 1-3 h . Stop reaction by adding 1 µL of 500 mM EDTA
4. run pulsed field gel (CHEF III, 1% LMP agarose, ½ strength TBE, angle 120, temperature 12 C, voltage 5.6V/cm, switch time ramping 5 - 25 s, run time 30 h) Load sample in 2 lanes.
5. stain part of the gel that contains molecular weight markers
6. cut sample lanes corresponding to mw. 100 - 200 kb
7. agarase gel in high NaCl agarase buffer. 1 U agarase / 100 mg gel
8. concentrate preparation to < 20 µL
9. transform suitable yeast strain w. preparation using electroporation
10. plate on selective minimal media plates
11. incubate 30 C for 4-5 days
12. pick colonies

### Example 4: cDNA libraries used in the production of EVACs

*1. Daucus carota,* carrot root library:
   - Full length
   - Oligo dT primed, directional cDNA library
   - cDNA library made using a pool of 3 Evolva EVE 4, 5 & 8 vectors (Fig. 4, 5, 6)
   - Number of independent clones: 41.6 x 10⁶
   - Average size: 0.9 - 2.9 kb
   - Number of different genes present: 5000 -10000
*2. Xanthophyllomyces dendrorhous,* (yeast), hole organism library
   - Full length
   - Oligo dT primed, directional cDNA library
   - cDNA library made using a pool of 3 Evolva EVE 4, 5 & 8 vectors (Fig. 4, 5, 6)
   - Number of independent clones: 48.0 x 10⁶
   - Average size: 1.0 - 3.8 kb
   - Number of different genes present: 5000 -10000
3. Target carotenoid gene cDNA library
   - Full length and normalised
   - Directional cDNA cloning
   - Library made by cloning each gene individually in 2 Evolva EVE 4, 5 & 8 vectors (Fig. 4, 5, 6)
   - Number of different genes: 48
   - Species and genes used:
      - Gentiana sp., ggps, psy, pds, zds, lcy-b, Icy-e, bhy, zep
      - Rhodobacter capsulatus, idi, crtC, crtF
      - Erwinia uredovora, crtE, crtB, crtl, crtY, crtZ
      - Nostoc anabaena, zds
      - Synechococcus PCC7942, pds
      - Erwinia herbicola, crtE, crtB, crtl, crtY, crtZ
      - Staphylococcus aureus, crtM, crtN
      - Xanthophyllomyces dendrorhous, crtl, crtYb
      - Capsicum annuum, ccs, crtL
      - Nicotiana tabacum, crtL, bchy
      - Prochlorococcus sp., lcy-b, lcy-e
      - Saccharomyces cerevisiae, idi
      - Corynebacterium sp., crtl, crtYe, crtYf, crtEb
      - Lycopersicon esculentum, psy-1
      - Neurospora crassa, al1

### Example 5: Transformation of EVACs

### Example 5a: Transformation

1. Inoculate a single colony into 100 ml YPD broth and grow with aeration at 30°C to mid log, 2 x 10⁶ to 2 x 10⁷ cells/ml.
2. Spin to pellet cells at 400 x g for 5 minutes; discard supernatant.
3. Resuspend cells in a total of 9 ml TE, pH 7.5. Spin to pellet cells and discard supernatant.
4. Gently resuspend cells in 5 ml 0.1 M Lithium/Cesium Acetate solution, pH 7.5.
5. Incubate at 30°C for 1 hour with gentle shaking.
6. Spin at 400 x g for 5 minutes to pellet cells and discard supernatant.
7. Gently resuspend in 1 ml TE, pH 7.5. Cells are now ready for transformation.
8. In a 1.5 ml tube combine:
   - 100 µl yeast cells
   - 5 µl Carrier DNA (10 mg/ml)
   - 5 µl Histamine Solution
   - 1/5 of an EVAC preparation in a 10 µl volume (max). (One EVAC preparation is made of 100 µg of concatenation reaction mixture)
9. Gently mix and incubate at room temperature for 30 minutes.
10. In a separate tube, combine 0.8 ml 50% (w/v) PEG 4000 and 0.1 ml TE and 0.1 ml of 1 M LiAc for each transformation reaction. Add 1 ml of this. PEG/TE/LiAc mix to each transformation reaction. Mix cells into solution with gentle pipetting.
11. Incubate at 30°C for 1 hour.
12. Heat shock at 42°C for 15 minutes; cool to 30°C.
13. Pellet cells in a microcentrifuge at high speed for 5 seconds and remove supernatant.
14. Resuspend in 200 µl of rich media and plate in appropriate selective media
15. Incubate at 30°C for 48-72 hours until transformant colonies appear.

### Example 5b: Transformation of EVACs using electroporation

100 ml of YPD is inoculated with one yeast colony and grown to OD₆₀₀ = 1.3 to 1.5. The culture is harvested by centrifuging at 4000 x g and 4°C. The cells are resuspended in 16 ml sterile H₂O. Add 2 ml 10 x TE buffer, pH 7.5 and swirl to mix. Add 2 ml 10 × lithium acetate solution (1 M, pH 7.5) and swirl to mix. Shake gently, 45 min at 30°C. Add 1.0 ml 0.5 M DTE while swirling. Shake gently 15 min at 30°C. The yeast suspension is diluted to 100 ml with sterile water. The cells are washed and concentrated by centrifuging at 4000 x g, resuspending the pellet in 50 ml ice-cold sterile water, centrifuging at 4000 x g, resuspending the pellet in 5 ml ice-cold sterile water, centrifuging at 4000 x g and resuspending the pellet in 0.1 ml ice-cold sterile 1 M sorbitol. The electroporation was done using a *Bio-Rad Gene Pulser.* In a sterile 1.5-ml microcentrifuge tube 40 µl concentrated yeast cells were mixed with 5 µl 1:10 diluted EVAC preparation. The yeast-DNA mix is transferred to an ice-cold 0.2-cm-gap disposable electroporation cuvette and pulsed at 1.5 kV, 25 µF, 200 Ω. 1 ml ice-cold 1 M sorbitol is added to the cuvette to recover the yeast. Aliquots are spread on selective plates containing 1 M sorbitol. Incubate at 30°C until colonies appear.

### Example 6: Rare restriction enzymes with recognition sequence and cleavage points

In this example, rare restriction enzymes are listed together with their recognition sequence and cleavage points. (^) indicates cleavage points 5'-3' sequence and (_) indicates cleavage points in the complementary sequence.
W=AorT; N=A,C,G,orT

| 6a) | Unique, palindromic overhang |
|---|---|
| AscI | GG^CGCG_CC |
| AsiSI | GCG_AT^CGC |
| CciNI | GC^GGCC_GC |
| CspBI | GC^GGCC_GC |
| FseI | GG_CCGG^CC |
| MchAI | GC^GGCC_GC |
| NotI | GC^GGCC_GC |
| PacI | TTA_AT^TAA |
| SbfI | CC TGCA^GG |
| SdaI | CC_TGCA^GG |
| SgfI | GCG_AT^CGC |
| SgrAI | CR^CCGG_YG |
| Sse232I | CG^CCGG_CG |
| Sse8387I | CC_TGCA^GG |

| 6b) | No overhang |
|---|---|
| BstRZ246I | ATTT^AAAT |
| BstSWI | ATTT^AAAT |
| MspSWI | ATTT^AAAT |
| MssI | GTTT^AAAC |
| PmeI | GTTT^AAAC |
| SmiI | ATTT^AAAT |
| SrfI | GCCC^GGGC |
| SwaI | ATTT^AAAT |

| 6c) | Non-palindromic and/or variable overhang |
|---|---|
| AarI | CACCTGCNNNN^NNNN_ |
| AbeI | CC^TCA_GC |
| AloI | ^NNNNN_NNNNNNNGAACNNNNNNTCCNNNNNNN_NNNNN^ |
| BaeI | ^NNNNN_NNNNNNNNNNACNNNNGTAYCNNNNNNN_NNNNNA^ |
| BbvCI | CC^TCA_GC |
| CpoI | CG^GWC_CG |
| CspI | CG^GWC_CG |
| Pf127I | RG^GWC_CY |
| PpiI | ^NNNNN_NNNNNNNGAACNNNNNCTCNNNNNNNN_NNNNN^ |
| PpuMI | RG^GWC_CY |
| PpuXI | RG^GWC_CY |
| Psp5II | RG^GWC_CY |
| PspPPI | RG^GWC_CY |
| RsrII | CG^GWC_CG |
| Rsr2I | CG^GWC_CG |
| SanDI | GG^GWC_CC |
| SapI | GCTCTTCN^NNN_ |
| SdiI | GGCCN_NNN^NGGCC |
| SexAI | A^CCWGG_T |
| SfiI | GGCCN_NNN^GGCC |
| Sse1825I | GG^GWC_CC |
| Sse8647I | AG^GWC_CT |
| VpaK32I | GCTCTTCN^NNN_ |

| 6d) | Meganucleases |
|---|---|
| I-Sce I | TAGGGATAA_CAGG^GTAAT |
| I-Ceu I | ACGGTC_CTAA^GGTAG |
| I-Cre I | AAACGTC_GTGA^GACAGTTT |
| I-Sce II | GGTC_ACCC^TGAAGTA |
| I-Sce III | GTTTTGG_TAAC^TATTTAT |
| Endo. Sce I | GATGCTGC_AGGC^ATAGGCTTGTTTA |
| PI-Sce I | GG_GTGC^GGAGAA |
| PI-Psp I | TGGCAAACAGCTA_TTAT^GGGTATTATGGGT |
| I-Ppo I | CTCTC_TTAA^GGTAG |
| HO | TTTCCGC_AACA^GT |
| I-Tev I | NN_NN^NNTCAGTAGATGTTTTTCTTGGTCTACCGTTT |

More meganucleases have been identified, but their precise sequence of recognition has not been determined, see e.g. www.meganuclease.com

### Example 7: Concatemer size limitation experiments (use of stoppers)

### Materials used:

pYAC4 (Sigma. Burke et al. 1987, science, vol 236, p 806) was digested w. EcoR1 and BamH1 and dephosphorylated
pSE420 (invitrogen) was linearised using EcoR1 and used as the model fragment for concatenation.
T4 DNA ligase (Amersham-pharmacia biotech) was used for ligation according to manufacturers instructions.

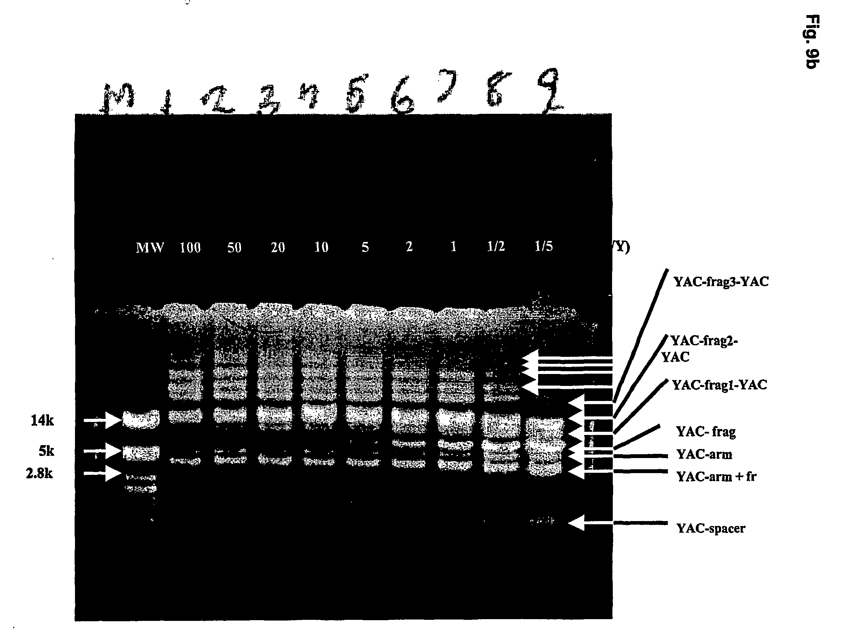

Method: Fragments and arms were mixed in the ratios(concentrations are arbitrary units) indicated on figures 9a and 9b. Ligation was allowed to proceed for 1 h at 16C. Reaction was stopped by the addition of 1 µL 500 mM EDTA. Products were analysed by standard agarose GE (1 % agarose, % strength TBE) or by PFGE(CHEF III, 1% LMP agarose, ½ strength TBE, angle 120, temperature 12 C, voltage 5.6V/cm, switch time ramping 5 - 25 s, run time 30 h)

The results are shown in Figure 9, wherein it is shown that the size of concatemers is proportional to the ratio of cassettes per YAC arms.

### Example 8: Integration of expression cassettes into artificial chromosomes

Integration of expression cassettes into YAC12 was done essentially as done by Sears D.D., Hieter P., Simchen G., Genetics, 1994, 138, 1055-1065.

An AscI site was introduced into the Bgl II site of the integration vectors pGS534 and pGS525.

A β-galactosidase gene, as well as crtE, crtB, crtI and crtY from *Erwinia Uredovora* were cloned into pEVE4. These expression cassettes were ligated into AscI of the modified integration vectors pGS534 and pGS525.

Linearised pGS534 and pGS525 containing the expression cassettes were transformed into haploid yeast strains containing the appropriate target YAC which carries the Ade" gene. Red Ade- transformants were selected (the parent host strain is red due to the ade2-101 mutation).

Additional confirmation of correct integration of the β-gatactosidase expression cassette was done using a β-galactosidase assay.

### Example 9: Re-transformation of cells that already contain Artificial chromosomes to obtain at least 2 artificial chromosomes per cell

Yeast strains containing YAC12, Sears D.D., Hieter P., Simchen G., Genetics, 1994, 138, 1055-1065 were transformed with EVACs following the protocol described in example 4a. The transformed cells were plated on plates that select for cells that contained both YAC12 and EVACs.

### Example 10: Example of different expression patterns "phenotypes" obtained using the same yeast clones under different expression conditions:

Colonies were picked with a sterile toothpick and streaked sequentially onto plates corresponding to the four repressed and/or induced conditions (-Ura/-Trp, -Ura/-Trp/-Met, -Ura/-Trp/+200 µM Cu₂SO₄, -Ura/-Trp/-Met/+200 µM Cu₂SO₄). 20 mg adenin was added to the media to suppress the ochre phenotype.

## Claims

1. A nucleotide concatemer comprising in the 5'→3' direction a cassette of nucleotide sequence of the general formula
[rs₂-SP-PR-X-TR-SP-rs₁]ₙ
wherein
rs₁ and rs₂ together denote a functional restriction site,
SP individually denotes a spacer of at least two nucleotide bases.
PR denotes a promoter, capable of functioning in a cell,
X denotes an expressible nucleotide sequence,
TR denotes a terminator, and
SP individually denotes a spacer of at least two nucleotide bases, and
n ≥ 2. and
wherein at least a first cassette is different from a second cassette.

2. The concatemer according to claim 1. wherein the expressible nucleotide sequence comprises a DNA sequence selected from the group comprising cDNA, genomic DNA.

3. The concatemer according to any of the preceding claims 1 to 2, wherein the rs₁-rs₂ restriction site of at least two cassettes are recognised by the same restriction enzyme, more preferably are identical.

4. The concatemer according to any of claims 1 to 3, wherein at least one cassette comprises an intron between the promoter and the expressible nucleotide sequence, more preferably substantially all cassettes comprise an intron between the promoter and the expressible nucleotide sequence.

5. The concatemer according to any of the preceding claims 1 to 4 comprised in an artificial chromosome.

6. The concatemer according to claim 5, wherein the artificial chromosome is selected from the group comprising a Yeast Artificial Chromosome, a mega Yeast Artificial Chromosome, a Bacterial Artificial Chromosome, a mouse artificial chromosome, a Mammalian Artificial Chromosome, an Insect Artificial Chromosome, an Avian Artificial Chromosome, a Bacteriophage Artificial Chromosome, a Baculovirus Artificial Chromosome, or a Human Artificial Chromosome.

7. The contatemer according to any of the preceding claims, wherein a restriction site comprises at least 6 bases.

8. A method for concatenation comprising the steps of concatenating at least two cassettes of nucleotide sequences each cassette comprising a first sticky end, a spacer sequence, a promoter, an expressible nucleotide sequence, a terminator, a spacer sequence and a second sticky end, wherein the method further comprises
starting from a primary vector
[RS1-RS2-SP-PR-X-TR-SP-RS2'-RS1']
wherein X denotes an expressible nucleotide sequence,
RS1 and RS1' denote restriction sites,
RS2 and RS2' denote restriction sites different from RS1 and RS1',
SP individually denotes a spacer sequence of at least two nucleotides,
PR denotes a promoter,
TR denotes a terminator,
i) cutting the primary vector with the aid of at least one restriction enzyme specific for RS2 and RS2' obtaining cassettes having the general formula [rs₂-SP-PR-X-TR-SP-rs₁] wherein rs₁ and rs₂ together denote a functional restriction site RS2 or RS2', 11) assembling the cut out cassettes through interaction between rs₁ and rs₂.

9. The method according to claim 8, further comprising addition of vector arms each having a RS2 or RS2' in one end and a non-complementary overhang or a blunt end in the other end.

10. The method according to claim 8, further comprising addition of stopper fragments, the stopper fragments each having a RS2 or RS2' in one end and a non-complementary overhang or a blunt end in the other end.

11. The method according to claim 10, further comprising ligating vector arms to the stopper fragments.

12. The method according to claim 8, further comprising
i) isolating mRNA from an expression state,
ii) obtaining substantially full length cDNA clones corresponding to the mRNA sequences,
iii) inserting the substantially full length cDNA clones into a cloning site in a cassette in a primary vector, said cassette being of the general formula in 5'→3' direction:
[RS1 -RS2-SP-PR-CS-TR-SP-RS2'-RS1']
wherein CS denotes a cloning site.

13. The method according to claim 8, wherein RS1 and RS1' are identical, and wherein RS2 and RS2' are identical.

14. The method according to any of the claims 8 to 13, whereby the concatemer is ligated into an artificial chromosome selected from the group comprising yeast artificial chromosome, mega yeast artificial chromosome, bacterial artificial chromosome, mouse artificial chromosome, human artificial chromosome.

15. The method according to any of the preceding claims 8 to 14, whereby RS2 and RS2' in at least two cassettes are cleaved by one restriction enzyme, preferably RS2 and RS2' in substantially all cassettes are cleaved by one restriction enzyme

16. The method according to any of the preceding claims 8 to 15, wherein RS2 and RS2' comprise more than 7 bases.

17. An isolated cell comprising at least one concatemer of individual oligonucleotide cassettes, each concatemer comprising oligonucleotide of the following formula in 5'→3' direction:
[rs₂-SP-PR-X-TR-SP-rs_{1]n}
wherein
rs₁ and rs₂ together denote a restriction site,
SP individually denotes a spacer of at least two nucleotide bases,
PR denotes a promoter, capable of functioning in the cell,
X denotes an expressible nucleotide sequence,
TR denotes a terminator, and
SP individually denotes a spacer of at least two nucleotide bases,
wherein n ≥ 2, and
wherein at least two expressible nucleotide sequences are from different expression states.

18. An isolated cell comprising at least one concatemer of individual oligonucleotide cassettes, each concatemer comprising oligonucleotide of the following formula in 5'→3' direction:
[rs₂-SP-PR-X-TR-SP-rs₁]ₙ
wherein
rs₁ and rs₂ together denote a restriction site,
SP individually denotes a spacer of at least two nucleotide bases,
PR denotes a promoter, capable of functioning in the cell,
X denotes an expressible nucleotide sequence,
TR denotes a terminator, and
SP individually denotes a spacer of at least two nucleotide bases,
wherein n ≥ 2, and
wherein rs₁-rs₂ in at least two cassettes is recognised by the same restriction enzyme.

19. The cell according to claim 17 or 18, wherein substantially all rs₁-rs₂ sequences are recognised by the same restriction enzyme, more preferably wherein substantially all rs₁-rs₂ sequences are substantially identical.

20. The cell according to claim 17 or 18, being a yeast cell selected from the group comprising baker's yeast, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (e.g. C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Kluyveromyces, Hansenula, Kloeckera, Pichia, Pachysolen spp., or Torulopsis bombicola.

21. The cell according to any of the preceding claims 17 to 20 wherein at least one concatemer, preferably substantially all concatemers is/are concatemer/s according to claims 1 to 7.

22. An artificial chromosome comprising at least one nucleotide concatemer, the concatemer comprising in the 5'→3' direction cassettes of nucleotide sequences of the general formula
[rs₂-SP-PR-X-TR-SP-rs₁]ₙ
wherein
rs₁ and rs₂ together denote a restriction site,
SP denotes a spacer of at least two nucleotide bases,
PR denotes a promoter, capable of functioning in a cell,
X denotes an expressible nucleotide sequence,
TR denotes a terminator, and
SP denotes a spacer of at least two nucleotide bases, and
n≥2.

23. The artificial chromosome, wherein said concatemer is the concatemer according to any of claims 1 to 7.

24. An isolated cell comprising at least one artificial chromosome according to any of claims 22 and 23.

25. An in vitro method for producing a transgenic cell comprising inserting into a host cell a concatemer according to any of claims 1 to 7.

26. A primary vector comprising a nucleotide sequence cassette of the general formula in 5'→3' direction:
[RS1-RS2-SP-PR-CS-TR-SP-RS2'-RS1']
wherein
RS1 and RS1' denote restriction sites,
RS2 and RS2' denotes restriction sites different from RS1 and RS1'.
SP individually denotes a spacer sequence of at least two nucleotides,
PR denotes a promoter,
CS denotes a cloning site,
TR denotes a terminator.

27. The vector according to claim 26, wherein the nucleotide sequence is a DNA sequence.

28. The vector according to any of the preceding claims 26 to 27, further comprising an intron sequence between the promoter and the cloning site and/or between the cloning site and the terminator.

29. The vector according to any of the preceding claims 26 to 28. wherein the cloning site comprises an expressible nucleotide sequence.

30. The vector according to any of the preceding claims 26 to 29, wherein RS2 and RS2' are identical.

31. The vector according to any of the preceding claims 26 to 30, wherein RS1 and RS1' are identical.

32. The vector according to any of the preceding claims 26 to 31, further comprising a spacer sequence between TR and RS2'.

33. The vector according to any of the preceding claims 26 to 32, wherein the promoter is an externally controllable promoter.

34. The vector according to any of the preceding claims 26 to 33, wherein the primary vector comprising the cassette is a plasmid vector having a high copy number, being capable of being propagated in E. coli, and having a selectable marker for maintenance in E. coli.

35. The concatemer according to any of the preceding claims 1 to 7, wherein at least one cassette comprise the cassette from a primary vector according to claims 26 to 34, more preferably substantial all cassettes comprise the cassette from a primary vector according to Claims 26 to 34.

36. A method of preparing a primary vector comprising
inserting an expressible nucleotide sequence into a cloning site in a primary vector comprising a cassette, the cassette comprising a nucleotide sequence of the general formula in 5'→3' direction:
[RS1-RS2-SP-PR-CS-TR-SP-RS2'-RS1']
wherein
RS1 and RS1' denote restriction sites,
RS2 and RS2' denotes restriction sites different from RS1 and RS1',
SP individually denotes a spacer sequence of at least two nucleotides,
PR denotes a promoter,
CS denotes a cloning site,
TR denotes a terminator.

37. The method according to claim 36, further comprising
isolating total mRNA from an expression state, and
obtaining full length cDNA for insertion into the vector.

38. The method according to claim 37, further comprising selection of cDNA to obtain substantially full length cDNA.

39. A nucleotide library comprising at least two primary vectors each vector comprising a nucleotide sequence cassette of the general formula in 5'→3' direction:
[RS1-RS2-SP-PR-X-TR-SP-RS2'-RS1']
wherein
RS1 and RS1' denote restriction sites,
RS2 and RS2' denote restriction sites different from RS1 and RS1',
SP individually denotes a spacer sequence of at least two nucleotides,
PR denotes a promoter,
X denotes an expressible nucleotide sequence,
TR denotes a terminator.
- wherein the expressible nucleotide sequences are isolated from at least one expression state,
- and wherein at least a first and a second primary vector comprise an expressible nucleotide sequence coding for the same peptide under the control of two different promoters in said first and second primary vector.

40. The library according to claim 39, wherein the host cell is selected from the group comprising bacteria such as E. coli or Bacillus subtilis, or fungi such as yeast.

41. The library according to claim 39, wherein substantially all vectors comprise the same RS2 and RS2' sequence.

42. The library according to any of the preceding claims 39 to 41, comprising at least one primary vector according to claims 26 to 34.

43. A method for preparing a nucleotide library comprising obtaining expressible nucleotide sequences, cloning the expressible nucleotide sequences into cloning sites of a mixture of primary vectors, the primary vectors comprising a cassette, the cassettes comprising a nucleotide sequence of the general formula in 5'→3' direction:
[RS1-RS2-SP-PR-CS-TR-SP-RS2-RS1]
wherein
RS1 and RS1' denote restriction sites,
RS2 and RS2' denote restriction sites different from RS1 and RS1',
SP individually denotes a spacer sequence of at least two nucleotides,
PR denotes a promoter,
CS denotes a cloning site,
TR denotes a terminator,
and transferring the primary vectors Into a host cell.

## Patentansprüche

1. Nukleinsäurekonkatamer, das in der 5'→3' Richtung eine Kassette einer Nukleotidsequenz der allgemeinen Formel
[rs₂-SP-PR-X-TR-SP-rs₁]ₙ
umfaßt, worin
rs₁ und rs₂ zusammen eine funktionelle Restriktionsschnittstelle bezeichnen,
SP einzeln einen Abstandshalter aus mindestens zwei Nukleotidbasen bezeichnet,
PR einen Promotor bezeichnet, der in einer Zelle funktionieren kann,
X eine exprimierbare Nukleotidsequenz bezeichnet,
TR einen Terminator bezeichnet, und
SP einzeln einen Abstandhalter aus mindestens zwei Nukleotidbasen bezeichnet, und
n≥2 ist, und
worin mindestens eine erste Kassette von einer zweiten Kassette verschieden ist.

2. Konkatamer nach Anspruch 1, worin die exprimierbare Nukleotidsequenz eine DNA-Sequenz umfaßt ausgewählt ist aus der Gruppe umfassend cDNA, genomische DNA.

3. Konkatamer nach einem der vorstehenden Ansprüche 1 bis 2, worin die rs₁-rs₂-Restriktionsschnittstelle von mindestens 2 Kassetten von dem gleichen Restriktionsenzym erkannt wird, vorzugsweise identisch ist.

4. Konkatamer nach einem der Ansprüche 1 bis 3, worin mindestens eine Kassette ein Intron zwischen dem Promotor und der exprimierbaren Nukleotidsequenz umfaßt, worin vorzugsweise im Wesentlichen alle Kassetten ein Intron zwischen dem Promotor und der exprimierbaren Nukleotidsequenz umfassen.

5. Konkatamer nach einem der vorstehenden Ansprüche 1 bis 4, das in einem artifiziellen Chromosom vorliegt.

6. Konkatamer nach Anspruch 5, worin das artifizielle Chromosom ausgewählt ist aus der Gruppe umfassend ein artifizielles Hefechromosom, ein artifizielles mega-Hefechromosom, ein artifizielles Bakterienchromosom, ein artifizielles Mauschromosom, ein artifizielles Säugerchromosom, ein artifizielles Insektenchromosom, ein artifizielles Affenchromosom, ein artifizielles Bakteriophagenchromosom, ein artifizielles Baculoviruschromosom, oder ein artifiziellen humanes Chromosom.

7. Konkatamer nach einem der vorstehenden Ansprüche, worin eine Restriktionsschnittstelle mindestens 6 Basen umfasst.

8. Verfahren zur Konkatenation, welches die Schritte umfaßt, Verknüpfen von mindestens zwei Kassetten von Nukleotidsequenzen, wobei jede Kassette ein erstes überstehendes Ende aufweist, eine Abstandshaltersequenz, einen Promotor, eine exprimierbare Nukleotidsequenz, einen Terminator, eine Abstandshaltersequenz und ein zweites überstehendes Ende, und wobei das Verfahren weiter umfaßt,
Beginnen mit einem primären Vektor
[RS1-RS2-SP-PR-X-TR-SP-RS2'-RS1'],
worin
X eine exprimierbare Nukleotidsequenz bezeichnet,
RS 1 und RS 1' Restriktionsschnittstellen bezeichnen,
RS2 und RS2' Restriktionsschnittstellen bezeichnen, die verschieden sind von RS 1 und RS1', SP einzeln eine Abstandshaltersequenz mit mindestens zwei Nukleotidbasen bezeichnet,
PR einen Promotor bezeichnet,
TR einen Terminator bezeichnet,
i) Schneiden des primären Vektors mittels mindestens einem für RS2 und RS2' spezifischen Restriktionsenzym, wobei Kassetten erhalten werden mit der allgemeinen Formel [rs₂-SP-PR-X-TR-SP-rs₁], worin rs₁ und rs₂ zusammen eine funktionelle Restriktionsschnittstelle RS2 oder RS2' bezeichnen,
ii) Zusammenfügen der herausgeschnittenen Kassetten durch Interaktion zwischen rs₁ und rs₂.

9. Verfahren nach Anspruch 8, weiter umfassend die Zugabe von Vektorarmen, von denen jeder ein RS2 oder RS2' an einem Ende und einen nicht-komplementären Überhang oder ein glattes Ende am anderen Ende aufweist.

10. Verfahren nach Anspruch 8, weiter umfassend die Zugabe von Stopperfragmenten, wobei die Stopperfragmente jeweils ein RS2 oder RS2' an einem Ende und einen nicht-komplementären Überhang oder ein glattes Ende am anderen Ende aufweisen.

11. Verfahren nach Anspruch 10, weiter umfassend, Ligieren von Vektorarmen an die Stopperfragmente.

12. Verfahren nach Anspruch 8, weiter umfassend
(i) Isolieren von mRNA aus einem Expressionszustand,
(ii) Erhalten von im Wesentlichen Vollängen-cDNA-Klonen entsprechend der mRNA-Sequenzen,
(ii) Inserieren der im Wesentlichen Vollängen-cDNA-Klone in eine Klonierungsstelle in eine Kassette eines primären Vektors, wobei die Kassette in 5'→3' Richtung die allgemeine Formel [RS1-RS2-SP-PR-CS-TR-SP-RS2'-RS1'] aufweist, worin CS eine Klonierungsstelle bezeichnet.

13. Verfahren nach Anspruch 8, worin RS 1 und RS1' identisch sind und worin RS2 und RS2' identisch sind.

14. Verfahren nach einem der Ansprüche 8 bis 13, wobei das Konkatamer an ein artifizielles Chromosom ligiert ist ausgewählt ist aus der Gruppe umfassend artifizielles Hefechromosom, artifizielles mega-Hefechromosom, artifizielles Bakterienchromosom, artifizielles Mauschromosom, artifizielles humanes Chromosom.

15. Verfahren nach einem der Ansprüche 8 bis 14, wobei RS2 und RS2' in mindestens zwei Kassetten durch ein Restriktionsenzym geschnitten werden, vorzugsweise wobei RS2 und RS2' in im Wesentlichen allen Kassetten durch ein Restriktionsenzym geschnitten werden.

16. Verfahren nach einem der vorstehenden Ansprüche 8 bis 15, wobei RS2 und RS2' mehr als 7 Basen umfassen.

17. Isolierte Zelle, die mindestens ein Konkatamer einzelner Oligonucleotid-Kassetten umfaßt, worin jedes Konkatamer in 5'→3' Richtung ein Oligonukleotid folgenden Formel
[rs₂-SP-PR-X-TR-SP-rs₁]ₙ
umfaßt, worin
rs₁ und rs₂ zusammen eine funktionelle Restriktionsschnittstelle bezeichnen,
SP einzeln einen Abstandshalter aus mindestens zwei Nukleotidbasen bezeichnet,
PR einen Promotor bezeichnet, der in einer Zelle funktionieren kann,
X eine exprimierbare Nukleotidsequenz bezeichnet,
TR einen Terminator bezeichnet,
SP einzeln einen Abstandhalter aus mindestens zwei Nukleotidbasen bezeichnet,
worin n≥2 ist, und
worin mindestens zwei exprimierbare Nukleotidsequenzen von unterschiedlichen Expressionsstadien herrühren.

18. Isolierte Zelle, welche mindestens ein Konkatamer einzelner Oligonukleotid-Kassetten umfasst, worin jedes Konkatamer in 5'→3' Richtung ein Oligonukleotid folgenden Formel
[rs₂-SP-PR-X-TR-SP-rs₁]ₙ
umfaßt, worin
rs₁ und rs₂ zusammen eine funktionelle Restriktionsschnittstelle bezeichnen,
SP einzeln einen Abstandshalter mit mindestens zwei Nukleotidbasen bezeichnet,
PR einen Promotor bezeichnet, der in einer Zelle funktionieren kann,
X eine exprimierbare Nukleotidsequenz bezeichnet,
TR einen Terminator bezeichnet, und
SP einzeln einen Abstandhalter mit mindestens zwei Nukleotidbasen bezeichnet, worin n≥2 ist, und
worin rs₁-rs₂ in mindestens zwei Kassetten von dem gleichen Restriktionsenzym erkannt werden.

19. Zelle nach Anspruch 17 oder 18, worin im Wesentlichen alle rs₁-rs₂-Sequenzen von dem gleichen Restriktionsenzym erkannt werden, vorzugsweise worin im Wesentlichen alle rs₁-rs₂-Sequenzen im Wesentlichen identisch sind.

20. Zelle nach Anspruch 17 oder 18, welche eine Hefezelle ist ausgewählt aus der Gruppe umfassend Bäcker-Hefe, Kluyveromyces marxianus, K. lactis, Candida utilis, Phaffia rhodozyma, Saccharomyces boulardii, Pichia pastoris, Hansenula polymorpha, Yarrowia lipolytica, Candida paraffinica, Schwanniomyces castellii, Pichia stipitis, Candida shehatae, Rhodotorula glutinis, Lipomyces lipofer, Cryptococcos curvatus, Candida spp. (beispielsweise C. palmioleophila), Yarrowia lipolytica, Candida guilliermondii, Candida, Rhodotorula spp., Saccharomycopsis spp., Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii, Pichia spp., Kluyveromyces, Hansenula Kloeckera, Pichia, Pachysolen spp., oder Torulopsis bombicola.

21. Zelle nach einem der vorstehenden Ansprüche 17 bis 20, worin mindestens ein Konkatamer, vorzugsweise im Wesentlichen alle Konkatamere ein Konkatamer(e) nach einem der Ansprüche 1 bis 7 ist/sind.

22. Artifizielles Chromosom, welches mindestens ein Nukleotid-Konkatamer umfasst, worin das Konkatamer in 5'→3' Richtung Kassetten von Nukleotidsequenzen der allgemeinen Formel
[rs₂-SP-PR-X-TR-SP-rs₁]ₙ
umfaßt, worin
rs₁ und rs₂ zusammen eine funktionelle Restriktionsschnittstelle bezeichnen,
SP einen Abstandshalter aus mindestens zwei Nukleotidbasen bezeichnet,
PR einen Promotor bezeichnet, der in einer Zelle funktionieren kann,
X eine exprimierbare Nukleotidsequenz bezeichnet,
TR einen Terminator bezeichnet, und
SP einen Abstandhalter aus mindestens zwei Nukleotidbasen bezeichnet, und
n≥2 ist.

23. Artifizielles Chromosom, worin das Konkatamer nach einem der Ansprüche 1 bis 7 ist.

24. Isolierte Zelle, welche mindestens ein artifizielles Chromosom nach einem der Ansprüche 22 und 23 umfasst.

25. In vitro Verfahren zur Herstellung einer transgenen Zelle, welches umfasst, Einbringen eines Konkatamers nach einem der Ansprüche 1 bis 7 in eine Wirtszelle.

26. Primärer Vektor, welcher eine Nukleotidsequenz-Kassette in 5'→3' Richtung der allgemeinen Formel
[RS1-RS2-SP-PR-CS-TR-SP-RS2'-RS1']
umfaßt, worin
RS 1 und RS 1' Restriktionsschnittstellen bezeichnen,
RS2' und RS2' Restriktionsschnittstellen bezeichnen, die verschieden sind von RS1 und RS1',
SP einzeln eine Abstandshaltersequenz aus mindestens zwei Nukleotidbasen bezeichnet,
PR einen Promotor bezeichnet,
CS eine Klonierungsstelle bezeichnet,
TR einen Terminator bezeichnet.

27. Vektor nach Anspruch 26, worin die Nukleotidsequenz eine DNA-Sequenz ist.

28. Vektor nach einem der vorstehenden Ansprüche 26 bis 27, welcher weiter umfasst, eine Intronsequenz zwischen dem Promotor und der Klonierungsstelle und/oder zwischen der Klonierungsstelle und dem Terminator.

29. Vektor nach einem der vorstehenden Ansprüche 26 bis 28, worin die Klonierungsstelle eine exprimierbare Nukleotidsequenz umfasst.

30. Vektor nach einem der vorstehenden Ansprüche 26 bis 29, worin RS2 und RS2' identisch sind.

31. Vektor nach einem der vorstehenden Ansprüche 26 bis 30, worin RS1 und RS1' identisch sind.

32. Vektor nach einem der vorstehenden Ansprüche 26 bis 31, welcher weiter eine Abstandshaltersequenz zwischen TR und RS2' umfasst.

33. Vektor nach einem der vorstehenden Ansprüche 26 bis 32, worin der Promotor ein extern steuerbarer Promotor ist.

34. Vektor nach einem der vorstehenden Ansprüche 26 bis 33, worin der primäre Vektor, der die Kassette umfasst, ein Plasmidvektor mit einer hohen Kopienzahl ist, der in E. coli vermehrt werden kann und zum Erhalt in E. coli einen selektierbaren Marker aufweist.

35. Konkatamer nach einem der Ansprüche 1 bis 7, worin mindestens eine Kassette die Kassette aus einem primären Vektor nach einem der Ansprüche 26 bis 34 umfasst, mehr bevorzugt worin im Wesentlichen alle Kassetten die Kassette aus einem primären Vektor nach einem der Ansprüche 26 bis 34 umfasst.

36. Verfahren zur Herstellung eines primären Vektors, welches umfasst,
Einbringen einer exprimierbaren Nukleotidsequenz in eine Klonierungsstelle in einem primären Vektor, der eine Kassette umfasst, worin die Kassette eine Nukleotidsequenz in 5'→3' Richtung der allgemeinen Formel
[RS1-RS2-SP-PR-CS-TR-SP-RS2'-RS1']
umfaßt, worin
RS1 und RS1' Restriktionsschnittstellen bezeichnen,
RS2' und RS2' Restriktionsschnittstellen bezeichnen, die verschieden sind von RS1 und RS1',
SP einzeln eine Abstandshaltersequenz mit mindestens zwei Nukleotidbasen bezeichnet,
PR einen Promotor bezeichnet,
CS eine Klonierungsstelle bezeichnet,
TR einen Terminator bezeichnet.

37. Verfahren nach Anspruch 36, welches weiter umfasst,
Isolieren von Gesamt-mRNA eines Expressionsstadiums, und
Erhalten von Vollängen-cDNA zum Inserieren in den Vektor.

38. Verfahren nach Anspruch 37, welches weiter umfasst, Selektieren von cDNA, um im Wesentlichen Vollängen-cDNA zu erhalten.

39. Nukleotid-Bank, welche mindestens zwei primäre Vektoren enthält, worin jeder Vektor eine Nukleotidsequenz-Kassette in 5'→3' Richtung der allgemeinen Formel
[RS1-RS2-SP-PR-X-TR-SP-RS2'-RS1']
umfaßt, worin
RS1 und RS1' Restriktionsschnittstellen bezeichnen,
RS2' und RS2' Restriktionsschnittstellen bezeichnen, die verschieden sind von RS1 und RS1
SP einzeln eine Abstandshaltersequenz aus mindestens zwei Nukleotidbasen bezeichnet,
PR einen Promotor bezeichnet,
X eine exprimierbare Nukleotidsequenz bezeichnet,
TR einen Terminator bezeichnet,
worin die exprimierbaren Nukleotidsequenzen isoliert werden aus mindestens einem Expressionsstadium,
und worin mindestens ein erster und ein zweiter primärer Vektor eine exprimierbare Nukleotidsequenz enthalten, die das gleiche Peptid unter der Kontrolle von zwei unterschiedlichen Promotoren in dem ersten und zweiten primären Vektor codiert.

40. Bank nach Anspruch 39, worin die Wirtszelle ausgewählt ist aus der Gruppe umfassend Bakterien, wie E.coli oder Bacillus subtilis, oder Pilzen, wie Hefe.

41. Bank nach Anspruch 39, worin im Wesentlichen alle Vektoren die gleichen RS2 und RS2'-Sequenz umfassen.

42. Bank nach einem der vorstehenden Ansprüche 39 bis 41, welche mindestens einen primären Vektor nach Anspruch 26 bis 34 umfasst.

43. Verfahren zur Herstellung eines Nukleotidbank, welches umfasst,
Erhalten von exprimierbaren Nukleotidsequenzen,
Klonieren der exprimierbaren Nukleotidsequenzen in Klonierungsstellen eines Gemisches primärer Vektoren,
worin die primären Vektoren eine Kassette umfassen, worin die Kassetten eine Nukleotidsequenz in 5'→3' Richtung der allgemeinen Formel
[RS1-RS2-SP-PR-CS-TR-SP-RS2-RS1']
umfaßt, worin
RS1 und RS1' Restriktionsschnittstellen bezeichnen,
RS2' und RS2' Restriktionsschnittstellen bezeichnen, die verschieden sind von RS1 und RS1',
SP einzeln eine Abstandshaltersequenz mit mindestens zwei Nukleotidbasen bezeichnet,
PR einen Promotor bezeichnet,
CS eine Klonierungsstelle bezeichnet,
TR einen Terminator bezeichnet, und
Überführen des primären Vektors in eine Wirtszelle.

## Revendications

1. Concatémère de nucléotides comprenant, dans la direction 5' → 3', une cassette de séquence nucléotidique de la formule générale :
[rs₂-SP-PR-X-TR-SP-rs₁]ₙ
dans laquelle :
rs₁ et rs₂ représentent ensemble un site de restriction fonctionnel,
SP représente individuellement un espaceur d'au moins deux bases nucléotidiques,
PR représente un promoteur, capable de fonctionner dans une cellule,
X représente une séquence nucléotidique pouvant être exprimée,
TR représente un terminateur, et
SP représente individuellement un espaceur d'au moins deux bases nucléotidiques,
n ≥ 2, et
dans lequel au moins une première cassette est différente d'une seconde cassette.

2. Concatémère selon la revendication 1, dans lequel la séquence nucléotidique pouvant être exprimée comprend une séquence d'ADN choisie dans le groupe comprenant un ADNc, un ADN génomique.

3. Concatémère selon l'une quelconque des revendications précédentes 1 et 2, dans lequel les sites de restriction rs₁-rs₂ d'au moins deux cassettes sont reconnus par la même enzyme de restriction, de manière davantage préférée lesdits sites de restriction sont identiques.

4. Concatémère selon l'une quelconque des revendications 1 à 3, dans lequel au moins une cassette comprend un intron entre le promoteur et la séquence nucléotidique pouvant être exprimée, de manière davantage préférée sensiblement toutes les cassettes comprennent un intron entre le promoteur et la séquence nucléotidique pouvant être exprimée.

5. Concatémère selon l'une quelconque des revendications précédentes 1 à 4, contenu dans un chromosome artificiel.

6. Concatémère selon la revendication 5, dans lequel le chromosome artificiel est choisi dans le groupe comprenant un chromosome artificiel de levure, un méga chromosome artificiel de levure, un chromosome artificiel bactérien, un chromosome artificiel de souris, un chromosome artificiel de mammifère, un chromosome artificiel d'insecte, un chromosome artificiel aviaire, un chromosome artificiel de bactériophage, un chromosome artificiel de baculovirus, ou un chromosome artificiel humain.

7. Concatémère selon l'une quelconque des revendications précédentes, dans lequel un site de restriction comprend au moins 6 bases.

8. Procédé de concaténation comprenant les étapes consistant à concaténer au moins deux cassettes de séquences nucléotidiques, chaque cassette comprenant une première extrémité cohésive, une séquence d'espaceur, un promoteur, une séquence nucléotidique pouvant être exprimée, un terminateur, une séquence d'espaceur et une seconde extrémité cohésive, dans lequel le procédé comprend en outre les étapes consistant à :
en commençant avec un vecteur primaire
[RS1-RS2-SP-PR-X-TR-SP-RS2'-RS1'],
dans lequel :
X représente une séquence nucléotidique pouvant être exprimée,
RS1 et RS1' représentent des sites de restriction,
RS2 et RS2' représentent des sites de restriction différents de RS1 et RS1',
SP représente individuellement une séquence d'espaceur d'au moins deux nucléotides,
PR représente un promoteur,
TR représente un terminateur,
i) couper le vecteur primaire à l'aide d'au moins une enzyme de restriction spécifique de RS2 et RS2' pour obtenir des cassettes présentant la formule générale [rs₂-SP-PR-X-TR-SP-rs₁], dans laquelle rs₁ et rs₂ représentent ensemble un site de restriction fonctionnel RS2 et RS2',
ii) assembler les cassettes coupées par interaction entre rs₁ et rs₂.

9. Procédé selon la revendication 8, comprenant en outre l'addition de bras de vecteur ayant chacun un RS2 ou RS2' à une extrémité et un dépassement non complémentaire ou une extrémité franche à l'autre extrémité.

10. Procédé selon la revendication 8, comprenant en outre l'addition de fragments d'arrêt, les fragments d'arrêt ayant chacun un RS2 ou RS2' à une extrémité et un dépassement non complémentaire ou une extrémité franche à l'autre extrémité.

11. Procédé selon la revendication 10, comprenant en outre la ligature des bras de vecteur aux fragments d'arrêt.

12. Procédé selon la revendication 8, comprenant en outre :
i) isoler un ARNm dans un état d'expression,
ii) obtenir des clones d'ADNc sensiblement de pleine longueur correspondant aux séquences d'ARNm,
iii) insérer les clones d'ADNc sensiblement de pleine longueur dans un site de clonage dans une cassette dans un vecteur primaire, ladite cassette présentant la formule générale dans la direction 5' → 3' :
[RS1-RS2-SP-PR-CS-TR-SP-RS2'-RS1'],
dans laquelle CS représente un site de clonage.

13. Procédé selon la revendication 8, dans lequel RS1 et RS1' sont identiques, et dans lequel RS2 et RS2' sont identiques.

14. Procédé selon l'une quelconque des revendications 8 à 13, au moyen duquel le concatémère est ligaturé dans un chromosome artificiel choisi dans le groupe comprenant un chromosome artificiel de levure, un méga chromosome artificiel de levure, un chromosome artificiel bactérien, un chromosome artificiel de souris, un chromosome artificiel humain.

15. Procédé selon l'une quelconque des revendications précédentes 8 à 14, au moyen duquel RS2 et RS2' dans au moins deux casettes sont clivés par une enzyme de restriction, de préférence RS2 et RS2' sensiblement dans toutes les cassettes sont clivés par une enzyme de restriction.

16. Procédé selon l'une quelconque des revendications précédentes 8 à 15, dans lequel RS2 et RS2' comprennent plus de 7 bases.

17. Cellule isolée comprenant au moins un concatémère de cassettes d'oligonucléotides individuels, chaque concatémère comprenant un oligonucléotide de la formule suivante dans la direction 5' → 3' :
[rs₂-SP-PR-X-TR-SP-rs₁]ₙ
dans laquelle :
rs₁ et rs₂ représentent ensemble un site de restriction,
SP représente individuellement un espaceur d'au moins deux bases nucléotidiques,
PR représente un promoteur, capable de fonctionner dans la cellule,
X représente une séquence nucléotidique pouvant être exprimée,
TR représente un terminateur, et
SP représente individuellement une séquence d'espaceur d'au moins deux nucléotides,
dans laquelle n ≥ 2, et
dans laquelle au moins deux séquences nucléotidiques pouvant être exprimées proviennent de différents états d'expression.

18. Cellule isolée comprenant au moins un concatémère de cassettes d'oligonucléotides individuels, chaque concatémère comprenant un oligonucléotide de la formule suivante dans la direction 5' → 3' :
[rs₂-SP-PR-X-TR-SP-rs₁]ₙ,
dans laquelle :
rs₁ et rs₂ représentent ensemble un site de restriction,
SP représente individuellement un espaceur d'au moins deux bases nucléotidiques,
PR représente un promoteur, capable de fonctionner dans la cellule,
X représente une séquence nucléotidique pouvant être exprimée,
TR représente un terminateur, et
SP représente individuellement une séquence d'espaceur d'au moins deux nucléotides,
dans laquelle n ≥ 2, et
dans laquelle rs₁-rs₂ dans au moins deux cassettes sont reconnus par la même enzyme de restriction.

19. Cellule selon la revendication 17 ou 18, dans laquelle sensiblement toutes les séquences rs₁-rs₂ sont reconnues par la même enzyme de restriction, de manière davantage préférée dans laquelle sensiblement toutes les séquences rs₁-rs₂ sont sensiblement identiques.

20. Cellule selon la revendication 17 ou 18, étant une cellule de levure choisie dans le groupe comprenant la levure de boulangerie, *Kluyveromyces marxianus, K. lactis, Candida utilis, Phaffia rhodozyma, Saccharomyces boulardii, Pichia pastoris, Hansenula polymorpha, Yarrowla lipolytica, Candida paraffinica, Schwanniomyces castellii, Pichia stipitis, Candida shehatae, Rhodotorula glutinis, Lipomyces lipofer, Cryptococcus curvatus,* le genre *Candida* (par exemple, *C. palmioleophila*), *Yarrowia lipolytica, Candida guilliermondii, Candida,* le genre Rhodotorula, le genre *Saccharomycopsis, Aureobasidium pullulans, Candida brumptii, Candida hydrocarbofumarica, Torulopsis, Candida tropicalis, Saccharomyces cerevisiae, Rhodotorula rubra, Candida flaveri, Eremothecium ashbyii,* le genre *Pichia, Kluyveromyces, Hansenula, Kloeckera, Pichia,* le genre *Pachysolen,* ou Torulopsis bombicola.

21. Cellule selon l'une quelconque des revendications précédentes 17 à 20, dans laquelle au moins un concatémère, de préférence sensiblement tous les concatémères sont des concatémères selon les revendications 1 à 7.

22. Chromosome artificiel comprenant au moins un concatémère de nucléotides, le concatémère comprenant, dans la direction 5' → 3', des cassettes de séquences nucléotidiques de formule générale :
[rs₂-SP-PR-X-TR-SP-rs₁]ₙ,
dans laquelle :
rs₁ et rs₂ représentent ensemble un site de restriction,
SP représente un espaceur d'au moins deux bases nucléotidiques,
PR représente un promoteur, capable de fonctionner dans une cellule,
X représente une séquence nucléotidique pouvant être exprimée,
TR représente un terminateur, et
SP représente un espaceur d'au moins deux bases nucléotidiques,
n ≥ 2.

23. Chromosome artificiel, dans lequel ledit concatémère est le concatémère selon l'une quelconque des revendications 1 à 7.

24. Cellule isolée comprenant au moins un chromosome artificiel selon l'une quelconque des revendications 22 et 23.

25. Procédé *in vitro* destiné à développer une cellule transgénique comprenant l'insertion dans une cellule hôte d'un concatémère selon l'une quelconque des revendications 1 à 7.

26. Vecteur primaire comprenant une cassette de séquence nucléotidique de formule générale dans la direction 5' → 3' :
[RS1-RS2-SP-PR-CS-TR-SP-RS2'-RS1'],
dans laquelle :
RS1 et RS1' représentent des sites de restriction,
RS2 et RS2' représentent des sites de restriction différents de RS1 et RS1',
SP représente individuellement une séquence d'espaceur d'au moins deux nucléotides,
PR représente un promoteur,
CS représente un site de clonage,
TR représente un terminateur.

27. Vecteur selon la revendication 26, dans lequel la séquence nucléotidique est une séquence d'ADN.

28. Vecteur selon l'une quelconque des revendications précédentes 26 et 27, comprenant en outre une séquence d'intron entre le promoteur et le site de clonage et/ou entre le site de clonage et le terminateur.

29. Vecteur selon l'une quelconque des revendications précédentes 26 à 28, dans lequel le site de clonage comprend une séquence nucléotidique pouvant être exprimée.

30. Vecteur selon l'une quelconque des revendications précédentes 26 à 29, dans lequel RS2 et RS2' sont identiques.

31. Vecteur selon l'une quelconque des revendications précédentes 26 à 30, dans lequel RS1 et RS1' sont identiques.

32. Vecteur selon l'une quelconque des revendications précédentes 26 à 31, comprenant en outre une séquence d'espaceur entre TR et RS2'.

33. Vecteur selon l'une quelconque des revendications précédentes 26 à 32, dans lequel le promoteur est un promoteur à contrôle externe.

34. Vecteur selon l'une quelconque des revendications précédentes 26 à 33, dans lequel le vecteur primaire comprenant la cassette est un vecteur plasmidique ayant un nombre élevé de copies, étant capable d'être propagé dans *E. coli,* et ayant un marqueur sélectionnable pour l'entretien dans *E. coli.*

35. Concatémère selon l'une quelconque des revendications précédentes 1 et 7, dans lequel au moins une cassette comprend la cassette provenant d'un vecteur primaire selon les revendications 26 à 34, de manière davantage préférée sensiblement toutes les cassettes comprennent la cassette provenant d'un vecteur primaire selon les revendications 26 à 34.

36. Procédé de préparation d'un vecteur primaire comprenant :
insérer une séquence nucléotidique pouvant être exprimée dans un site de clonage dans un vecteur primaire comprenant une cassette, la cassette comprenant une séquence nucléotidique de formule générale dans la direction 5' → 3' :
[RS1-RS2-SP-PR-CS-TR-SP-RS2'-RS1'],
dans laquelle :
RS1 et RS1' représentent des sites de restriction,
RS2 et RS2' représentent des sites de restriction différents de RS1 et RS1',
SP représente individuellement une séquence d'espaceur d'au moins deux nucléotides,
PR représente un promoteur,
CS représente un site de clonage,
TR représente un terminateur.

37. Procédé selon la revendication 36, comprenant en outre :
isoler un ARNm total dans un état d'expression, et
obtenir un ADNc de pleine longueur en vue de l'insertion dans le vecteur.

38. Procédé selon la revendication 37, comprenant en outre le choix d'un ADNc afin d'obtenir un ADNc sensiblement de pleine longueur.

39. Banque de nucléotides comprenant au moins deux vecteurs primaires, chaque vecteur comprenant une cassette de séquence nucléotidique de formule générale dans la direction 5' → 3' :
[RS1-RS2-SP-PR-X-TR-SP-RS2'-RS1'],
dans laquelle :
RS1 et RS1' représentent des sites de restriction,
RS2 et RS2' représentent des sites de restriction différents de RS1 et RS1',
SP représente individuellement une séquence d'espaceur d'au moins deux nucléotides,
PR représente un promoteur,
X représente une séquence nucléotidique pouvant être exprimée,
TR représente un terminateur,
dans laquelle les séquences nucléotidiques pouvant être exprimées sont isolées dans au moins un état d'expression,
et dans laquelle au moins un premier et un second vecteurs primaires comprennent une séquence nucléotidique pouvant être exprimée codant pour le même peptide sous le contrôle de deux différents promoteurs dans lesdits premier et second vecteurs primaires.

40. Banque selon la revendication 39, dans laquelle la cellule hôte est choisie dans le groupe comprenant des bactéries telles que *E. coli* ou *Bacillus subtilis,* ou des champignons tels qu'une levure.

41. Banque selon la revendication 39, dans laquelle sensiblement tous les vecteurs comprennent la même séquence RS2 et RS2'.

42. Banque selon l'une quelconque des revendications précédentes 39 à 41, comprenant au moins un vecteur primaire selon les revendications 26 à 34.

43. Procédé de préparation d'une banque de nucléotides comprenant l'obtention de séquences nucléotidiques pouvant être exprimées, le clonage des séquences nucléotidiques pouvant être exprimées dans les sites de clonage d'un mélange de vecteurs primaires, les vecteurs primaires comprenant une cassette, les cassettes comprenant une séquence nucléotidique de formule générale dans la direction 5' → 3' :
[RS1-RS2-SP-PR-CS-TR-SP-RS2'-RS1'],
dans laquelle :
RS1 et RS1' représentent des sites de restriction,
RS2 et RS2' représentent des sites de restriction différents de RS1 et RS1',
SP représente individuellement une séquence d'espaceur d'au moins deux nucléotides,
PR représente un promoteur,
CS représente un site de clonage,
TR représente un terminateur.
et le transfert des vecteurs primaires dans une cellule hôte.


### SEQUENCE LISTING

<110> Evolva Biotech AS
   Goldsmith, Neil
   Sørensen, Alexandra M. P.
   Nielsen, Søren V.S.
   Naesby, Michael
<120> Concatemers of differentially expressed multiple genes
<130> P 500 PC00
<150> DK PA 2001 00127
   <151> 2001-01-25
<150> US 60/301,022
   <151> 2001-06-27
<160> 4
<170> PatentIn version 3.1
<210> 1
   <211> 3417
   <212> DNA
   <213> Synthetic
<220>
   <221> misc_feature
   <222> (1902)..(2759)
   <223> Ampicillin resistance gene
<220>
   <221> rep_origin
   <222> (959)..(1899)
   <223> ColE1
<220>
   <221> misc_feature
   <222> (2891) .. (3347)
   <223> f1-phage origin of replication
<220>
   <221> terminator
   <222> (495) .. (823)
   <223> ADH1
<220>
   <221> promoter
   <222> (49) .. (437)
   <223> Met25 promoter
<400> 1 
ctgatttgcc cgggcagttc aggctcatca ggcgcgccat gcagggattc ttcggatg
ca
60

agggttcgaa tcccttagct ctcattattt tttgcttttt ctcttgaggt cacatgat
cg
120

caaaatggca aatggcacgt gaagctgtcg atattgggga actgtggtgg ttggcaaa
tg
180

actaattaag ttagtcaagg cgccatcctc atgaaaactg tgtaacataa taaccgaa
gt
240

gtcgaaaagg tggcaccttg tccaattgaa cacgctcgat gaaaaaaata agatatat
at
300

aaggttaagt aaagcgtctg ttagaaagga agtttttcct ttttcttgct ctcttgtc
tt
360

ttcatctact atttccttcg tgtaatacag ggtcgtcaga tacatagata caattcta
tt
420

acccccatcc atacaagctt ggcgccgaat tcgtcgaccc ggggatccgc ggccgcag
gc
480

ctaaattgat ctagagcttt ggacttcttc gccagaggtt tggtcaagtc tccaatca
ag
540

gttgtcggct tgtctacctt gccagaaatt tacgaaaaga tggaaaaggg tcaaatcg
tt
600

ggtagatacg ttgttgacac ttctaaataa gcgaatttct tatgatttat gattttta
tt
660

attaaataag ttataaaaaa aataagtgta tacaaatttt aaagtgactc ttaggttt
ta
 
720

aaacgaaaat tcttgttctt gagtaactct ttcctgtagg tcaggttgct ttctcagg
ta
780

tagcatgagg tcgctcttat tgaccacacc tctaccggca tgcccatggg ttaactga
tc
840

aatgcatcct gcatggcgcg cctgatgagc ctgaactgcc cgggcaaatc agctggac
gt
900

ctgcctgcat taatgaatcg gccaacgcgc ggggagaggc ggtttgcgta ttgggcgc
tc
960

ttccgcttcc tcgctcactg actcgctgcg ctcggtcgtt cggctgcggc gagcggta
tc
1020

agctcactca aaggcggtaa tacggttatc cacagaatca ggggataacg caggaaag
aa
1080

catgtgagca aaaggccagc aaaaggccag gaaccgtaaa aaggccgcgt tgctggcg
tt
1140

tttccatagg ctccgccccc ctgacgagca tcacaaaaat cgacgctcaa gtcagagg
tg
1200

gcgaaacccg acaggactat aaagatacca ggcgtttccc cctggaagct ccctcgtg
cg
1260

ctctcctgtt ccgaccctgc cgcttaccgg atacctgtcc gcctttctcc cttcggga
ag
1320

cgtggcgctt tctcatagct cacgctgtag gtatctcagt tcggtgtagg tcgttcgc
tc
1380

caagctgggc tgtgtgcacg aaccccccgt tcagcccgac cgctgcgcct tatccggt
aa
1440

ctatcgtctt gagtccaacc cggtaagaca cgacttatcg ccactggcag cagccact
gg
 
1500

taacaggatt agcagagcga ggtatgtagg cggtgctaca gagttcttga agtggtgg
cc
1560

taactacggc tacactagaa ggacagtatt tggtatctgc gctctgctga agccagtt
ac
1620

cttcggaaaa agagttggta gctcttgatc cggcaaacaa accaccgctg gtagcggt
gg
1680

tttttttgtt tgcaagcagc agattacgcg cagaaaaaaa ggatctcaag aagatcct
tt
1740

gatcttttct acggggtctg acgctcagtg gaacgaaaac tcacgttaag ggattttg
gt
1800

catgagatta tcaaaaagga tcttcaccta gatcctttta aattaaaaat gaagtttt
aa
1860

atcaatctaa agtatatatg agtaaacttg gtctgacagt taccaatgct taatcagt
ga
1920

ggcacctatc tcagcgatct gtctatttcg ttcatccata gttgcctgac tccccgtc
gt
1980

gtagataact acgatacggg agggcttacc atctggcccc agtgctgcaa tgataccg
cg
2040

agacccacgc tcaccggctc cagatttatc agcaataaac cagccagccg gaagggcc
ga
2100

gcgcagaagt ggtcctgcaa ctttatccgc ctccatccag tctattaatt gttgccgg
ga
2160

agctagagta agtagttcgc cagttaatag tttgcgcaac gttgttgcca ttgctaca
gg
2220

catcgtggtg tcacgctcgt cgtttggtat ggcttcattc agctccggtt cccaacga
tc
 
2280

aaggcgagtt acatgatccc ccatgttgtg caaaaaagcg gttagctcct tcggtcct
cc
2340

gatcgttgtc agaagtaagt tggccgcagt gttatcactc atggttatgg cagcactg
ca
2400

taattctctt actgtcatgc catccgtaag atgcttttct gtgactggtg agtactca
ac
2460

caagtcattc tgagaatagt gtatgcggcg accgagttgc tcttgcccgg cgtcaata
cg
2520

ggataatacc gcgccacata gcagaacttt aaaagtgctc atcattggaa aacgttct
tc
2580

ggggcgaaaa ctctcaagga tcttaccgct gttgagatcc agttcgatgt aacccact
cg
2640

tgcacccaac tgatcttcag catcttttac tttcaccagc gtttctgggt gagcaaaa
ac
2700

aggaaggcaa aatgccgcaa aaaagggaat aagggcgaca cggaaatgtt gaatactc
at
2760

actcttcctt tttcaatatt attgaagcat ttatcagggt tattgtctca tgagcgga
ta
2820

catatttgaa tgtatttaga aaaataaaca aataggggtt ccgcgcacat ttccccga
aa
2880

agtgccacct gacgcgccct gtagcggcgc attaagcgcg gcgggtgtgg tggttacg
cg
2940

cagcgtgacc gctacacttg ccagcgccct agcgcccgct cctttcgctt tcttccct
tc
3000

ctttctcgcc acgttcgccg gctttccccg tcaagctcta aatcgggggc tcccttta
gg
 
3060

gttccgattt agtgctttac ggcacctcga ccccaaaaaa cttgattagg gtgatggt
tc
3120

acgtagtggg ccatcgccct gatagacggt ttttcgccct ttgacgttgg agtccacg
tt
3180

ctttaatagt ggactcttgt tccaaactgg aacaacactc aaccctatct cggtctat
tc
3240

ttttgattta taagggattt tgccgatttc ggcctattgg ttaaaaaatg agctgatt
ta
3300

acaaaaattt aacgcgaatt ttaacaaaat attaacgctt acaatttcca ttcgccat
tc
3360

aggctgcgca actgttggga agggcgatcg gtgcgggcct cttcgctatt acgccag

3417
<210> 2
   <211> 3501
   <212> DNA
   <213> Synthetic
<220>
   <221> misc_feature
   <222> (1986)..(2843)
   <223> Ampicillin resistance gene
<220>
   <221> rep_origin
   <222> (1043)..(1983)
   <223> ColE1
<220>
   <221> misc_feature
   <222> (2975) .. (3431)
   <223> f1-phage origin of replication
<220>
   <221> terminator
   <222> (579) .. (907)
   <223> ADH1
<220>
   <221> promoter
   <222> (49)..(519)
   <223> Cup1 promoter
<400> 2 
ctgatttgcc cgggcagttc aggctcatca ggcgcgccat gcagggataa gccgatcc
ca
60

ttaccgacat ttgggcgcta tacgtgcata tgttcatgta tgtatctgta tttaaaac
ac
120

ttttgtatta tttttcctca tatatgtgta taggtttata cggatgattt aattatta
ct
180

tcaccaccct ttatttcagg ctgatatctt agccttgtta ctagttagaa aaagacat
tt
240

ttgctgtcag tcactgtcaa gagattcttt tgctggcatt tcttctagaa gcaaaaag
ag
300

cgatgcgtct tttccgctga accgttccag caaaaaagac taccaacgca atatggat
tg
360

tcagaatcat ataaaagaga agcaaataac tccttgtctt gtatcaattg cattataa
ta
420

tcttcttgtt agtgcaatat catatagaag tcatcgaaat agatattaag aaaaacaa
ac
480

tgtacaatca atcaatcaat catcacataa aatgttcaaa gcttggcgcc gaattcgt
cg
540

acccggggat ccgcggccgc aggcctaaat tgatctagag ctttggactt cttcgcca
ga
600

ggtttggtca agtctccaat caaggttgtc ggcttgtcta ccttgccaga aatttacg
 
aa
660

aagatggaaa agggtcaaat cgttggtaga tacgttgttg acacttctaa ataagcga
at
720

ttcttatgat ttatgatttt tattattaaa taagttataa aaaaaataag tgtataca
aa
780

ttttaaagtg actcttaggt tttaaaacga aaattcttgt tcttgagtaa ctctttcc
tg
840

taggtcaggt tgctttctca ggtatagcat gaggtcgctc ttattgacca cacctcta
cc
900

ggcatgccca tgggttaact gatcaatgca tcctgcatgg cgcgcctgat gagcctga
ac
960

tgcccgggca aatcagctgg acgtctgcct gcattaatga atcggccaac gcgcgggg
ag
1020

aggcggtttg cgtattgggc gctcttccgc ttcctcgctc actgactcgc tgcgctcg
gt
1080

cgttcggctg cggcgagcgg tatcagctca ctcaaaggcg gtaatacggt tatccaca
ga
1140

atcaggggat aacgcaggaa agaacatgtg agcaaaaggc cagcaaaagg ccaggaac
cg
1200

taaaaaggcc gcgttgctgg cgtttttcca taggctccgc ccccctgacg agcatcac
aa
1260

aaatcgacgc tcaagtcaga ggtggcgaaa cccgacagga ctataaagat accaggcg
tt
1320

tccccctgga agctccctcg tgcgctctcc tgttccgacc ctgccgctta ccggatac
ct
1380

gtccgccttt ctcccttcgg gaagcgtggc gctttctcat agctcacgct gtaggtat
 
ct
1440

cagttcggtg taggtcgttc gctccaagct gggctgtgtg cacgaacccc ccgttcag
cc
1500

cgaccgctgc gccttatccg gtaactatcg tcttgagtcc aacccggtaa gacacgac
tt
1560

atcgccactg gcagcagcca ctggtaacag gattagcaga gcgaggtatg taggcggt
gc
1620

tacagagttc ttgaagtggt ggcctaacta cggctacact agaaggacag tatttggt
at
1680

ctgcgctctg ctgaagccag ttaccttcgg aaaaagagtt ggtagctctt gatccggc
aa
1740

acaaaccacc gctggtagcg gtggtttttt tgtttgcaag cagcagatta cgcgcaga
aa
1800

aaaaggatct caagaagatc ctttgatctt ttctacgggg tctgacgctc agtggaac
ga
1860

aaactcacgt taagggattt tggtcatgag attatcaaaa aggatcttca cctagatc
ct
1920

tttaaattaa aaatgaagtt ttaaatcaat ctaaagtata tatgagtaaa cttggtct
ga
1980

cagttaccaa tgcttaatca gtgaggcacc tatctcagcg atctgtctat ttcgttca
tc
2040

catagttgcc tgactccccg tcgtgtagat aactacgata cgggagggct taccatct
gg
2100

ccccagtgct gcaatgatac cgcgagaccc acgctcaccg gctccagatt tatcagca
at
2160

aaaccagcca gccggaaggg ccgagcgcag aagtggtcct gcaactttat ccgcctcc
 
at
2220

ccagtctatt aattgttgcc gggaagctag agtaagtagt tcgccagtta atagtttg
cg
2280

caacgttgtt gccattgcta caggcatcgt ggtgtcacgc tcgtcgtttg gtatggct
tc
2340

attcagctcc ggttcccaac gatcaaggcg agttacatga tcccccatgt tgtgcaaa
aa
2400

agcggttagc tccttcggtc ctccgatcgt tgtcagaagt aagttggccg cagtgtta
tc
2460

actcatggtt atggcagcac tgcataattc tcttactgtc atgccatccg taagatgc
tt
2520

ttctgtgact ggtgagtact caaccaagtc attctgagaa tagtgtatgc ggcgaccg
ag
2580

ttgctcttgc ccggcgtcaa tacgggataa taccgcgcca catagcagaa ctttaaaa
gt
2640

gctcatcatt ggaaaacgtt cttcggggcg aaaactctca aggatcttac cgctgttg
ag
2700

atccagttcg atgtaaccca ctcgtgcacc caactgatct tcagcatctt ttactttc
ac
2760

cagcgtttct gggtgagcaa aaacaggaag gcaaaatgcc gcaaaaaagg gaataagg
gc
2820

gacacggaaa tgttgaatac tcatactctt cctttttcaa tattattgaa gcatttat
ca
2880

gggttattgt ctcatgagcg gatacatatt tgaatgtatt tagaaaaata aacaaata
gg
2940

ggttccgcgc acatttcccc gaaaagtgcc acctgacgcg ccctgtagcg gcgcatta
 
ag
3000

cgcggcgggt gtggtggtta cgcgcagcgt gaccgctaca cttgccagcg ccctagcg
cc
3060

cgctcctttc gctttcttcc cttcctttct cgccacgttc gccggctttc cccgtcaa
gc
3120

tctaaatcgg gggctccctt tagggttccg atttagtgct ttacggcacc tcgacccc
aa
3180

aaaacttgat tagggtgatg gttcacgtag tgggccatcg ccctgataga cggttttt
cg
3240

ccctttgacg ttggagtcca cgttctttaa tagtggactc ttgttccaaa ctggaaca
ac
3300

actcaaccct atctcggtct attcttttga tttataaggg attttgccga tttcggcc
ta
3360

ttggttaaaa aatgagctga tttaacaaaa atttaacgcg aattttaaca aaatatta
ac
3420

gcttacaatt tccattcgcc attcaggctg cgcaactgtt gggaagggcg atcggtgc
gg
3480

gcctcttcgc tattacgcca g

3501
<210> 3
   <211> 4188
   <212> DNA
   <213> Synthetic
<220>
   <221> misc_feature
   <222> (2673)..(3530)
   <223> Ampicillin resistance gene
<220>
   <221> rep_origin
   <222> (1730)..(2670)
   <223> ColE1
<220>
   <221> misc_feature
   <222> (3662).. (4118)
   <223> f1-phage origin of replication
<220>
   <221> terminator
   <222> (1027)..(1355)
   <223> ADH1
<220>
   <221> promoter
   <222> (582)..(969)
   <223> Met25 promoter
<220>
   <221> misc_feature
   <222> (1365)..(1603)
   <223> ARS1 (autonomous replicating sequence) for Yeast replica tion
<220>
   <221> misc_feature
   <222> (49) .. (574)
   <223> lambda spacer DNA (22428-22923)
<400> 3 
ctgatttgcc cgggcagttc aggctcatca ggcgcgccat gcagggattc tggaaatt
gc
60

aacgaaggaa gaaacctcgt tgctggaagc ctggaagaag tatcgggtgt tgctgaac
cg
120

tgttgataca tcaactgcac ctgatattga gtggcctgct gtccctgtta tggagtaa
tc
180

gttttgtgat atgccgcaga aacgttgtat gaaataacgt tctgcggtta gttagtat
at
240
 
tgtaaagctg agtattggtt tatttggcga ttattatctt caggagaata atggaagt
tc
300

tatgactcaa ttgttcatag tgtttacatc accgccaatt gcttttaaga ctgaacgc
at
360

gaaatatggt ttttcgtcat gttttgagtc tgctgttgat atttctaaag tcggtttt
tt
420

ttcttcgttt tctctaacta ttttccatga aatacatttt tgattattat ttgaatca
at
480

tccaattacc tgaagtcttt catctataat tggcattgta tgtattggtt tattggag
ta
540

gatgcttgct tttctgagcc atagctctga tatcagatct tcttcggatg caagggtt
cg
600

aatcccttag ctctcattat tttttgcttt ttctcttgag gtcacatgat cgcaaaat
gg
660

caaatggcac gtgaagctgt cgatattggg gaactgtggt ggttggcaaa tgactaat
ta
720

agttagtcaa ggcgccatcc tcatgaaaac tgtgtaacat aataaccgaa gtgtcgaa
aa
780

ggtggcacct tgtccaattg aacacgctcg atgaaaaaaa taagatatat ataaggtt
aa
840

gtaaagcgtc tgttagaaag gaagtttttc ctttttcttg ctctcttgtc ttttcatc
ta
900

ctatttcctt cgtgtaatac agggtcgtca gatacataga tacaattcta ttacccccc
at
960

ccatacaagc ttggcgccga attcgtcgac ccggggatcc gcggccgcag gcctaaat
tg
1020
 
atctagagct ttggacttct tcgccagagg tttggtcaag tctccaatca aggttgtc
gg
1080

cttgtctacc ttgccagaaa tttacgaaaa gatggaaaag ggtcaaatcg ttggtaga
ta
1140

cgttgttgac acttctaaat aagcgaattt cttatgattt atgattttta ttattaaa
ta
1200

agttataaaa aaaataagtg tatacaaatt ttaaagtgac tcttaggttt taaaacga
aa
1260

attcttgttc ttgagtaact ctttcctgta ggtcaggttg ctttctcagg tatagcat
ga
1320

ggtcgctctt attgaccaca cctctaccgg catgcccatg ggttcttttg aaaagcaa
gc
1380

ataaaagatc taaacataaa atctgtaaaa taacaagatg taaagataat gctaaatc
at
1440

ttggcttttt gattgattgt acaggaaaat atacatcgca gggggttgac ttttacca
tt
1500

tcaccgcaat ggaatcaaac ttgttgaaga gaatgttcac aggcgcatac gctacaat
ga
1560

cccgattctt gctagccttt tctcggtctt gcaaacaacc gccaactgat caatgcat
cc
1620

tgcatggcgc gcctgatgag cctgaactgc ccgggcaaat cagctggacg tctgcctg
ca
1680

ttaatgaatc ggccaacgcg cggggagagg cggtttgcgt attgggcgct cttccgct
tc
1740

ctcgctcact gactcgctgc gctcggtcgt tcggctgcgg cgagcggtat cagctcac
tc
1800
 
aaaggcggta atacggttat ccacagaatc aggggataac gcaggaaaga acatgtga
gc
1860

aaaaggccag caaaaggcca ggaaccgtaa aaaggccgcg ttgctggcgt ttttccat
ag
1920

gctccgcccc cctgacgagc atcacaaaaa tcgacgctca agtcagaggt ggcgaaac
cc
1980

gacaggacta taaagatacc aggcgtttcc ccctggaagc tccctcgtgc gctctcct
gt
2040

tccgaccctg ccgcttaccg gatacctgtc cgcctttctc ccttcgggaa gcgtggcg
ct
2100

ttctcatagc tcacgctgta ggtatctcag ttcggtgtag gtcgttcgct ccaagctg
gg
2160

ctgtgtgcac gaaccccccg ttcagcccga ccgctgcgcc ttatccggta actatcgt
ct
2220

tgagtccaac ccggtaagac acgacttatc gccactggca gcagccactg gtaacagg
at
2280

tagcagagcg aggtatgtag gcggtgctac agagttcttg aagtggtggc ctaactac
gg
2340

ctacactaga aggacagtat ttggtatctg cgctctgctg aagccagtta ccttcgga
aa
2400

aagagttggt agctcttgat ccggcaaaca aaccaccgct ggtagcggtg gtttttt
gt
2460

ttgcaagcag cagattacgc gcagaaaaaa aggatctcaa gaagatcctt tgatcttt
tc
2520

tacggggtct gacgctcagt ggaacgaaaa ctcacgttaa gggattttgg tcatgaga
tt
2580
 
atcaaaaagg atcttcacct agatcctttt aaattaaaaa tgaagtttta aatcaatc
ta
2640

aagtatatat gagtaaactt ggtctgacag ttaccaatgc ttaatcagtg aggcacct
at
2700

ctcagcgatc tgtctatttc gttcatccat agttgcctga ctccccgtcg tgtagata
ac
2760

tacgatacgg gagggcttac catctggccc cagtgctgca atgataccgc gagaccca
cg
2820

ctcaccggct ccagatttat cagcaataaa ccagccagcc ggaagggccg agcgcaga
ag
2880

tggtcctgca actttatccg cctccatcca gtctattaat tgttgccggg aagctaga
gt
2940

aagtagttcg ccagttaata gtttgcgcaa cgttgttgcc attgctacag gcatcgtg
gt
3000

gtcacgctcg tcgtttggta tggcttcatt cagctccggt tcccaacgat caaggcga
gt
3060

tacatgatcc cccatgttgt gcaaaaaagc ggttagctcc ttcggtcctc cgatcgtt
gt
3120

cagaagtaag ttggccgcag tgttatcact catggttatg gcagcactgc ataattct
ct
3180

tactgtcatg ccatccgtaa gatgcttttc tgtgactggt gagtactcaa ccaagtca
tt
3240

ctgagaatag tgtatgcggc gaccgagttg ctcttgcccg gcgtcaatac gggataat
ac
3300

cgcgccacat agcagaactt taaaagtgct catcattgga aaacgttctt cggggcga
aa
3360
 
actctcaagg atcttaccgc tgttgagatc cagttcgatg taacccactc gtgcaccc
aa
3420

ctgatcttca gcatctttta ctttcaccag cgtttctggg tgagcaaaaa caggaagg
ca
3480

aaatgccgca aaaaagggaa taagggcgac acggaaatgt tgaatactca tactcttc
ct
3540

ttttcaatat tattgaagca tttatcaggg ttattgtctc atgagcggat acatattt
ga
3600

atgtatttag aaaaataaac aaataggggt tccgcgcaca tttccccgaa aagtgcca
cc
3660

tgacgcgccc tgtagcggcg cattaagcgc ggcgggtgtg gtggttacgc gcagcgtg
ac
3720

cgctacactt gccagcgccc tagcgcccgc tcctttcgct ttcttccctt cctttctc
gc
3780

cacgttcgcc ggctttcccc gtcaagctct aaatcggggg ctccctttag ggttccga
tt
3840

tagtgcttta cggcacctcg accccaaaaa acttgattag ggtgatggtt cacgtagt
gg
3900

gccatcgccc tgatagacgg tttttcgccc tttgacgttg gagtccacgt tctttaat
ag
3960

tggactcttg ttccaaactg gaacaacact caaccctatc tcggtctatt cttttgat
tt
4020

ataagggatt ttgccgattt cggcctattg gttaaaaaat gagctgattt aacaaaaa
tt
4080

taacgcgaat tttaacaaaa tattaacgct tacaatttcc attcgccatt caggctgc
gc
4140
 
aactgttggg aagggcgatc ggtgcgggcc tcttcgctat tacgccag

4188
<210> 4
   <211> 11466
   <212> DNA
   <213> Synthetic
<220>
   <221> misc_feature
   <222> (3560) .. (4247)
   <223> Tetrahymena thermophila macronuclear telomere
<220>
   <221> misc_feature
   <222> (6024)..(6711)
   <223> Tetrahymena thermophila macronuclear telomere
<220>
   <221> misc_feature
   <222> (9644)..(10388)
   <223> Autonomous replicating sequence
<220>
   <221> misc_feature
   <222> (10488)..(11465)
   <223> Centromere IV
<220>
   <221> rep_origin
   <222> (7198)..(7198)
   <223> Origin of replication, PMB1
<220>
   <221> misc_feature
   <222> (1962) .. (2765)
   <223> URA3, orotidine-5'-phosphate decarboxylase coding sequence
<220>
   <221> misc_feature
   <222> (4893) .. (5552)
   <223> HIS3, imidazoleglycerolphosphate dehydratase, coding sequence
<220>
   <221> misc_feature
   <222> (7956)..(8816)
   <223> AP(R), beta-lactamase, ampR ampicillin resistance, coding sequence
<220>
   <221> misc_feature
   <222> (9129)..(9803)
   <223> TRP1, phosphoribosylanthranilate isomerase, coding sequence
<400> 4

ttctcatgtt tgacagctta tcatcgataa gctttaatgc ggtagtttat cacagtta
aa
60

ttgctaacgc agtcaggcac cgtgtatgaa atctaacaat gcgctcatcg tcatcctc
gg
120

caccgtcacc ctggatgctg taggcatagg cttggttatg ccggtactgc cgggcctc
tt
180

gcgggatatc gtccattccg acagcatcgc cagtcactat ggcgtgctgc tagcgcta
ta
240

tgcgttgatg caatttctat gcgcacccgt tctcggagca ctgtccgacc gctttggc
cg
300

ccgcccagtc ctgctcgctt cgctacttgg agccactatc gactacgcga tcatggcg
ac
360

cacacccgtc ctgtggatca attcccttta gtataaattt cactctgaac catcttgg
aa
420

ggaccggtaa ttatttcaaa tctctttttc aattgtatat gtgttatgtt atgtagta
ta
480

ctctttcttc aacaattaaa tactctcggt agccaagttg gtttaaggcg caagactt
ta
540

atttatcact acggaattgg cgcgccaatt ccgtaatctt gagatcgggc gttcgatc
gc
600

cccgggagat tttttgtttt tttatgtctt ccattcactt cccagacttg caagttga
aa
660

tatttctttc aagggaattg atcctctacg ccggacgcat cgtggccggc atcaccgg
cg
720

ccacaggtgc ggttgctggc gcctatatcg ccgacatcac cgatggggaa gatcgggc
tc
780

gccacttcgg gctcatgagc gcttgtttcg gcgtgggtat ggtggcaggc cccgtggc
cg
840

ggggactgtt gggcgccatc tccttgcatg caccattcct tgcggcggcg gtgctcaa
cg
900

gcctcaacct actactgggc tgcttcctaa tgcaggagtc gcataaggga gagcgtcg
ac
960

cgatgccctt gagagccttc aacccagtca gctccttccg gtgggcgcgg ggcatgac
ta
1020

tcgtcgccgc acttatgact gtcttcttta tcatgcaact cgtaggacag gtgccggc
ag
1080

cgctctgggt cattttcggc gaggaccgct ttcgctggag cgcgacgatg atcggcct
gt
1140

cgcttgcggt attcggaatc ttgcacgccc tcgctcaagc cttcgtcact ggtcccgc
ca
1200

ccaaacgttt cggcgagaag caggccatta tcgccggcat ggcggccgac gcgctggg
ct
1260

acgtcttgct ggcgttcgcg acgcgaggct ggatggcctt ccccattatg attcttct
cg
1320

cttccggcgg catcgggatg cccgcgttgc aggccatgct gtccaggcag gtagatga
cg
1380

accatcaggg acagcttcaa ggatcgctcg cggctcttac cagcctaact tcgatcac
tg
1440

gaccgctgat cgtcacggcg atttatgccg cctcggcgag cacatggaac gggttggc
at
1500

ggattgtagg cgccgcccta taccttgtct gcctccccgc gttgcgtcgc ggtgcatg
ga
1560

gccgggccac ctcgacctga atggaagccg gcggcacctc gctaacggat tcaccact
cc
1620

aagaattgga gccaatcaat tcttgcggag aactgtgaat gcgcaaacca acccttgg
ca
1680

gaacatatcc atcgcgtccg ccatctccag cagccgcacg cggcgcatcc cccccccc
ct
1740

ttcaattcaa ttcatcattt tttttttatt cttttttttg atttcggttt ctttgaaa
tt
1800

tttttgattc ggtaatctcc gaacagaagg aagaacgaag gaaggagcac agacttag
at
1860

tggtatatat acgcatatgt agtgttgaag aaacatgaaa ttgcccagta ttcttaac
cc
1920

aactgcacag aacaaaaacc tgcaggaaac gaagataaat catgtcgaaa gctacata
ta
1980

aggaacgtgc tgctactcat cctagtcctg ttgctgccaa gctatttaat atcatgca
cg
2040

aaaagcaaac aaacttgtgt gcttcattgg atgttcgtac caccaaggaa ttactgga
gt
2100

tagttgaagc attaggtccc aaaatttgtt tactaaaaac acatgtggat atcttgac
tg
2160

atttttccat ggagggcaca gttaagccgc taaaggcatt atccgccaag tacaattt
tt
2220

tactcttcga agacagaaaa tttgctgaca ttggtaatac agtcaaattg cagtactc
tg
2280

cgggtgtata cagaatagca gaatgggcag acattacgaa tgcacacggt gtggtggg
cc
2340

caggtattgt tagcggtttg aagcaggcgg cagaagaagt aacaaaggaa cctagagg
cc
2400

ttttgatgtt agcagaattg tcatgcaagg gctccctatc tactggagaa tatactaa
gg
2460

gtactgttga cattgcgaag agcgacaaag attttgttat cggctttatt gctcaaag
ag
2520

acatgggtgg aagagatgaa ggttacgatt ggttgattat gacacccggt gtgggttt
ag
2580

atgacaaggg agacgcattg ggtcaacagt atagaaccgt ggatgatgtg gtctctac
ag
2640

gatctgacat tattattgtt ggaagaggac tatttgcaaa gggaagggat gctaaggt
ag
2700

agggtgaacg ttacagaaaa gcaggctggg aagcatattt gagaagatgc ggccagca
aa
2760

actaaaaaac tgtattataa gtaaatgcat gtatactaaa ctcacaaatt agagcttc
aa
2820

tttaattata tcagttatta ctcgggcgta atgattttta taatgacgaa aaaaaaaa
aa
2880

ttggaaagaa aagggggggg gggcagcgtt gggtcctggc cacgggtgcg catgatcg
tg
2940

ctcctgtcgt tgaggacccg gctaggctgg cggggttgcc ttactggtta gcagaatg
aa
3000

tcaccgatac gcgagcgaac gtgaagcgac tgctgctgca aaacgtctgc gacctgag
ca
3060

acaacatgaa tggtcttcgg tttccgtgtt tcgtaaagtc tggaaacgcg gaagtcag
cg
3120

ccctgcacca ttatgttccg gatctgcatc gcaggatgct gctggctacc ctgtggaa
ca
3180

cctacatctg tattaacgaa gcgctggcat tgaccctgag tgatttttct ctggtccc
gc
3240

cgcatccata ccgccagttg tttaccctca caacgttcca gtaaccgggc atgttcat
ca
3300

tcagtaaccc gtatcgtgag catcctctct cgtttcatcg gtatcattac ccccatga
ac
3360

agaaattccc ccttacacgg aggcatcaag tgaccaaaca ggaaaaaacc gcccttaa
ca
3420

tggcccgctt tatcagaagc cagacattaa cgcttctgga gaaactcaac gagctgga
cg
3480

cggatgaaca ggcagacatc tgtgaatcgc ttcacgacca cgctgatgag ctttaccg
ca
3540

gccctcgagg gataagcttc atttttagat aaaatttatt aatcatcatt aatttctt
ga
3600

aaaacatttt atttattgat cttttataac aaaaaaccct tctaaaagtt tatttttg
aa
3660

tgaaaaactt ataaaaattt atgaaaacta caaaaaataa aatttttaat taaaataa
tt
3720

ttgataagaa cttcaatctt tgactagcta gcttagtcat ttttgagatt taattaat
at
3780

tttatgttta ttcatatata aactattcaa aatattatag aatttaaaca ttttaaca
tc
3840

ttaatcattc ataaataact aaaaatcaaa gtattacatc aataaataac ttttactc
aa
3900

tgtcaaagaa ttattggggt tggggttggg gttggggttg gggttggggt tggggttg
gg
3960

gttggggttg gggttggggt tggggttggg gttggggttg gggttggggt tggggttg
gg
4020

gttggggttg gggttggggt tggggttggg gttggggttg gggttggggt tggggttg
gg
4080

gttggggttg gggttggggt tggggttggg gttggggttg gggttggggt tggggttg
gg
4140

gttggggttg gggttggggt tggggttggg gttggggttg gggtgggaaa acagcatt
ca
4200

ggtattagaa gaatatcctg attcaggtga aaatattgtt gatgcgcggg atcctcgg
gg
4260

acaccaaata tggcgatctc ggccttttcg tttcttggag ctgggacatg tttgccat
cg
4320

atccatctac caccagaacg gccgttagat ctgctgccac cgttgtttcc accgaaga
aa
4380

ccaccgttgc cgtaaccacc acgacggttg ttgctaaaga agctgccacc gccacggc
ca
4440

ccgttgtagc cgccgttgtt gttattgtag ttgctcatgt tatttctggc acttcttg
gt
4500

tttcctctta agtgaggagg aacataacca ttctcgttgt tgtcgttgat gcttaaat
tt
4560

tgcacttgtt cgctcagttc agccataata tgaaatgctt ttcttgttgt tcttacgg
aa
4620

taccacttgc cacctatcac cacaactaac tttttcccgt tcctccatct cttttata
tt
4680

ttttttctcg atcgagttca agagaaaaaa aaagaaaaag caaaaagaaa aaaggaaa
gc
4740

gcgcctcgtt cagaatgaca cgtatagaat gatgcattac cttgtcatct tcagtatc
at
4800

actgttcgta tacatactta ctgacattca taggtataca tatatacaca tgtatata
ta
4860

tcgtatgctg cagctttaaa taatcggtgt cactacataa gaacaccttt ggtggagg
ga
4920

acatcgttgg taccattggg cgaggtggct tctcttatgg caaccgcaag agccttga
ac
4980

gcactctcac tacggtgatg atcattcttg cctcgcagac aatcaacgtg gagggtaa
tt
5040

ctgctagcct ctgcaaagct ttcaagaaaa tgcgggatca tctcgcaaga gagatctc
ct
5100

actttctccc tttgcaaacc aagttcgaca actgcgtacg gcctgttcga aagatcta
cc
5160

accgctctgg aaagtgcctc atccaaaggc gcaaatcctg atccaaacct ttttactc
ca
5220

cgcgccagta gggcctcttt aaaagcttga ccgagagcaa tcccgcagtc ttcagtgg
tg
5280

tgatggtcgt ctatgtgtaa gtcaccaatg cactcaacga ttagcgacca gccggaat
gc
5340

ttggccagag catgtatcat atggtccaga aaccctatac ctgtgtggac gttaatca
ct
5400

tgcgattgtg tggcctgttc tgctactgct tctgcctctt tttctgggaa gatcgagt
gc
5460

tctatcgcta ggggaccacc ctttaaagag atcgcaatct gaatcttggt ttcatttg
ta
5520

atacgcttta ctagggcttt ctgctctgtc atctttgcct tcgtttatct tgcctgct
ca
5580

ttttttagta tattcttcga agaaatcaca ttactttata taatgtataa ttcattat
gt
5640

gataatgcca atcgctaaga aaaaaaaaga gtcatccgct aggtggaaaa aaaaaaat
ga
5700

aaatcattac cgaggcataa aaaaatatag agtgtactag aggaggccaa gagtaata
ga
5760

aaaagaaaat tgcgggaaag gactgtgtta tgacttccct gactaatgcc gtgttcaa
ac
5820

gatacctggc agtgactcct agcgctcacc aagctcttaa aacgagaatt aagaaaaa
gt
5880

cgtcatcttt cgataagttt ttcccacagc aaagcaatag tagaaaaaaa caatggga
aa
5940

cgttgaatga agacaaagcg tcgtggttta aaaggaaata cgctcacgta catgctag
gg
6000

aacaggaccg tgcagcggat cccgcgcatc aacaatattt tcacctgaat caggatat
tc
6060

ttctaatacc tgaatgctgt tttcccaccc caaccccaac cccaacccca accccaac
cc
6120

caaccccaac cccaacccca accccaaccc caaccccaac cccaacccca accccaac
cc
6180

caaccccaac cccaacccca accccaaccc caaccccaac cccaacccca accccaac
cc
6240

caaccccaac cccaacccca accccaaccc caaccccaac cccaacccca accccaac
cc
6300

caaccccaac cccaacccca accccaaccc caaccccaac cccaacccca accccaat
aa
6360

ttctttgaca ttgagtaaaa gttatttatt gatgtaatac tttgattttt agttattt
at
6420

gaatgattaa gatgttaaaa tgtttaaatt ctataatatt ttgaatagtt tatatatg
aa
6480

taaacataaa atattaatta aatctcaaaa atgactaagc tagctagtca aagattga
ag
6540

ttcttatcaa aattatttta attaaaaatt ttattttttg tagttttcat aaattttt
at
6600

aagtttttca ttcaaaaata aacttttaga agggtttttt gttataaaag atcaataa
at
6660

aaaatgtttt tcaagaaatt aatgatgatt aataaatttt atctaaaaat gaagctta
tc
6720

cctcgagggc tgcctcgcgc gtttcggtga tgacggtgaa aacctctgac acatgcag
ct
6780

cccggagacg gtcacagctt gtctgtaagc ggatgccggg agcagacaag cccgtcag
gg
6840

cgcgtcagcg ggtgttggcg ggtgtcgggg cgcagccatg acccagtcac gtagcgat
ag
6900

cggagtgtat actggcttaa ctatgcggca tcagagcaga ttgtactgag agtgcacc
at
6960

atgcggtgtg aaataccgca cagatgcgta aggagaaaat accgcatcag gcgctctt
cc
7020

gcttcctcgc tcactgactc gctgcgctcg gtcgttcggc tgcggcgagc ggtatcag
ct
7080

cactcaaagg cggtaatacg gttatccaca gaatcagggg ataacgcagg aaagaaca
tg
7140

tgagcaaaag gccagcaaaa ggccaggaac cgtaaaaagg ccgcgttgct ggcgtttt
tc
7200

cataggctcc gcccccctga cgagcatcac aaaaatcgac gctcaagtca gaggtggc
ga
7260

aacccgacag gactataaag ataccaggcg tttccccctg gaagctccct cgtgcgct
ct
7320

cctgttccga ccctgccgct taccggatac ctgtccgcct ttctcccttc gggaagcg
tg
7380

gcgctttctc atagctcacg ctgtaggtat ctcagttcgg tgtaggtcgt tcgctcca
ag
7440

ctgggctgtg tgcacgaacc ccccgttcag cccgaccgct gcgccttatc cggtaact
at
7500

cgtcttgagt ccaacccggt aagacacgac ttatcgccac tggcagcagc cactggta
ac
7560

aggattagca gagcgaggta tgtaggcggt gctacagagt tcttgaagtg gtggccta
ac
7620

tacggctaca ctagaaggac agtatttggt atctgcgctc tgctgaagcc agttacct
tc
7680

ggaaaaagag ttggtagctc ttgatccggc aaacaaacca ccgctggtag cggtggtt
tt
7740

tttgtttgca agcagcagat tacgcgcaga aaaaaaggat ctcaagaaga tcctttga
tc
7800

ttttctacgg ggtctgacgc tcagtggaac gaaaactcac gttaagggat tttggtca
tg
7860

agattatcaa aaaggatctt cacctagatc cttttaaatt aaaaatgaag ttttaaat
ca
7920

atctaaagta tatatgagta aacttggtct gacagttacc aatgcttaat cagtgagg
ca
7980

cctatctcag cgatctgtct atttcgttca tccatagttg cctgactccc cgtcgtgt
ag
8040

ataactacga tacgggaggg cttaccatct ggccccagtg ctgcaatgat accgcgag
ac
8100

ccacgctcac cggctccaga tttatcagca ataaaccagc cagccggaag ggccgagc
gc
8160

agaagtggtc ctgcaacttt atccgcctcc atccagtcta ttaattgttg ccgggaag
ct
8220

agagtaagta gttcgccagt taatagtttg cgcaacgttg ttgccattgc tgcaggca
tc
8280

gtggtgtcac gctcgtcgtt tggtatggct tcattcagct ccggttccca acgatcaa
gg
8340

cgagttacat gatcccccat gttgtgcaaa aaagcggtta gctccttcgg tcctccga
tc
8400

gttgtcagaa gtaagttggc cgcagtgtta tcactcatgg ttatggcagc actgcata
at
8460

tctcttactg tcatgccatc cgtaagatgc ttttctgtga ctggtgagta ctcaacca
ag
8520

tcattctgag aatagtgtat gcggcgaccg agttgctctt gcccggcgtc aacacggg
at
8580

aataccgcgc cacatagcag aactttaaaa gtgctcatca ttggaaaacg ttcttcgg
gg
8640

cgaaaactct caaggatctt accgctgttg agatccagtt cgatgtaacc cactcgtg
ca
8700

cccaactgat cttcagcatc ttttactttc accagcgttt ctgggtgagc aaaaacag
ga
8760

aggcaaaatg ccgcaaaaaa gggaataagg gcgacacgga aatgttgaat actcatac
tc
8820

ttcctttttc aatattattg aagcatttat cagggttatt gtctcatgag cggataca
ta
8880

tttgaatgta tttagaaaaa taaacaaata ggggttccgc gcacatttcc ccgaaaag
tg
8940

ccacctgacg tctaagaaac cattattatc atgacattaa cctataaaaa taggcgta
tc
9000

acgaggccct ttcgtcttca agaattaatt cggtcgaaaa aagaaaagga gagggcca
ag
9060

agggagggca ttggtgacta ttgagcacgt gagtatacgt gattaagcac acaaaggc
ag
9120

cttggagtat gtctgttatt aatttcacag gtagttctgg tccattggtg aaagtttg
cg
9180

gcttgcagag cacagaggcc gcagaatgtg ctctagattc cgatgctgac ttgctggg
ta
9240

ttatatgtgt gcccaataga aagagaacaa ttgacccggt tattgcaagg aaaatttc
aa
9300

gtcttgtaaa agcatataaa aatagttcag gcactccgaa atacttggtt ggcgtgtt
tc
9360

gtaatcaacc taaggaggat gttttggctc tggtcaatga ttacggcatt gatatcgt
cc
9420

aactgcatgg agatgagtcg tggcaagaat accaagagtt cctcggtttg ccagttat
ta
9480

aaagactcgt atttccaaaa gactgcaaca tactactcag tgcagcttca cagaaacc
tc
9540

attcgtttat tcccttgttt gattcagaag caggtgggac aggtgaactt ttggattg
ga
9600

actcgatttc tgactgggtt ggaaggcaag agagccccga aagcttacat tttatgtt
ag
9660

ctggtggact gacgccagaa aatgttggtg atgcgcttag attaaatggc gttattgg
tg
9720

ttgatgtaag cggaggtgtg gagacaaatg gtgtaaaaga ctctaacaaa atagcaaa
tt
9780

tcgtcaaaaa tgctaagaaa taggttatta ctgagtagta tttatttaag tattgttt
gt
9840

gcacttgcct gcaggccttt tgaaaagcaa gcataaaaga tctaaacata aaatctgt
aa
9900

aataacaaga tgtaaagata atgctaaatc atttggcttt ttgattgatt gtacagga
aa
9960

atatacatcg cagggggttg acttttacca tttcaccgca atggaatcaa acttgttg
aa
10020

gagaatgttc acaggcgcat acgctacaat gacccgattc ttgctagcct tttctcgg
tc
10080

ttgcaaacaa ccgccggcag cttagtatat aaatacacat gtacatacct ctctccgt
at
10140

cctcgtaatc attttcttgt atttatcgtc ttttcgctgt aaaaacttta tcacactt
at
10200

ctcaaataca cttattaacc gcttttacta ttatcttcta cgctgacagt aatatcaa
ac
10260

agtgacacat attaaacaca gtggtttctt tgcataaaca ccatcagcct caagtcgt
ca
10320

agtaaagatt tcgtgttcat gcagatagat aacaatctat atgttgataa ttagcgtt
gc
10380

ctcatcaatg cgagatccgt ttaaccggac cctagtgcac ttaccccacg ttcggtcc
ac
10440

tgtgtgccga acatgctcct tcactatttt aacatgtgga attaattcta aatcctct
tt
10500

atatgatctg ccgatagata gttctaagtc attgaggttc atcaacaatt ggatttc
tg
10560

tttactcgac ttcaggtaaa tgaaatgaga tgatacttgc ttatctcata gttaactc
ta
10620

agaggtgata cttatttact gtaaaactgt gacgataaaa ccggaaggaa gaataaga
aa
10680

actcgaactg atctataatg cctattttct gtaaagagtt taagctatga aagcctcg
gc
10740

attttggccg ctcctaggta gtgctttttt tccaaggaca aaacagtttc tttttctt
ga
10800

gcaggtttta tgtttcggta atcataaaca ataaataaat tatttcattt atgtttaa
aa
10860

ataaaaaata aaaaagtatt ttaaattttt aaaaaagttg attataagca tgtgacct
tt
10920

tgcaagcaat taaattttgc aatttgtgat tttaggcaaa agttacaatt tctggctc
gt
10980

gtaatatatg tatgctaaag tgaactttta caaagtcgat atggacttag tcaaaaga
aa
11040

ttttcttaaa aatatatagc actagccaat ttagcacttc tttatgagat atattata
ga
11100

ctttattaag ccagatttgt gtattatatg tatttacccg gcgaatcatg gacataca
tt
11160

ctgaaatagg taatattctc tatggtgaga cagcatagat aacctaggat acaagtta
aa
11220

agctagtact gttttgcagt aatttttttc ttttttataa gaatgttacc acctaaat
aa
11280

gttataaagt caatagttaa gtttgatatt tgattgtaaa ataccgtaat atatttgc
at
11340

gatcaaaagg ctcaatgttg actagccagc atgtcaacca ctatattgat caccgata
ta
11400

tggacttcca caccaactag taatatgaca ataaattcaa gatattcttc atgagaat
gg
11460

cccaga

11466